# EUROPEAN PATENT SPECIFICATION

(11) **EP 2 763 963 B1**
(45) Date of publication and mention of the grant of the patent: **26.05.2021**
(21) Application number: 12766094.2
(22) Date of filing: 28.09.2012
(51) Int. Cl.: C07D 207/08, C07D 207/40, C07D 307/46, C07D 409/12, C07D 207/333, C07D 207/267, A01N 43/36, C07C 201/00, C07C 201/12, C07C 205/45, C07C 253/10, C07C 255/17

(54) **ENANTIONSELECTIVE PROCESSES TO INSECTICIDAL 3-ARYL-3-TRIFLUOROMETHYL-SUBSTITUTED PYRROLIDINES**
ENANTIONSELEKTIVE VERFAHREN FÜR INSEKTIZIDE 3-ARYL-3-TRIFLUOROMETHYL-SUBSTITUIERTE PYRROLIDINE
PROCÉDÉS ÉNANTIOSÉLECTIFS POUR DES INSECTICIDES DE PYRROLIDINES 3-ARYL-3-TRIFLUOROMÉTHYL-SUBSTITUÉES

(30) Priority: 03.10.2011 WO PCT/EP2011/067224; 15.12.2011 US 201161576135 P
(43) Date of publication of application: 13.08.2014
(73) Proprietor: Syngenta Participations AG, 4058 Basel (CH)
(72) Inventor: SMEJKAL, Tomas, 4332 Stein (CH); SMITS, Helmars, 4332 Stein (CH); WENDEBORN, Sebastian, Volker, 4332 Stein (CH); BERTHON, Guillaume, 4332 Stein (CH); CASSAYRE, Jérôme, Yves, 4332 Stein (CH); EL QACEMI, Myriem, 4332 Stein (CH)
(74) Representative: HGF
(86) International application number: PCT/EP2012/069171
(87) International publication number: WO 2013/050301

(56) References cited:
- EP-A1- 2 230 230
- WO-A1-2011/080211
- WO-A1-2011/128299
- WO-A1-2011/154555
- WO-A1-2012/045700
- YUTA TANAKA ET AL: "Catalytic Enantioselective Construction of [beta]-Quaternary Carbons via a Conjugate Addition of Cyanide to [beta],[beta]-Disubstituted [alpha],[beta]-Unsaturated Carbonyl Compounds", JOURNAL OF THE AMERICAN CHEMICAL SOCIETY, vol. 132, no. 26, 7 July 2010 (2010-07-07) , pages 8862-8863, XP055044860, ISSN: 0002-7863, DOI: 10.1021/ja1035286
- FEI LI ET AL: "Biscinchona alkaloids as highly efficient bifunctional organocatalysts for the asymmetric conjugate addition of malonates to nitroalkenes at ambient temperature", TETRAHEDRON, vol. 67, no. 52, 27 August 2011 (2011-08-27), pages 10186-10194, XP055044857, ISSN: 0040-4020, DOI: 10.1016/j.tet.2011.08.070
- HIROYUKI KAWAI ET AL: "Organocatalytic Asymmetric Synthesis of Trifluoromethyl-substituted Diarylpyrrolines: Enantioselective Conjugate Cyanation of [beta]-Aryl-[beta]-trifluoromethyl-disubst ituted Enones", ANGEWANDTE CHEMIE INTERNATIONAL EDITION, vol. 51, no. 20, 14 May 2012 (2012-05-14), pages 4959-4962, XP055044598, ISSN: 1433-7851, DOI: 10.1002/anie.201201278
- HIROYUKI KAWAI ET AL: "Catalytic enantioselective synthesis of [beta]-trifluoromethyl pyrrolines", CHEMICAL COMMUNICATIONS, vol. 48, no. 34, 1 April 2012 (2012-04-01) , pages 4067-4069, XP055044699, ISSN: 1359-7345, DOI: 10.1039/c2cc18049a
- KAZUTAKA MATOBA ET AL: "Enantioselective Synthesis of Trifluoromethyl-Substituted 2-Isoxazolines: Asymmetric Hydroxylamine/Enone Cascade Reaction", ANGEWANDTE CHEMIE INTERNATIONAL EDITION, vol. 49, no. 33, 2 August 2010 (2010-08-02), pages 5762-5766, XP055003070, ISSN: 1433-7851, DOI: 10.1002/anie.201002065

## Description

The present invention relates to the synthesis of intermediates useful for the preparation of substituted pyrrolidine derivatives, including those having pesticidal activity. The invention relates more particularly to the stereoselective syntheses of these intermediates

Certain pyrrolidine derivatives with insecticidal properties are disclosed in, for example WO2008/128711, WO2010/043315, WO2011/080211. Such pyrrolidine derivatives include at least one chiral centre at one of the ring members of the pyrrolidine moiety. Various intermediates in the synthesis
of such derivatives are disclosed, for example, in Yuta et al, J.Amer. Chem. Soc. 132, 26, (2010) 8862-8863, Fei et al, Tetrahedron, 67, 52 (2011) 10186-10194, Hiroyuki et al, Angewandte Chemie Int. Ed., 51, 20 (2012) 4959-4962, Hiroyuki et al, Chemical Communications, 48, 34, (2012) 4067-4069, Kazutaka et al, Angewandte Chemie Int. Ed., 49, 33, (2010) 5762-5766, EP2230230 A1, WO2011/128299 A1, WO 2011/154555 A1, WO 2012/045700 A1 and WO 2011/080211 A1.

The present invention provides a process for selectively synthesizing enantiomers of such pyrrolidine derivatives. Also described herein are intermediates that can be used in the synthesis of such derivatives. A route to enantio-enriched intermediates is desirable in view of the differential biological activity of the enantiomers of such pyrrolidine derivatives. Use of enantio-enriched intermediates can therefore reduce the amount of active ingredient needed to control key pests, thereby reducing costs and impact on the environment.

Accordingly, a first aspect the invention provides a pyrrolidine derivative of formula VI 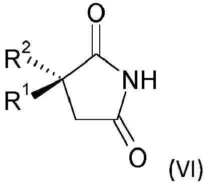 wherein
R¹ is chlorodifluoromethyl or trifluoromethyl;
R² is aryl or heteroaryl, each optionally substituted by one to five Q¹; and each Q¹ is independently selected from hydrogen, halogen, cyano, C₁-C₈alkyl, C₁-C₈haloalkyl, C₁-C₈alkoxy and C₁-C₈haloalkoxy.

According to the second aspect of the invention pyrrolidine derivative of formula VI can be made by a process comprising the steps of:
(a-i) reacting a compound of formula Ia 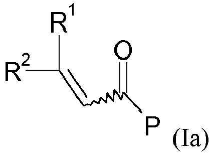 wherein
   P is alkyl, aryl or heteroaryl, each optionally substituted, wherein the heteroaryl is connected at P via a ring carbon atom;
   R¹ is chlorodifluoromethyl or trifluoromethyl;
   R² is aryl or heteroaryl, each optionally substituted; with a source of cyanide in the presence a chiral catalyst to give a compound of formula IIa 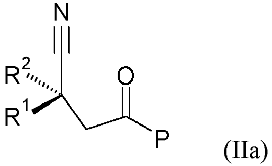 wherein P, R¹ and R² are as defined for the compound of formula Ia; and
(a-ii) oxidising the compound of formula IIa with a peroxy acid, or peroxide in the presence of an acid, preferably a strong acid., to give a compound of formula VI 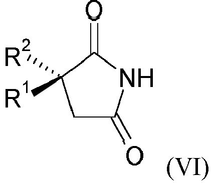 wherein R¹ and R² are as defined for the compound of formula Ia.

The ability to prepare compounds of formula VI from compounds of formula IIa via the Baeyer-Villiger oxidation reaction was unexpected and provides an efficient route to enantio-enriched pyrrolidine derivatives, and can also be applied to reactions with racemic mixtures.

In a further aspect the invention provides a process for the enantio-selective preparation of a pyrrolidine derivative of formula VI comprising the steps of:
(b-i) reacting a compound of formula Ia 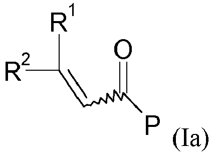 wherein
   P is optionally substituted heteroaryl, and wherein the heteroaryl contains at least one ring nitrogen or oxygen atom, wherein the heteroaryl is connected at P via a ring carbon atom;
   R¹ is chlorodifluoromethyl or trifluoromethyl; and
   R² is aryl or heteroaryl, each optionally substituted; with a source of cyanide in the presence a chiral catalyst to give a compound of formula IIb 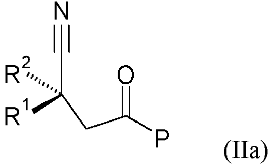 wherein P, R¹ and R² are as defined for the compound of formula Ia; an
(b-ii-1) oxidatively cleaving the compound of formula IIb to give a compound of formula XIX 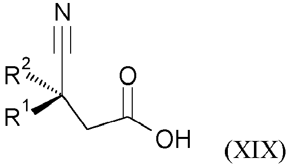 wherein R¹ and R² are as defined for the compound of formula Ia; and
(b-ii-2) hydrolysing and dehydrating the compound of formula XIX. to give a compound of formula VI 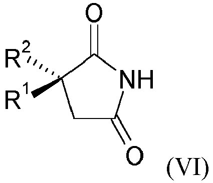 wherein R¹ and R² are as defined for the compound of formula Ia; wherein dehydration is performed in the presence of acid.

In a further aspect the invention provides a process for the enantio-selective preparation of a pyrrolidine derivative of formula VI: comprising the steps of:
(c-iii-1) partially hydrolysing the compound of formula IIb to give a compound of formula V 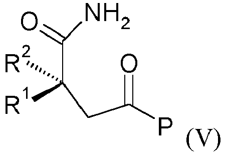 wherein P, R¹ and R² are as defined for the compound of formula IIb; and
(c-iii-2) cyclising the compound of formula V, e.g. by heating, to give a compound of formula VI 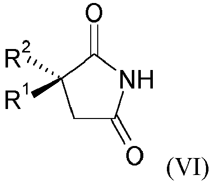 wherein R¹ and R² are as defined for the compound of formula I; or
(c-iv-1) hydrolysing the compound of formula IIb to give a compound of formula VII 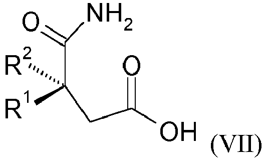 wherein R¹ and R² are as defined for the compound of formula IIb and
(c-iv-2) cyclising the compound of formula VII, e.g. by heating, to give a compound of formula VI 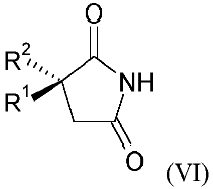 wherein R¹ and R² are as defined for the compound of formula Ia.

Preferred substituent definitions are given below.

Also described herein is a mixture comprising a compound of formula VI and a compound of formula VIA 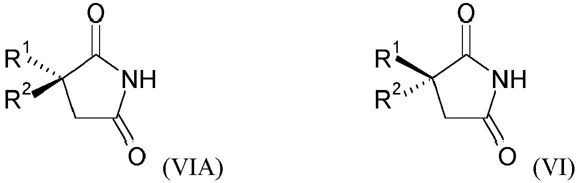 wherein
R¹ is chlorodifluoromethyl or trifluoromethyl;
R² is aryl or heteroaryl, each optionally substituted
by one to five Q¹; and
each Q¹ is independently selected from hydrogen, halogen, cyano, C₁-C₈alkyl, C₁-C₈haloalkyl, C₁-C₈alkoxy and C₁-C₈haloalkoxy;
wherein the mixture is enriched for the compound of formula VI.

In a further aspect the invention provides a process for preparing pyrrolidine derivatives comprising the steps of:
(a-i) reacting a compound of formula Ia 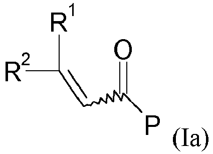 wherein
   P is alkyl, aryl or heteroaryl, each optionally substituted, wherein the heteroaryl is connected at P via a ring carbon atom;
   R¹ is chlorodifluoromethyl or trifluoromethyl;
   R² is aryl or heteroaryl, each optionally substituted by one to five Q¹; and
   each Q¹ is independently selected from hydrogen, halogen, cyano, C₁-C₈alkyl, C₁-C₈haloalkyl, C₁-C₈alkoxy and C₁-C₈haloalkoxy;
   with a source of cyanide to give a compound of formula IIa 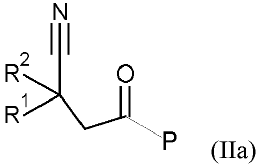 wherein P, R¹ and R² are as defined for the compound of formula Ia; and
(a-ii) oxidising the compound of formula IIa with a peroxide in the presence of strong acid to give a compound of formula VI 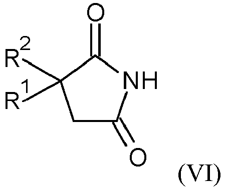 wherein R¹ and R² are as defined for the compound of formula Ia.

The cyanide addition can be done in presence of a base and /or a catalyst. Examples of bases include triethyl amine, sodium carbonate, potassium carbonate, sodium hydroxide, potassium hydroxide. Examples of chiral catalysts include crown ethers and phase transfer catalysts such as tetrabutylammonium bromide.

In enantiomerically enriched mixtures as described herein, the molar proportion of the enriched compound in the mixture compared to the total amount of both enantiomers is for example greater than 50%, e.g. at least 55, 60, 65, 70, 75, 80, 85, 90, 95, 96, 97, 98, or at least 99%.

Alkyl groups (either alone or as part of a larger group, such as alkoxy-, alkylthio-, alkylsulfinyl-, alkylsulfonyl-, alkylcarbonyl- or alkoxycarbonyl-) can be in the form of a straight or branched chain and are, for example, methyl, ethyl, propyl, prop-2-yl, butyl, but-2-yl, 2-methyl-prop-1-yl or 2-methyl-prop-2-yl. The alkyl groups are, unless indicated to the contrary, preferably C₁-C₆, more preferably C₁-C₄, most preferably C₁-C₃ alkyl groups.

Alkylene groups can be in the form of a straight or branched chain and are, for example, - CH₂-, -CH₂-CH₂-, -CH(CH₃)-, -CH₂-CH₂-CH₂-, -CH(CH₃)-CH₂-, or -CH(CH₂CH₃)-. The alkylene groups are, unless indicated to the contrary, preferably C₁-C₆, more preferably C₁-C₃, more preferably C₁-C₂, most preferably C₁ alkylene groups.

Alkenyl groups can be in the form of straight or branched chains, and can be, where appropriate, of either the (E)- or (Z)-configuration. Examples are vinyl and allyl. The alkenyl groups are, unless indicated to the contrary, preferably C₂-C₆, more preferably C₂-C₄, most preferably C₂-C₃ alkenyl groups.

Alkynyl groups can be in the form of straight or branched chains. Examples are ethynyl and propargyl. The alkynyl groups are, unless indicated to the contrary, preferably C₂-C₆, more preferably C₂-C₄, most preferably C₂-C₃ alkynyl groups.

Halogen is fluorine, chlorine, bromine or iodine.

Haloalkyl groups (either alone or as part of a larger group, such as haloalkoxy-, haloalkylthio-, haloalkylsulfinyl-, haloalkylsulfonyl-, haloalkylcarbonyl- or haloalkoxycarbonyl-) are alkyl groups which are substituted by one or more of the same or different halogen atoms and are, for example, difluoromethyl, trifluoromethyl, chlorodifluoromethyl or 2,2,2-trifluoroethyl.

Haloalkenyl groups are alkenyl groups which are substituted by one or more of the same or different halogen atoms and are, for example, 2,2-difluoro-vinyl or 1,2-dichloro-2-fluorovinyl.

Haloalkynyl groups are alkynyl groups which are substituted by one or more of the same or different halogen atoms and are, for example, 1-chloro-prop-2-ynyl.

Cycloalkyl groups can be in mono- or bi-cyclic form and are, for example, cyclopropyl, cyclobutyl, cyclohexyl and bicyclo[2.2.1]heptan-2-yl. The cycloalkyl groups are, unless indicated to the contrary, preferably C₃-C₈, more preferably C₃-C₆ cycloalkyl groups.

Aryl groups are aromatic ring systems which can be in mono-, bi- or tricyclic form. Examples of such rings include phenyl, naphthyl, anthracenyl, indenyl or phenanthrenyl. Preferred aryl groups are phenyl and naphthyl, phenyl being most preferred. Where an aryl moiety is said to be substituted, the aryl moiety is, unless indicated to the contrary, preferably substituted by one to four substituents, most preferably by one to three substituents.

Heteroaryl groups are aromatic ring system containing at least one heteroatom and consisting either of a single ring or of two or more fused rings. Preferably, single rings will contain up to three heteroatoms and bicyclic systems up to four heteroatoms which will preferably be chosen from nitrogen, oxygen and sulfur. Examples of monocyclic groups include pyridyl, pyridazinyl, pyrimidinyl, pyrazinyl, pyrrolyl, pyrazolyl, imidazolyl, triazolyl, furanyl, thiophenyl, oxazolyl, isoxazolyl, oxadiazolyl, thiazolyl, isothiazolyl and thiadiazolyl. Examples of bicyclic groups include quinolinyl, cinnolinyl, quinoxalinyl, indolyl, indazolyl, benzimidazolyl, benzothiophenyl, benzothiazolyl and benzotriazolyl. Monocyclic heteroaryl groups are preferred, pyridyl being most preferred. Where a heteroaryl moiety is said to be substituted, the heteroaryl moiety is, unless indicated to the contrary, preferably substituted by one to four substituents, most preferably by one to three substituents.

Heterocyclyl groups are defined to include heteroaryl groups and in addition their unsaturated or partially unsaturated analogues. Examples of monocyclic groups include thietanyl, pyrrolidinyl, tetrahydrofuranyl, [1,3]dioxolanyl, piperidinyl, piperazinyl, [1,4]dioxanyl, and morpholinyl or their oxidised versions such as 1-oxo-thietanyl and 1,1-dioxo-thietanyl. Examples of bicyclic groups include 2,3-dihydro-benzofuranyl, benzo[1,3]dioxolanyl, and 2,3-dihydro-benzo[1,4]dioxinyl. Where a heterocyclyl moiety is said to be substituted, the heterocyclyl moiety is, unless indicated to the contrary, preferably substituted by one to four substituents, most preferably by one to three substituents.
Unless stated otherwise where gruops are optionally substituted they may be substituted e.g. by one to five groups, e.g. by one to three groups, preferably independently selected from nitro, cyano, hydroxy, halogen, mercapto, isocyano, cyanate, isothiocyanate, carboxy, carbamoyl, aminosulfonyl, monoalkylamino, dialkylamino, N-alkylcarbonylamino, alkyl, alkenyl, alkynyl, cycloalkyl, cycloalkenyl, SF₅, alkoxy, alkenyloxy, alkynyloxy, cycloalkyloxy, cycloalkenyloxy, alkoxy- carbonyl, alkenyloxycarbonyl, alkynyloxycarbonyl, aryloxycarbonyl, alkylcarbonyl, alkenylcarbonyl, alkynylcarbonyl, arylcarbonyl, alkylthio, cycloalkylthio, alkenylthio, cycloalkenylthio, alkynylthio, alkylsulfenyl, alkylsulfinyl including isomers, alkylsulfonyl, monoalkylaminosulfonyl, dialkylaminosulfonyl, alkylphosphinyl, alkylphosphonyl, alkylphosphinyl including isomers, alkylphosphonyl including isomers, N-alkyl-aminocarbonyl, -dialkyl-aminocarbonyl, N-alkylcarbonyl-aminocarbonyl, N-alkylcarbonyl-N-alkylaminocarbonyl, aryl, aryloxy, benzyl, benzyloxy, benzylthio, arylthio, arylamino, benzylamino, trialkylsilyl, alkoxyalkyl, alkylthioalkyl, alkylthioalkoxy, alkoxyalkoxy, phenethyl, benzyloxy, haloalkyl, haloalkoxy, haloalkylthio, haloalkylcarbonyl, haloalkoxycarbonyl, haloalkoxyalkoxy, haloalkoxyalkylthio, haloalkoxyalkylcarbonyl or haloalkoxyalkyl, cycloalkylamino-carbonyl, alkylsulfinylimino, alkylsulfonylimino, alkoxyimino, and a heterocyclic group;preferably nitro, cyano, hydroxy, mercapto, isocyano, cyanate, isothiocyanate, carboxy, carbamoyl, aminosulfonyl, mono-C₁-C₁₂alkylamino, di-C₂-C₂₄alkylamino, N-C₁-C₁₂alkylcarbonylamino, C₁-C₁₂alkyl, C₂-C₆alkenyl, C₂-C₆alkynyl, C₃-C₈cycloalkyl, C₃-C₈cycloalkenyl, SF₅, C₁-C₁₂alkoxy, C₂-C₆alkenyloxy, C₂-C₆alkynyloxy, C₃-C₈cycloalkyloxy, C₃-C₈cycloalkenyloxy, C₁-C₁₂alkoxycarbonyl, C₂-C₆alkenyloxycarbonyl, C₂-C₆alkynyloxycarbonyl, aryloxycarbonyl, C₁-C₁₂alkylcarbonyl, C₂-C₆alkenylcarbonyl, C₂-C₆alkynylcarbonyl, arylcarbonyl, C₁-C₁₂alkylthio, C₃-C₈cycloalkylthio, C₂-C₆alkenylthio, C₃-C₈cycloalkenylthio, C₂-C₆alkynylthio, C₁-C₁₂alkylsulfenyl, C₁-C₁₂alkylsulfinyl including isomers, C₁-C₁₂alkylsulfonyl, mono-C₁-C₁₂alkylaminosulfonyl, di-C₂-C₂₄alkylaminosulfonyl, C₁-C₁₂alkylphosphinyl, C₁-C₁₂alkylphosphonyl, C₁-C₁₂alkylphosphinyl including isomers, C₁-C₁₂alkylphosphonyl including isomers, N-C₁-C₁₂alkyl-aminocarbonyl, -di-C₂-C₂₄alkyl-aminocarbonyl, N-C₁-C₁₂alkylcarbonyl-aminocarbonyl, N-C₁-C₁₂alkylcarbonyl-N-C₁-C₁₂alkylaminocarbonyl, aryl, aryloxy, benzyl, benzyloxy, benzylthio, arylthio, arylamino, benzylamino, trialkylsilyl, C₁-C₁₂alkoxyalkyl, C₁-C₁₂alkylthioalkyl, C₁-C₁₂alkylthioalkoxy, C₁-C₁₂alkoxyalkoxy, phenethyl, benzyloxy, C₁-C₁₂haloalkyl, C₁-C₁₂haloalkoxy, C₁-C₁₂haloalkylthio, C₁-C₁₂haloalkylcarbonyl, C₁-C₁₂haloalkoxycarbonyl, C₁-C₁₂haloalkoxyalkoxy, C₁-C₁₂haloalkoxyC₁-C₁₂alkylthio, C₁-C₁₂haloalkoxyC₁-C₁₂alkylcarbonyl or C₁-C₁₂haloalkoxy-C₁-C₁₂alkyl, C₃-C₈cycloalkylamino-carbonyl, C₁-C₁₂alkylsulfinylimino, C₁-C₁₂alkylsulfonylimino, C₁-C₁₂alkoxyimino, and a heterocyclic group, wherein aryl is phenyl and heterocyclic groups are heteroaryl groups as defined above. Preferred optional substituents are cyano, halogen, C₁-C₄alkyl, C₁-C₄haloalkyl, C₁-C₄alkoxy, and C₁-C₄haloalkoxy.

Bearing in mind the stereocentre which is the subject of the invention, the invention otherwise includes all isomers of compounds of formula I, salts and N-oxides thereof, including enantiomers, diastereomers and tautomers. Tautomers of the compounds of formula I include the enamine form, for example. These are covered by the invention.

Preferred substituent values in compounds of formula I are as follows, which may be combined in any order. These preferred substituent values also apply to other compounds of the invention in which the same substituents are present.

Preferably R¹ is trifluoromethyl.

Preferably R² is aryl or aryl substituted by one to five Q¹, or heteroaryl or heteroaryl substituted by one to five Q¹. Preferably R² is group A 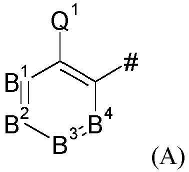 wherein B¹, B², B³, B⁴ and Q¹ are as defined below. More preferably R² is group A1 or A2 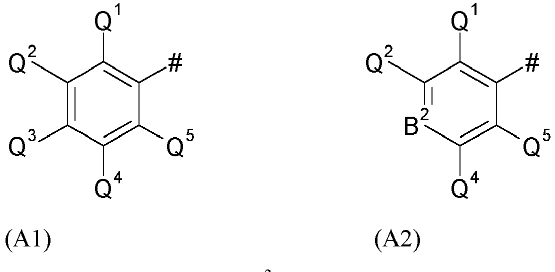 More preferably R² is group A3 or A4

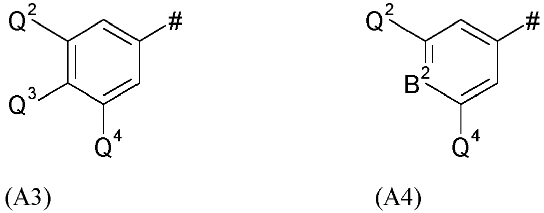

B¹, B², B³, B⁴ are independently C-Q¹ or nitrogen.

Q¹, Q², Q³, Q⁴, and Q⁵ are independently hydrogen , halogen, cyano, nitro, C₁-C₈alkyl, C₁-C₈haloalkyl, C₂-C₈alkenyl, C₂-C₈haloalkenyl, C₂-C₈alkynyl, C₂-C₈haloalkynyl, hydroxy, C₁-C₈alkylamino, C₁-C₈alkoxy, C₁-C₈haloalkoxy, mercapto, C₁-C₈alkylthio, C₁-C₈haloalkylthio, C₁-C₈alkylsulfinyl, C₁-C₈haloalkylsulfinyl, C₁-C₈alkylsulfonyl, C₁-C₈haloalkylsulfonyl, C₁-C₈alkylcarbonyl, C₁-C₈alkoxycarbonyl, optionally substituted aryl or optionally substituted heterocyclyl. Preferably, Q¹, Q², Q³, Q⁴, and Q⁵ are each independently hydrogen, halogen, cyano, C₁-C₈alkyl, C₁-C₈haloalkyl, C₁-C₈alkoxy or C₁-C₈haloalkoxy, more preferably bromo, chloro, fluoro, cyano, methyl, trifluoromethyl, methoxy or trifluoromethoxy, preferably bromo, chloro or trifluoromethyl. Preferably at least two of Q¹, Q², Q³, Q⁴, and Q⁵ are not hydrogen.

When P is defined as hydroxy, alkoxy, aryloxy, alkylsulfinyl, arylsulfinyl or heteroaryl, each optionally substituted, and wherein the heteroaryl contains at least one ring nitrogen atom, and the heteroaryl is connected at P via a ring nitrogen atom, then P is preferably hydroxy, C₁-C₁₂alkoxy, C₁-C₁₂haloalkoxy phenyloxy, C₁-C₁₂sulfinyl, phneylsulfinyl or heteroaryl, wherein phenyl (including phenyloxy) and heteroaryl are optionally substituted by one to five groups independently selected from cyano, halogen, C₁-C₄alkyl, C₁-C₄haloalkyl, C₁-C₄alkoxy, and C₁-C₄haloalkoxy, and heteroaryl is pyridyl, pyridazinyl, pyrimidinyl, pyrazinyl, pyrrolyl, pyrazolyl, imidazolyl, triazolyl, furanyl, thiophenyl, oxazolyl, isoxazolyl, oxadiazolyl, thiazolyl, isothiazolyl and thiadiazolyl,quinolinyl, cinnolinyl, quinoxalinyl, indolyl, indazolyl, benzimidazolyl, benzothiophenyl, benzothiazolyl or benzotriazolyl, more preferably P is hydroxyl, C₁-C₆alkoxy, N-pyrrolyl, N-imidazolyl, N-1,2-4-triazolyl, N-benzotriazolyl, or C₁-C₆alkylsulfinyl.

When P is defined as alkyl, aryl or heteroaryl, each optionally substituted (and e.g. wherein the heteroaryl is connected to at P via a ring carbon atom), then preferably P is C₁-C₁₂alkyl, C₁-C₁₂haloalkyl, phenyl or heteroaryl, and heteroaryl is pyridyl, pyridazinyl, pyrimidinyl, pyrazinyl, pyrrolyl, pyrazolyl, imidazolyl, triazolyl, furanyl, thiophenyl, oxazolyl, isoxazolyl, oxadiazolyl, thiazolyl, isothiazolyl and thiadiazolyl,quinolinyl, cinnolinyl, quinoxalinyl, indolyl, indazolyl, benzimidazolyl, benzothiophenyl, benzothiazolyl or benzotriazolyl, e.g. wherein phenyl and heteroaryl are each optinally substituted by one to five groups independently selected from cyano, halogen, C₁-C₄alkyl, C₁-C₄haloalkyl, C₁-C₄alkoxy and C₁-C₄haloalkoxy.

When P is defined as optionally substituted heteroaryl, and wherein the heteroaryl contains at least one ring nitrogen or oxygen atom, wherein the heteroaryl is connected at P via a ring carbon atom, then preferably P is pyridyl, pyridazinyl, pyrimidinyl, pyrazinyl, pyrrolyl, pyrazolyl, imidazolyl, triazolyl, furanyl, thiophenyl, oxazolyl, isoxazolyl, oxadiazolyl, thiazolyl, isothiazolyl and thiadiazolyl,quinolinyl, cinnolinyl, quinoxalinyl, indolyl, indazolyl, benzimidazolyl, benzothiophenyl, benzothiazolyl or benzotriazolyl, each optinally substituted by ne to five groups independently selected from cyano, halogen, C₁-C₄alkyl, C₁-C₄haloalkyl, C₁-C₄alkoxy, and C₁-C₄haloalkoxy.

When P is defined as P is alkyl, hydroxy, alkoxy, aryloxy, alkylsulfinyl, or arylsulfinyl, each optionally substituted, then preferably P is C₁-C₁₂alkyl, C₁-C₁₂haloalkyl, hydroxy, C₁-C₁₂alkoxy, C₁-C₁₂haloalkoxy, phenyloxy, C₁-C₁₂sulfinyl, phenylsulfinyl, wherein phenyl is optionally substituted by one to five groups independently selected from cyano, halogen, C₁-C₄alkyl, C₁-C₄haloalkyl, C₁-C₄alkoxy, and C₁-C₄haloalkoxy.

Preferably A' is selected from P1 to P6

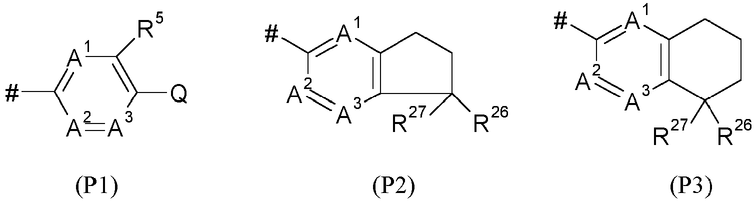

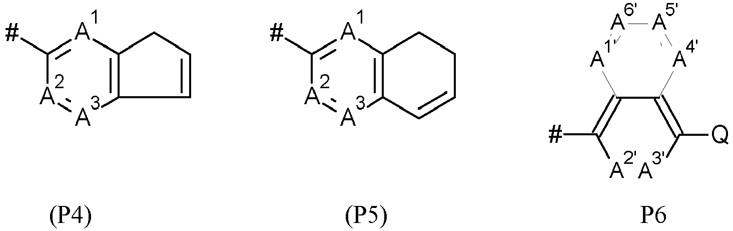

In one group of compounds A' is P1. In another group of compounds A' is P2. In another group of compounds A' is P3. In another group of compounds A' is P4. In another group of compounds A' is P5. In another group of compounds A' is P6. In another group of compounds A' is selected from P3 to P5. When P is P2 to P5, P is preferably P7 to P22

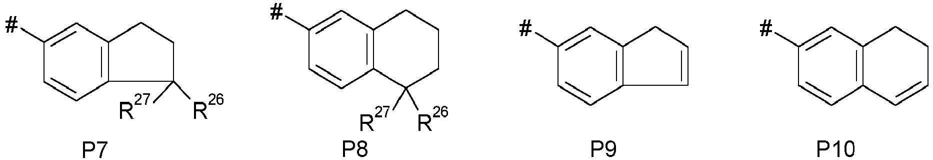

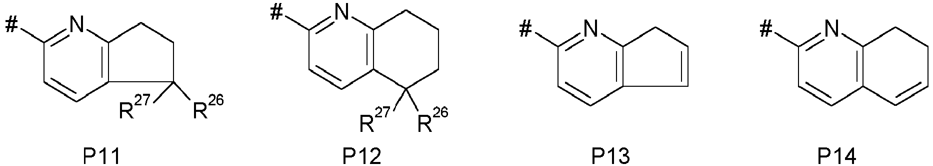

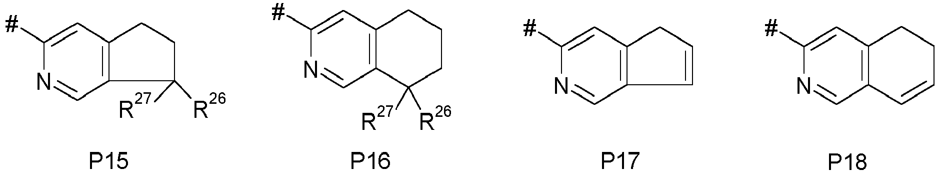

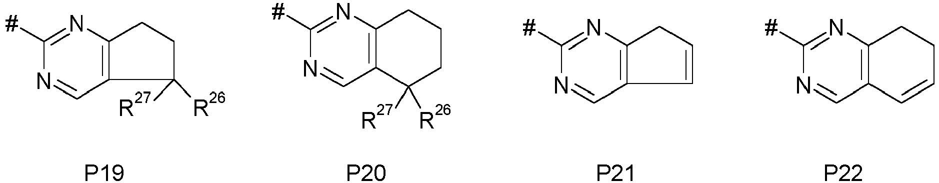

A¹, A² and A³ are independently of each other C-H, C-R⁵, or nitrogen. Preferably no more than two of A¹, A² and A³ are nitrogen. In one group of compounds A¹, A² and A³ are each C-R⁵. In one group of compounds A¹ is nitrogen and A² and A³ are both C-R⁵. In another group of compounds A² is nitrogen and A¹ and A³ are both C-R⁵. In another group of compounds A¹ and A² are both nitrogen and A³ is C-R⁵. In one group of compounds A¹, A² and A³ are each C-H. In one group of compounds A¹ is nitrogen and A² and A³ are both C-H. In another group of compounds A² is nitrogen and A¹ and A³ are both C-H. In another group of compounds A¹ and A² are both nitrogen and A³ is C-H. Preferably A¹, A² and A³ are each C-H.

A^{1'}, A^{2'}, A^{3'}, A^{4'}, A^{5'} and A^{6'} are independently of each other C-H, C-R⁵ or nitrogen provided that no more than two of A^{1'}, A^{2'}, A^{3'}, A^{4'}, A^{5'} and A^{6'} are nitrogen. Preferably A^{1'}, A^{2'}, A^{3'},A^{4'}, A^{5'} and A^{6'} are C-H.

The ring formed by A¹, A², and A³, or A^{1'}, A^{2'}, A^{3'}, A^{4'}, A^{5'} and A^{6'} may, for example, be phenyl, pyridyl, pyrimidine, pyrazine, pyridazine, naphthyl or quinoline. Each R⁵ is independently halogen, cyano, nitro, C₁-C₈alkyl, C₁-C₈haloalkyl, C₂-C₈alkenyl, C₂-Cshaloalkenyl, C₂-C₈alkynyl, C₂-C₈haloalkynyl, C₃-C₁₀cycloalkyl, C₁-C₈alkoxy, C₁-C₈haloalkoxy, C₁-C₈alkylthio, C₁-C₈haloalkylthio, C₁-C₈alkylsulfinyl, C₁-C₈haloalkylsulfinyl, C₁-C₈alkylsulfonyl or C₁-C₈haloalkylsulfonyl. Preferably, each R⁵ is independently halogen, C₁-C₈alkyl, C₁-C₈haloalkyl or C₂-C₈alkenyl. More preferably, each R⁵ is independently bromo, chloro, fluoro, methyl, trifluoromethyl or vinyl, most preferably each R⁵ is methyl.

Q is hydrogen, halogen, nitro, NH₂, cyano, C₁-C₈alkyl, C₁-C₈haloalkyl, C₂-C₈alkenyl, C₂-C₈haloalkenyl, C₂-C₈alkynyl, C₃-C₈haloalkynyl, C₃-C₁₀cycloalkyl, C₁-C₈alkylthio, C₁-C₈haloalkylthio, C₁-C₈alkylsulfinyl, C₁-C₈haloalkylsulfinyl, C₁-C₈alkylsulfonyl, C₁-C₈haloalkylsulfonyl, arylsulfonyl or arylsulfonyl substituted by one to five groups independently selected from C₁-C₄alkyl and nitro, -N(R⁶)R^{7b}, -C(=W⁵)N(R⁶)R⁷, -C(R¹⁵)(R¹⁶)N(R¹⁷)R¹⁸, - C(=W⁵)OR^{7a}, -C(=W⁵)R¹³, -OR¹⁴, aryl or aryl substituted by one to five Z¹, heterocyclyl or heterocyclyl substituted by one to five Z¹. Preferably, Q is cyano, halogen, nitro, NH₂, arylsulfonyl or arylsulfonyl substituted by one to five groups independently selected from C₁-C₄alkyl and nitro, heterocyclyl or heterocyclyl substituted by one to five Z¹, -OR¹⁴, - C(=O)N(R⁶)R⁷, -C(=O)OR^{7a}, -C(=O)R¹³, or -C(R¹⁵)(R¹⁶)N(R¹⁷)R¹⁸. More preferably, Q is cyano, halogen, nitro, NH₂, phenylsulfonyl or phenylsulfonyl substituted by one to five groups independently selected from C₁-C₄alkyl and nitro, -OR¹⁴, -C(=O)N(R⁶)R⁷, -C(=O)OR^{7a} , -C(=O)R¹³, -C(R¹⁵)(R¹⁶)N(R¹⁷)R¹⁸, or a heterocycle selected from H1 to H9

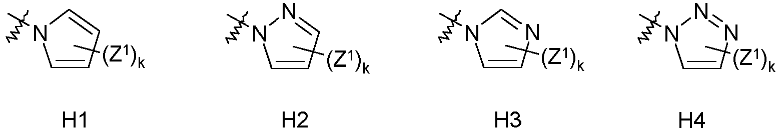

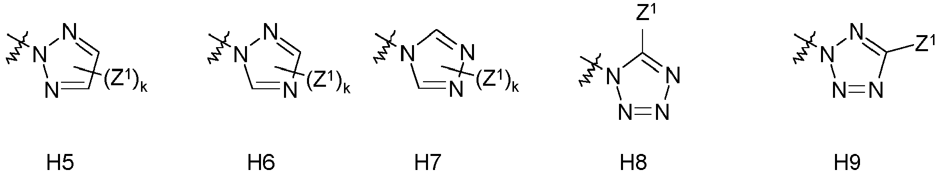

Even more preferably, Q is cyano, halogen, nitro, NH₂, C₁-C₈alkoxy, phenylsulfonyl or phenylsulfonyl substituted by one to five groups independently selected from C₁-C₄ alkyl and nitro, -C(=O)N(R⁶)R⁷, -C(=O)OR^{7a}, -C(=O)R¹³, -C(R¹⁵)(R¹⁶)N(R¹⁷)R¹⁸, or a heterocycle selected from H1 to H9.

k is 0, 1, or 2, preferably 0.

R⁶ is hydrogen, C₁-C₈alkyl, C₁-C₈alkoxy, C₂-C₈alkenyl, C₂-C₈alkynyl, C₃-C₁₀cycloalkyl, C₃-C₁₀cycloalkyl-C₁-C₄alkylene, C₁-C₈alkylcarbonyl or C₁-C₈alkoxycarbonyl. Preferably, R⁶ is hydrogen, C₁-C₈alkyl, C₁-C₈alkoxy, C₁-C₈alkylcarbonyl, or C₁-C₈alkoxycarbonyl. More preferably, R⁶ is hydrogen, methyl, ethyl, methylcarbonyl or methoxycarbonyl, more preferably hydrogen, methyl or ethyl, most preferably hydrogen.

R⁷ is hydrogen, alkyl or alkyl substituted by one to five R⁸, alkenyl or alkenyl substituted by one to five R⁸, alkynyl or alkynyl substituted by one to five R⁸, C₃-C₁₀cycloalkyl or C₃-C₁₀cycloalkyl substituted by one to five R⁹, C₃-C₁₀cycloalkyl-C₁-C₄alkylene or C₃-C₁₀cycloalkyl-C₁-C₄alkylene wherein the cycloalkyl moiety is substituted by one to five R⁹, C₁-C₈alkyl-N(R⁶)-C(=O)-C₁-C₄alkylene, C₁-C₈haloalkyl-N(R⁶)-C(=O)-C₁-C₄alkylene, C₃-C₈cycloalkyl-aminocarbonyl-C₁-C₄alkylene, C₁-C₆alkyl-O-N=CH-, C₁-C₆haloalkyl-O-N=CH-, aryl-C₁-C₆alkylene or aryl-C₁-C₆alkylene wherein the aryl moiety is substituted by one to five R¹⁰, heterocyclyl-C₁-C₆alkylene or heterocyclyl-C₁-C₆alkylene wherein the heterocyclyl moiety is substituted by one to five R¹⁰ and wherein each heterocyclyl moiety contains one or more ring members independently selected from O, N, C=O, C=N-OR¹², N-R¹², S, SO, SO₂, S=N-R¹² and SO=N-R¹², aryl or aryl substituted by one to five R¹⁰, heterocyclyl or heterocyclyl substituted by one to five R¹⁰ and wherein each heterocyclyl moiety contains one or more ring members independently selected from O, N, C=O, C=N-OR¹², N-R¹², S, SO, SO₂, S=N-R¹² and SO=N-R¹². Preferably, R⁷ is hydrogen, C₁-C₈alkyl or C₁-C₈alkyl substituted by one to five R⁸, C₃-C₁₀cycloalkyl or C₃-C₁₀cycloalkyl substituted by one to five R⁹, aryl-C₁-C₆alkylene or aryl-Ci-C₆alkylene wherein the aryl moiety is substituted by one to five R¹⁰, heterocyclyl-C₁-C₆alkylene or heterocyclyl-C₁-C₆alkylene wherein the heterocyclyl moiety is substituted by one to five R¹⁰ and wherein each heterocyclyl moiety contains one or more ring members independently selected from O, N, C=O, C=N-OR¹², N-R¹², S, SO, SO₂, S=N-R¹² and SO=N-R¹², aryl or aryl substituted by one to five R¹⁰, heterocyclyl or heterocyclyl substituted by one to five R¹⁰ and wherein each heterocyclyl moiety contains one or more ring members independently selected from O, N, C=O, C=N-OR¹², N-R¹², S, SO, SO₂, S=N-R¹² and SO=N-R¹², C₁-C₈alkyl-N(R⁶)-C(=O)-C₁-C₄ alkylene, C₁-C₈haloalkyl-N(R⁶)-C(=O)-C₁-C₄alkylene, C₃-C₈cycloalkylaminocarbonyl-C₁-C₄alkylene, C₁-C₆alkyl-O-N=CH-, or C₁-C₆haloalkyl-O-N=CH-. More preferably, R⁷ is hydrogen, C₁-C₈alkyl, C₁-C₈haloalkyl, phenyl-C₁-C₆alkylene or phenyl-C₁-C₆alkylene wherein the phenyl moiety is substituted by one to five R¹⁰, pyridyl-C₁-C₆alkylene or pyridyl-C₁-C₆alkylene wherein the pyridyl moiety is substituted by one to four R¹⁰, thiazolyl-C₁-C₆alkylene or thiazolyl-C₁-C₆alkylene wherein the thiazolyl moiety is substituted by one or two R¹⁰, phenyl or phenyl substituted by one to five R¹⁰, pyridyl or pyridyl substituted by one to four R¹⁰, thiazolyl or thiazolyl substituted by one or two R¹⁰, C₃-C₆cycloalkyl or C₃-C₆cycloalkyl wherein one ring atom is replaced by O or S, C₁-C₄alkyl-O-N=CH-, C₁-C₄haloalkyl-O-N=CH-, C₁-C₄alkyl-N(R⁶)-C(=O)-CH₂-, C₁-C₄haloalkyl-N(R⁶)-C(=O)-CH₂-, or a group of formula (Y)

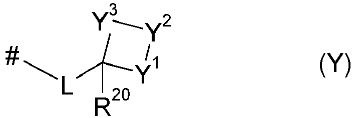

In one group of compounds R⁷ is not a group of formula (Y)

L is a single bond or C₁-C₆alkylene;
Y¹, Y² and Y³ are independently of another O, CR¹¹R¹², C=O, C=N-OR¹², N-R¹², S, SO, SO₂, S=N-R¹² or SO=N-R¹², provided that at least one of Y¹, Y² or Y³ is not CR²¹R²², C=O or C=N-OR¹². In the group of formula (Y), preferably two of Y¹, Y² and Y³ are CR²¹R²², and the other is O, N-R¹², S, SO, SO₂, S=N-R¹² or SO=N-R¹², more preferably two of Y¹, Y² and Y³ are CH₂ and the other is S, SO or SO₂. When L is a bond Y¹ and Y³ are preferably CH₂ and Y² is S, SO, SO₂, S=N-R¹² or SO=N-R¹². When L is alkylene, Y¹ is preferably S, SO, SO₂, S=N-R¹² or SO=N-R¹² and Y² and Y³ are CH₂.

R^{7a} is hydrogen, alkyl or alkyl substituted by one to five R⁸, alkenyl or alkenyl substituted by one to five R⁸, alkynyl or alkynyl substituted by one to five R⁸, cycloalkyl or cycloalkyl substituted by one to five R⁹, aryl-alkylene or aryl-alkylene wherein the aryl moiety is substituted by one to five R¹⁰, heteroaryl-alkylene or heteroaryl-alkylene wherein the heteroaryl moiety is substituted by one to five R¹⁰, aryl or aryl substituted by one to five R¹⁰, or heteroaryl or heteroaryl substituted by one to five R¹⁰. Preferably, R^{7a} is hydrogen, C₁-C₁₅alkyl or C₁-C₁₅alkyl substituted by one to five R⁸, C₂-C₁₅alkenyl or C₂-C₁₅alkenyl substituted by one to five R⁸, C₂-C₁₅alkynyl or C₂-C₁₅alkynyl substituted by one to five R⁸, C₃-C₁₀cycloalkyl or C₃-C₁₀cycloalkyl substituted by one to five R⁹, aryl-C₁-C₆alkylene or aryl-C₁-C₆alkylene wherein the aryl moiety is substituted by one to five R¹⁰, heteroaryl-C₁-C₆alkylene or heteroaryl-C₁-C₆alkylene wherein the heteroaryl moiety is is substituted by one to five R¹⁰ , or heteroaryl or heteroaryl substituted by one to five R¹⁰. More preferably R^{7a} is hydrogen, C₁-C₁₅alkyl, C₁-C₁₅haloalkyl C₂-C₁₅alkenyl, C₂-C₁₅haloalkenyl, C₂-C₁₅alkynyl, C₂-C₁₅haloalkynyl, phenyl-C₁-C₄alkylene or phenyl-C₁-C₄alkylene wherein the phenyl moiety is substituted by one to five halogen, pyridyl-C₁-C₄alkyl or pyridyl-C₁-C₄alkyl wherein the pyridyl moiety is substituted by one to four halogen, pyridyl or pyridyl substituted by one to four R¹⁰, most preferably R^{7a} is C₁-C₁₅alkyl, C₁-C₁₅haloalkyl, C₂-C₁₅alkenyl, C₂-C₁₅haloalkenyl, pyridyl or benzyl.

R^{7b} is hydrogen, alkyl, haloalkyl, alkenyl, haloalkenyl, alkynyl, haloalkynyl cycloalkyl, halocycloalkyl, alkylcarbonyl, haloalkylcarbonyl, alkoxycarbonyl, haloalkoxycarbonyl, or benzyl, more preferably R^{7b} is hydrogen, C₁-C₁₅alkyl, C₁-C₁₅haloalkyl, C₂-C₁₅alkenyl, C₂-C₁₅haloalkenyl, C₂-C₁₅alkynyl, C₂-C₁₅haloalkynyl, C₃-C₁₀cycloalkyl, C₁-C₁₅alkylcarbonyl or C₁-C₁₅alkoxycarbonyl; most preferably R^{7b} is C₁-C₁₅alkyl, C₁-C₁₅haloalkyl, C₂-C₁₅ alkenyl or C₂-C₁₅haloalkenyl.

Each R⁸ is independently halogen, cyano, nitro, hydroxy, NH₂, mercapto, C₁-C₈alkyl, C₁-C₈haloalkyl, C₁-C₈alkoxy, C₁-C₈haloalkoxy, C₁-C₈alkylthio, C₁-C₈haloalkylthio, C₁-C₈alkylsulfinyl, C₁-C₈haloalkylsulfinyl, C₁-C₈alkylsulfonyl, C₁-C₈haloalkylsulfonyl, C₁-C₈alkylamino, C₂-C₈dialkylamino, C₃-C₈cycloalkylamino, C₁-C₈alkylcarbonyl, C₁-C₈alkoxycarbonyl, C₁-C₈alkylaminocarbonyl, C₁-C₈dialkylaminocarbonyl, C₁-C₈haloalkylcarbonyl, C₁-C₈haloalkoxycarbonyl, C₁-C₈haloalkylaminocarbonyl, C₁-C₈halodialkylaminocarbonyl. Preferably, each R⁸ is independently halogen, cyano, nitro, hydroxy, C₁-C₈alkoxy, C₁-C₈haloalkoxy, C₁-C₈alkylcarbonyl, C₁-C₈alkoxycarbonyl, mercapto, C₁-C₈alkylthio, C₁-C₈haloalkylthio, C₁-C₈alkylsulfinyl, C₁-C₈haloalkylsulfinyl, C₁-C₈alkylsulfonyl. More preferably, each R⁸ is independently halogen, cyano, nitro, hydroxy, C₁-C₈alkoxy, C₁-C₈haloalkoxy, mercapto, C₁-C₈alkylthio, C₁-C₈haloalkylthio, more preferably bromo, chloro, fluoro, methoxy, or methylthio, most preferably chloro, fluoro, or methoxy.

Each R⁹ is independently halogen or C₁-C₈alkyl. Preferably, each R⁹ is independently chloro, fluoro or methyl, most preferably each R⁹ methyl.

Each R¹⁰ is independently halogen, cyano, nitro, C₁-C₈alkyl, C₁-C₈haloalkyl, C₂-C₈alkenyl, C₂-C₈haloalkenyl, C₂-C₈alkynyl, C₂-C₈haloalkynyl, hydroxy, C₁-C₈alkoxy, C₁-C₈haloalkoxy, mercapto, C₁-C₈alkylthio, C₁-C₈haloalkylthio, C₁-C₈alkylsulfinyl, C₁-C₈haloalkylsulfinyl, C₁-C₈alkylsulfonyl, C₁-C₈haloalkylsulfonyl, C₁-C₈alkylcarbonyl, C₁-C₈alkoxycarbonyl, aryl or aryl substituted by one to five R¹¹, or heterocyclyl or heterocyclyl substituted by one to five R¹¹. Preferably each R¹⁰ is independently halogen, cyano, nitro, C₁-C₈alkyl, C₁-C₈haloalkyl, C₁-C₈alkoxy, C₁-C₈haloalkoxy, more preferably bromo, chloro, fluoro, cyano, nitro, methyl, ethyl, trifluoromethyl, methoxy, difluoromethoxy, or trifluoromethoxy, most preferably bromo, chloro, fluoro, cyano or methyl.

Each R⁴ and R¹¹ is independently halogen, cyano, nitro, C₁-C₈alkyl, C₁-C₈haloalkyl, C₁-C₈alkoxy, C₁-C₈haloalkoxy or C₁-C₈alkoxycarbonyl; more preferably each R⁴ and R¹¹ is independently bromo, chloro, fluoro, cyano, nitro, methyl, ethyl, trifluoromethyl, methoxy, difluoromethoxy or trifluoromethoxy, more preferably bromo, chloro, fluoro, nitro or methyl, most preferably each R⁴ and R¹¹ is independently chloro, fluoro or methyl.

Each R¹² is independently hydrogen, cyano, cyano-C₁-C₈alkyl, C₁-C₈alkyl, C₁-C₈haloalkyl, C₃-C₈cycloalkyl, C₃-C₈cycloalkyl where one carbon atom is replaced by O, S, S(O) or SO₂, or C₃-C₈cycloalkyl-C₁-C₈alkylene, C₃-C₈cycloalkyl-C₁-C₈alkylene where one carbon atom in the cycloalkyl group is replaced by O, S, S(O) or SO₂, or C₃-C₈cycloalkyl-C₁-C₈haloalkylene, C₁-C₈hydroxyalkyl, C₁-C₈alkoxy-C₁-C₈alkylene, C₂-C₈alkenyl, C₂-C₈haloalkenyl, C₂-C₈alkynyl, C₂-C₈haloalkynyl, aryl or aryl substituted by one to three R¹¹, C₁-C₈alkylcarbonyl, C₁-C₈haloalkylcarbonyl, C₁-C₈alkoxycarbonyl, C₁-C₈haloalkoxycarbonyl, C₁-C₈alkylsulfonyl, C₁-C₈haloalkylsulfonyl, aryl-C₁-C₄alkylene or aryl-C₁-C₄alkylene where the aryl moiety is substituted by one to three R¹¹, or heteroaryl-C₁-C₄alkylene or heteroaryl-Ci-C₄alkylene where the heteroaryl moiety is substituted by one to three R¹¹, or C₁-C₄alkyl-(C₁-C₄alkyl-O-N=)C-CH₂-. Preferably, each R¹² is independently hydrogen, cyano, C₁-C₈alkyl, C₁-C₈haloalkyl, C₁-C₈alkylcarbonyl, C₁-C₈haloalkylcarbonyl, C₁-C₈alkoxycarbonyl, C₁-C₈haloalkoxycarbonyl, C₁-C₈alkylsulfonyl, C₁-C₈haloalkylsulfonyl, aryl-C₁-C₄alkylene or aryl-C₁-C₄alkylene where the aryl moiety is substituted by one to three R¹¹, or heteroaryl-Ci-C₄alkylene or heteroaryl-C₁-C₄alkylene where the heteroaryl moiety is substituted by one to three R¹¹. More preferably, each R¹² is independently hydrogen, cyano, C₁-C₈alkyl, C₁-C₈haloalkyl, C₁-C₈alkylcarbonyl, C₁-C₈haloalkylcarbonyl, C₁-C₈alkoxycarbonyl, C₁-C₈haloalkoxycarbonyl, C₁-C₈alkylsulfonyl, C₁-C₈haloalkylsulfonyl, phenyl-C₁-C₄alkylene or phenyl-C₁-C₄alkylene where the phenyl moiety is substituted by one to three R¹¹, or pyridyl-C₁-C₄alkylene or pyridyl-C₁-C₄alkylene where the pyridyl moiety is substituted by one to three R¹¹.

R¹³ is halogen or imidazole, preferably chloro, fluoro or bromo.

Each R¹⁴ is independently hydrogen, C₁-C₈alkyl, C₂-C₈alkenyl, C₂-C₈alkynyl, C₃-C₁₀cycloalkyl, C₁-C₆alkyl-C₃-C₈cycloalkyl, C₃-C₈cycloalkyl-C₁-C₆alkylene, C₁-C₁₀alkylcarbonyl, C₁-C₈alkoxycarbonyl, C₁-C₈alkylsulfonyl, C₁-C₈haloalkylsulfonyl, or arylsulfonyl or arylsulfonyl substituted by one to five groups independently selected from C₁-C₄alkyl and nitro; more preferably each R¹⁴ is independently hydrogen, C₁-C₈alkyl, phenylsulfonyl or phenylsulfonyl substituted by one to five groups independently selected from C₁-C₄alkyl and nitro.

R¹⁵ and R¹⁶ are each independently hydrogen, C₁-C₁₂alkyl or C₁-C₁₂alkyl substituted by one to five R⁸, C₃-C₈cycloalkyl or C₃-C₈cycloalkyl substituted by one to five R⁹, C₂-C₁₂alkenyl or C₂-C₁₂alkenyl substituted by one to five R⁸, C₂-C₁₂alkynyl or C₂-C₁₂alkynyl substituted by one to five R⁸, cyano, C₁-C₁₂alkoxycarbonyl or C₁-C₁₂alkoxycarbonyl substituted by one to five R⁸, C₁-C₁₂alkoxythiocarbonyl or C₁-C₁₂alkoxythiocarbonyl substituted by one to five R⁸, or R¹⁵ and R¹⁶ together with the carbon atom to which they are attached may form a 3 to 6-membered carbocyclic ring. Preferably, R¹⁵ and R¹⁶ are each independently hydrogen, C₁-C₁₂alkyl, C₁-C₁₂haloalkyl, C₃-C₈cycloalkyl, C₃-C₈halocycloalkyl, C₂-C₁₂alkenyl or C₂-C₁₂haloalkenyl, C₂-C₁₂alkynyl, C₂-C₁₂haloalkynyl cyano, C₁-C₁₂alkoxycarbonyl, C₁-C₁₂haloalkoxycarbonyl, C₁-C₁₂alkoxythiocarbonyl, C₁-C₁₂haloalkoxythiocarbonyl, or R¹⁵ and R¹⁶ together with the carbon atom to which they are attached may form a 3 to 6-membered carbocyclic ring. Preferably, R¹⁵ and R¹⁶ are each independently hydrogen, halogen, cyano, C₁-C₄alkyl or C₁-C₄haloalkyl.

R¹⁷ is hydrogen, NH₂, hydroxyl, C₁-C₁₂ alkoxy or C₁-C₁₂alkoxy substituted by one to five R⁸, C₁-C₁₂alkylcarbonylamino or C₁-C₁₂alkylcarbonylamino wherein the alkyl is substituted by one to five R⁸, C₁-C₁₂alkylamino or C₁-C₁₂alkylamino wherein the alkyl is substituted by one to five R⁸, C₁-C₁₂alkyl or C₁-C₁₂alkyl substituted by one to five R⁸, C₃-C₈cycloalkyl or C₃-C₈cycloalkyl substituted by one to five R⁹, cyano, C₂-C₁₂alkenyl or C₂-C₁₂alkenyl substituted by one to five R⁸, C₂-C₁₂alkynyl or C₂-C₁₂alkynyl substituted by one to five R⁸, C₁-C₁₂alkylcarbonyl or C₁-C₁₂alkylcarbonyl substituted by one to five R⁸, C₁-C₁₂alkoxycarbonyl or C₁-C₁₂alkoxycarbonyl substituted by one to five R⁸ or is selected from CH₂-R²⁵, C(=O)R¹⁹ and C(=S)R¹⁹. Preferably, R¹⁷ is hydrogen, NH₂, hydroxyl, C₁-C₁₂alkoxy, C₁-C₁₂haloalkoxy, C₁-C₁₂alkylcarbonylamino, C₁-C₁₂haloalkylcarbonylamino, C₁-C₁₂alkylamino, C₁-C₁₂haloalkylamino, C₁-C₁₂alkyl, C₁-C₁₂haloalkyl, C₃-C₈cycloalkyl, C₃-C₈halocycloalkyl, cyano, C₁-C₁₂alkenyl, C₁-C₁₂haloalkenyl, C₂-C₁₂alkynyl, C₂-C₁₂haloalkynyl, C₁-C₁₂alkylcarbonyl, C₁-C₁₂haloalkylcarbonyl, C₁-C₈alkoxycarbonyl, or C₁-C₈haloalkoxycarbonyl. More preferably, R¹⁷ is hydrogen, C₁-C₈alkyl, C₁-C₈alkoxy, C₁-C₈alkylcarbonyl, or C₁-C₈alkoxycarbonyl.

R¹⁸ is hydrogen, cyano, carbonyl, thiocarbonyl, C₁-C₁₂alkylcarbonyl or C₁-C₁₂ alkylcarbonyl substituted by one to five R⁸, C₁-C₁₂alkylthiocarbonyl or C₁-C₁₂alkylthiocarbonyl substituted by one to five R⁸, C₁-C₁₂alkylaminocarbonyl or C₁-C₁₂alkylaminocarbonyl wherein the alkyl is substituted by one to five R⁸, C₁-C₁₂alkylaminothiocarbonyl or C₁-C₁₂alkylaminothiocarbonyl wherein the alkyl is substituted by one to five R⁸, C₂-C₂₄ (total carbon number) dialkylaminocarbonyl or C₂-C₂₄ (total carbon number) dialkylaminocarbonyl wherein one or both alkyl is substituted by one to five R⁸, C₂-C₂₄ (total carbon number) dialkylaminothiocarbonyl or C₂-C₂₄ (total carbon number) dialkylaminothiocarbonyl wherein one or both alkyl is substituted by one to five R⁸, C₁-C₁₂alkoxyaminocarbonyl or C₁-C₁₂alkoxyaminocarbonyl wherein the alkoxy is substituted by one to five R⁸, C₁-C₁₂alkoxyaminothiocarbonyl or C₁-C₁₂alkoxyaminothiocarbonyl wherein the alkoxy is substituted by one to five R⁸, C₁-C₁₂alkoxycarbonyl or C₁-C₁₂alkoxycarbonyl substituted by one to five R⁸, C₁-C₁₂alkoxythiocarbonyl or C₁-C₁₂alkoxythiocarbonyl substituted by one to five R⁸, C₁-C₁₂thioalkoxycarbonyl or C₁-C₁₂thioalkoxycarbonyl substituted by one to five R⁸, C₁-C₁₂thioalkoxythiocarbonyl or C₁-C₁₂thioalkoxythiocarbonyl substituted by one to five R⁸, C₁-C₁₂alkylsulfonyl or C₁-C₁₂alkylsulfonyl substituted by one to five R⁸, C₃-C₁₂cycloalkylcarbonyl or C₃-C₁₂cycloalkylcarbonyl substituted by one to five R⁹, C₂-C₁₂alkenylcarbonyl or C₂-C₁₂alkenylcarbonyl substituted by one to five R⁸, C₂-C₁₂alkynylcarbonyl or C₂-C₁₂alkynylcarbonyl substituted by one to five R⁸, C₃-C₁₂cycloalkyl-C₁-C₁₂alkylcarbonyl or C₃-C₁₂cycloalkyl-C₁-C₁₂alkylcarbonyl substituted by one to five R⁹, C₁-C₁₂alkylsulfenyl-C₁-C₁₂alkylcarbonyl or C₁-C₁₂alkylsulfenyl-C₁-C₁₂alkylcarbonyl substituted by one to five R⁸, C₁-C₁₂alkylsulfinyl-C₁-C₁₂alkylcarbonyl or C₁-C₁₂alkylsulfinyl-C₁-C₁₂alkylcarbonyl substituted by one to five R⁸, C₁-C₁₂ alkylsulfonyl-C₁-C₁₂alkylcarbonyl or C₁-C₁₂alkylsulfonyl-C₁-C₁₂alkylcarbonyl substituted by one to five R⁸, C₁-C₁₂alkylcarbonyl-C₁-C₁₂alkylcarbonyl or C₁-C₁₂alkylcarbonyl-C₁-C₁₂alkylcarbonyl substituted by one to five R⁸, C₃-C₁₂cycloalkylaminocarbonyl or C₃-C₁₂cycloalkylaminocarbonyl wherein the cycloalkyl is substituted by one to five R⁹, C₂-C₁₂alkenylaminocarbonyl or C₂-C₁₂alkenylaminocarbonyl wherein the alkenyl is substituted by one to five R⁸, C₂-C₁₂alkynylaminocarbonyl or C₂-C₁₂alkynylaminocarbonyl wherein the alkynyl is substituted by one to five R⁸, or is selected from C(=O)R¹⁹ and C(=S)R¹⁹. Preferably R¹⁸ is hydrogen, cyano, carbonyl, thiocarbonyl, C₁-C₁₂alkylcarbonyl, C₁-C₁₂haloalkylcarbonyl, C₁-C₁₂alkylthiocarbonyl, C₁-C₁₂haloalkylthiocarbonyl, C₁-C₁₂alkylaminocarbonyl, C₁-C₁₂alkylaminothiocarbonyl, C₂-C₂₄ (total carbon number) dialkylaminocarbonyl, C₂-C₂₄ (total carbon number) dialkylaminothiocarbonyl, C₁-C₁₂alkoxyaminocarbonyl, C₁-C₁₂alkoxyaminothiocarbonyl, C₁-C₁₂alkoxycarbonyl, C₁-C₁₂haloalkoxycarbonyl, C₁-C₁₂alkoxythiocarbonyl, C₁-C₁₂haloalkoxythiocarbonyl, C₁-C₁₂thioalkoxycarbonyl, C₁-C₁₂thioalkoxythiocarbonyl, C₁-C₁₂alkylsulfonyl, C₁-C₁₂haloalkylsulfonyl, C₃-C₁₂cycloalkylcarbonyl, C₃-C₁₂halocycloalkylcarbonyl, C₂-C₁₂alkenylcarbonyl, C₂-C₁₂haloalkenylcarbonyl, C₂-C₁₂ alkynylcarbonyl, C₂-C₁₂haloalkynylcarbonyl, C₃-C₁₂cycloalkyl-C₁-C₁₂alkylcarbonyl, C₃-C₁₂halocycloalkyl-C₁-C₁₂alkylcarbonyl, C₂-C₁₂alkylsulfenyl-C₁-C₁₂alkylcarbonyl, C₂-C₁₂haloalkylsulfenyl-C₁-C₁₂alkylcarbonyl, C₁-C₁₂alkylsulfinyl-C₁-C₁₂alkylcarbonyl, C₁-C₁₂haloalkylsulfinyl-C₁-C₁₂alkylcarbonyl, C₁-C₁₂alkylsulfonyl-C₁-C₁₂alkylcarbonyl, C₁-C₁₂haloalkylsulfonyl-C₁-C₁₂alkylcarbonyl, C₁-C₁₂alkylcarbonyl-C₁-C₁₂alkylcarbonyl, C₁-C₁₂haloalkylcarbonyl-C₁-C₁₂alkylcarbonyl, C₃-C₁₂cycloalkylaminocarbonyl, C₂-C₁₂alkenylaminocarbonyl, C₂-C₁₂alkynylaminocarbonyl. More preferably, R¹⁸ is C₁-C₄alkylcarbonyl or C₁-C₄alkylcarbonyl substituted by one to five R⁸, C₃-C₆ cycloalkylcarbonyl or C₃-C₆cycloalkylcarbonyl wherein the cycloalkyl is substituted by one to five R⁹; even more Preferably, R¹⁸ is C₁-C₄alkylcarbonyl, C₁-C₄haloalkylcarbonyl, C₃-C₆cycloalkylcarbonyl or C₃-C₆halocycloalkylcarbonyl.

R¹⁷ and R¹⁸ together with the nitrogen atom to which they are bound may form a 3- to 6-membered heterocyclic ring which may be substituted by one to five R¹¹, or may be substituted with a keto, thioketo or nitroimino group.

R¹⁹ is aryl or aryl substituted by one to five R¹¹, heterocyclyl or heterocyclyl substituted by one to five R¹¹. The aryl is preferably phenyl and the heterocyclyl is preferably pyridyl.

R²⁰ is hydrogen or C₁-C₈alkyl.

Each R²¹ and R²² is independently hydrogen, halogen, C₁-C₈alkyl or C₁-C₈haloalkyl.

Each Z¹ is independently halogen, C₁-C₁₂alkyl or C₁-C₁₂alkyl substituted by one to five R⁸, nitro, C₁-C₁₂alkoxy or C₁-C₁₂alkoxy substituted by one to five R⁸, cyano, C₁-C₁₂alkylsulfinyl, C₁-C₁₂ alkylsulfonyl, C₁-C₁₂haloalkylsulfinyl, C₁-C₁₂haloalkylsulfonyl, hydroxyl or thiol.

Preferably each Z¹ is independently halogen, cyano, C₁-C₄alkyl, C₁-C₄haloalkyl, C₁-C₄alkoxy, or C₁-C₄haloalkoxy, more preferably each Z¹ is independently hydrogen, halogen, methyl, halomethyl, methoxy or halomethoxy.

R²⁶ is hydrogen, azido, halogen, hydroxy, optionally substituted amino, optionally substituted alkoxy, optionally substituted alkoxycarbonyl or -CO₂H, more preferably - N(R²⁸)(R²⁹), halogen, hydroxy, C₁-C₈alkoxy, C₁-C₈haloalkoxy, C₁-C₈alkoxycarbonyl, C₁-C₈haloalkoxycarbonyl, or -CO₂H.

R²⁷ is hydrogen, halogen, hydroxy, optionally substituted amino, optionally substituted alkyl, optionally substituted alkoxy, optionally substituted aloxycarbonyl, more preferably hydrogen, halogen, hydroxy, hydrogen, C₁-C₈alkyl, C₁-C₈alkoxy, C₁-C₈haloalkoxy, more preferably hydrogen, C₁-C₈alkyl, C₁-C₈alkoxy, or C₁-C₈haloalkoxy.

R²⁶ and R²⁷ may together be oxo, optionally substituted oxime, optionally substituted imine and optionally substituted hydrazone

R²⁸ is hydrogen, cyano, formyl, thioformyl, alkylcarbonyl, haloalkylcarbonyl, alkyl-thiocarbonyl, haloalkyl-thiocarbonyl, mono- or di-alkylaminocarbonyl, mono- or di-aikylamino-thiocarbonyl, alkoxyaminocarbonyl, alkoxyamino-thiocarbonyl, alkoxycarbonyl, alkoxyalkylcarbonyl, alkoxy-thiocarbonyl, alkylthio-carbonyl, alkylthio-thiocarbonyl, alkylsulfonyl, haloalkylsulfonyl, cycloalkylcarbonyl, alkenylcarbonyl, alkynylcarbonyl, alkynylalkylcarbonyl, cycloalkyl-alkylcarbonyl, alkylthioalkyl- carbonyl, alkylsulfinylalkylcarbonyl, alkylsulfonylalkylcarbonyl, alkylcarbonylalkylcarbonyl, cycloalkylaminocarbonyl, alkenylaminocarbonyl, alkynylaminocarbonyl, -CH₂-R³⁰, -C(0)R³⁰ or -C(S)R³⁰, and each group from alkylcarbonyl to alkynylaminocarbonyl among the definitions of R⁸ may be substituted; preferably R²⁸ is hydrogen, cyano, formyl, thioformyl, C₁-C₁₂alkylcarbonyl, C₁-C₁₂haloalkyl-carbonyl, C₁-C₁₂alkyl-thiocarbonyl, C₁-C₁₂haloalkyl-thiocarbonyl, mono-C₁-C₁₂ or di-C₂-C₂₄alkyl-aminocarbonyl, mono-C₁-C₁₂ or di-C₂-C₂₄alkylamino-thiocarbonyl, C₁-C₁₂alkoxy-aminocarbonyl, C₁-C₁₂alkoxyamino-thiocarbonyl, C₁-C₁₂alkoxy-carbonyl, C₁-C₁₂alkoxy-C₁-C₁₂alkyl-carbonyl, C₁-C₁₂alkoxy-thiocarbonyl, C₁-C₁₂alkylthio-carbonyl, C₁-C₁₂alkylthio-thiocarbonyl, C₁-C₁₂alkylsulfonyl, C₁-C₁₂haloalkylsulfonyl, C₃-C₈cycloalkyl-carbonyl, C₂-C₆alkenyl-carbonyl, C₂-C₆alkynyl-carbonyl, C₂-C₆alkynyl- C₁-C₁₂alkyl-carbonyl, C₃-C₈cycloalkyl- C₁-C₁₂alkyl-carbonyl, C₁-C₁₂alkylthio-C₁-C₁₂alkyl-carbonyl, C₁-C₁₂alkyl- sulfinyl- C₁-C₁₂alkyl-carbonyl, C₁-C₁₂alkylsulfonyl- C₁-C₁₂alkyl-carbonyl, C₁-C₁₂alkylcarbonyl- C₁-C₁₂alkyl-carbonyl, C₃-C₈cycloalkylamino-carbonyl, C₂-C₆alkenylamino-carbonyl, C₂-C₆alkynylamino-carbonyl, -CH₂-R¹⁰, -C(0)R¹⁰, or -C(S)R¹⁰, and each group from C₁-C₁₂alkyl-carbonyl to C₂-C₆alkynyl-amino-carbonyl, among the definitions of R⁸ may be optionally substituted; more preferably R²⁸ is hydrogen, cyano, carbonyl, thiocarbonyl, C₁-C₆alkyl-carbonyl, C₁-C₆haloalkyl-carbonyl, C₁-C₆alkyl-thiocarbonyl, C₁-C₆haloalkyl-thiocarbonyl, mono-C₁-C₆ or di-(C₂-C₁₂) alkyl-aminocarbonyl, mono-C₁-C₆ or di-(C₂-C₁₂)alkylamino-thiocarbonyl, C₁-C₆alkoxy-aminocarbonyl, C₁-C₆alkoxyamino-thiocarbonyl, C₁-C₆alkoxy-carbonyl, C₁-C₆alkoxy- C₁-C₆alkyl-carbonyl, C₁-C₆alkoxy-thiocarbonyl, C₁-C₆alkylthio-carbonyl, C₁-C₆alkylthio-thiocarbonyl, C₁-C₆alkylsulfonyl, C₁-C₆haloalkylsulfonyl, C₃-C₆cycloalkyl-carbonyl, C₂-C₄ alkenyl-carbonyl, C₂-C₄alkynyl-carbonyl, C₂-C₄alkynyl-C₁-C₂alkyl -carbonyl, C₃-C₆cycloalkyl-C₁-C₂alkyl-carbonyl, C₁-C₆alkylthio- C₁-C₆alkyl-carbonyl, C₁-C₆alkylsulfinyl- C₁-C₆alkyl-carbonyl, C₁-C₆ alkylsulfonyl- C₁-C₆alkyl-carbonyl, C₁-C₆alkylcarbonyl- C₁-C₆alkyl-carbonyl, C₃-C₆cycloalkylamino-carbonyl, C₂-C₄ alkenylamino-carbonyl, C₁-C₆alkynylamino-carbonyl, -CH₂-R³⁰-, -C(0)R³⁰ or -C(S)R³⁰ and each group from C₁-C₆alkyl-carbonyl to C₁-C₆alkynylamino-carbonylamong the definitions of R²⁸ may be optionally substituted. In one group of compounds R⁸ is C₁-C₆alkyl-carbonyl, C₁-C₆haloalkyl-carbonyl, C₃-C₆cycloalkyl-C₁-C₂alkyl-carbonyl or C₃-C₆cycloalkyl-carbonyl.

R²⁹ is hydrogen, amino, hydroxy, cyano, alkyl, haloalkyl, cycloalkyl, alkenyl, alkynyl, alkylimino, alkoxy, alkylcarbonyl, alkylcarbonylamino, alkoxyalkyl, cyanoalkyl, alkoxycarbonylalkyl, -CH₂-R³⁰, -C(0)R³⁰ or -C(S)R³⁰, and each group from alkyl to alkylcarbonylamino among the definitions of R⁹ may be substituted; preferably R²⁹ is hydrogen, amino, hydroxy, cyano, C₁-C₁₂alkyl, C₁-C₁₂haloalkyl, C₃-C₈cycloalkyl, C₂-C₆alkenyl, C₂-C₆alkynyl, C₁-C₁₂alkylimino, C₁-C₁₂alkoxy, C₁-C₁₂alkyl-carbonyl, C₁-C₁₂alkyl-carbonyiamino, C₁-C₁₂alkoxy-C₁-C₁₂alkyl, C₁-C₁₂cyanoalkyl, C₁-C₁₂alkoxycarbonyl- C₁-C₁₂alkyl, -CH₂-R³⁰, -C(0)R³⁰, or -C(S)R³⁰ and each group from C₁-C₁₂alkyl alkyl to C₁-C₁₂alkoxycarbonyl- C₁-C₁₂alkyl among the definitions of R²⁹ may be optionally substituted; preferably R²⁹ is hydrogen, amino, hydroxy, cyano, C₁-C₆alkyl, C₁-C₁₂haloalkyl, C₃-C₆ cycloalkyl, C₂-C₄alkenyl, C₂-C₄alkynyl, C₁-C₆alkylimino, C₁-C₆alkoxy, C₁-C₆alkyl-carbonyl, C₁-C₆alkyl-carbonylamino, C₁-C₆alkoxy-C₁-C₆alkyl, C₃-C₆cyanoalkyl, C₁-C₆alkoxycarbonyl- C₁-C₆alkyl, -CH₂-R³⁰, -C(0)R³⁰ or -C(S)R³⁰, and each group from C₁-C₆alkyl to C₁-C₆alkoxycarbonyl- C₁-C₆alkyl, among the definitions of R²⁹ may be optionally substituted. In one group of compounds R⁹ is hydrogen, C₁-C₆alkoxy or benzyl.

R²⁸ and R²⁹, together with the N atom to which they are bound, may form a 3- to 6-membered heterocyclic ring which may be substituted and may further comprise N, O or S.

R³⁰ is phenyl which may be substituted, a 5- to 6-membered heterocyclic group which may be substituted and comprises at least one of N, O and S, optionally substituted C₁-C₁₂alkyl, amino, mono- C₁-C₁₂ or di(C₂-C₂₄)alkylamino; preferably optionally substituted phenyl, pyridyl, pyrimidinyl, or a group (HI) to (H9), or an optionally substituted C₁-C₆alkyl, amino, mono- C₁-C₆ or di(C₁-C₁₂)alkylamino group.

Preferably R¹⁰⁰ is C₁-C₁₂ alkyl, phenyl or heteroaryl as defined above, optionslly substituted with one to five groups independently selected from cyano, halogen, C₁-C₄alkyl, C₁-C₄haloalkyl, C₁-C₄alkoxy, and C₁-C₄haloalkoxy, more preferably C₁-C₆alkyl, most preferably ethyl.

In one group of compounds, group A1 (applicable to all compounds of the invention bearing a group R¹ and R²):
R¹ is trifluoromethyl.
R² is group A 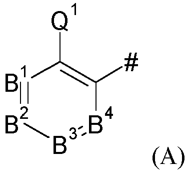
B¹, B², B³, B⁴ are independently C-Q¹ or nitrogen;
each Q¹ is independently hydrogen, halogen, cyano, C₁-C₈alkyl, C₁-C₈haloalkyl, C₁-C₈alkoxy or C₁-C₈haloalkoxy.

In one group of compounds, group A2, (applicable to all compounds bearing the group A') A' is selected from P1 to P6;
A¹, A², A³, and A⁴ are independently of each other C-H, C-R⁵, or nitrogen;
A^{1'}, A^{2'}, A^{3'}, A^{4'}, A^{5'} and A^{6'} are independently of each other C-H, C-R⁵ or nitrogen provided that no more than two of A^{1'}, A^{2'}, A^{3'}, A^{4'}, A^{5'} and A^{6'} are nitrogen;
each R⁵ is independently hydrogen, halogen, C₁-C₈alkyl, C₁-C₈haloalkyl or C₂-C₈alkenyl;
Q is cyano, halogen, nitro, NH₂, arylsulfonyl or arylsulfonyl substituted by one to five groups independently selected from C₁-C₄alkyl and nitro, heterocyclyl or heterocyclyl substituted by one to five Z¹, -OR¹⁴, -C(=O)N(R⁶)R⁷, -C(=O)OR^{7a}, -C(=O)R¹³, or -C(R¹⁵)(R¹⁶)N(R¹⁷)R¹⁸;
k is 0, 1, or 2;
R⁶ is hydrogen, methyl, ethyl, methylcarbonyl or methoxycarbonyl;
R⁷ is hydrogen, C₁-C₈alkyl or C₁-C₈alkyl substituted by one to five R⁸, C₃-C₁₀cycloalkyl or C₃-C₁₀cycloalkyl substituted by one to five R⁹, aryl-C₁-C₆alkylene or aryl-C₁-C₆alkylene wherein the aryl moiety is substituted by one to five R¹⁰, heterocyclyl-C₁-C₆alkylene or heterocyclyl-C₁-C₆alkylene wherein the heterocyclyl moiety is substituted by one to five R¹⁰ and wherein each heterocyclyl moiety contains one or more ring members independently selected from O, N, C=O, C=N-OR¹², N-R¹², S, SO, SO₂, S=N-R¹² and SO=N-R¹², aryl or aryl substituted by one to five R¹⁰, heterocyclyl or heterocyclyl substituted by one to five R¹⁰ and wherein each heterocyclyl moiety contains one or more ring members independently selected from O, N, C=O, C=N-OR¹², N-R¹², S, SO, SO₂, S=N-R¹² and SO=N-R¹², C₁-C₈alkyl-N(R⁶)-C(=O)-C₁-C₄ alkylene, C₁-C₈haloalkyl-N(R⁶)-C(=O)-C₁-C₄alkylene, C₃-C₈cycloalkylaminocarbonyl-C₁-C₄alkylene, C₁-C₆alkyl-O-N=CH-, or C₁-C₆haloalkyl-O-N=CH;
R^{7a} is hydrogen, C₁-C₁₅alkyl or C₁-C₁₅alkyl substituted by one to five R⁸, C₂-C₁₅alkenyl or C₂-C₁₅alkenyl substituted by one to five R⁸, C₂-C₁₅alkynyl or C₂-C₁₅alkynyl substituted by one to five R⁸, C₃-C₁₀cycloalkyl or C₃-C₁₀cycloalkyl substituted by one to five R⁹, aryl-Ci-C₆alkylene or aryl-C₁-C₆alkylene wherein the aryl moiety is substituted by one to five R¹⁰, heteroaryl-C₁-C₆alkylene or heteroaryl-C₁-C₆alkylene wherein the heteroaryl moiety is is substituted by one to five R¹⁰, or heteroaryl or heteroaryl substituted by one to five R¹⁰;
R^{7b} is hydrogen, C₁-C₁₅alkyl, C₁-C₁₅haloalkyl, C₂-C₁₅alkenyl, C₂-C₁₅haloalkenyl, C₂-C₁₅alkynyl, C₂-C₁₅haloalkynyl, C₃-C₁₀cycloalkyl, C₁-C₁₅alkylcarbonyl or C₁-C₁₅alkoxycarbonyl;
each R⁸ is independently halogen, cyano, nitro, hydroxy, C₁-C₈alkoxy, C₁-C₈haloalkoxy, C₁-C₈alkylcarbonyl, C₁-C₈alkoxycarbonyl, mercapto, C₁-C₈alkylthio, C₁-C₈haloalkylthio, C₁-C₈alkylsulfinyl, C₁-C₈haloalkylsulfinyl, C₁-C₈alkylsulfonyl;
each R⁹ is independently halogen or C₁-C₈alkyl. Preferably, each R⁹ is independently chloro, fluoro or methyl;
each R¹⁰ is independently halogen, cyano, nitro, C₁-C₈alkyl, C₁-C₈haloalkyl, C₁-C₈alkoxy, C₁-C₈haloalkoxy;
each R¹¹ is independently halogen, cyano, nitro, C₁-C₈alkyl, C₁-C₈haloalkyl, C₁-C₈alkoxy, C₁-C₈haloalkoxy or C₁-C₈alkoxycarbonyl;
each R¹² is independently hydrogen, cyano, C₁-C₈alkyl, C₁-C₈haloalkyl, C₁-C₈alkyl-carbonyl, C₁-C₈haloalkylcarbonyl, C₁-C₈alkoxycarbonyl, C₁-C₈haloalkoxycarbonyl, C₁-C₈alkyl-sulfonyl, C₁-C₈haloalkylsulfonyl, aryl-C₁-C₄alkylene or aryl-C₁-C₄alkylene where the aryl moiety is substituted by one to three R¹¹, or heteroaryl-C₁-C₄alkylene or heteroaryl-Ci-C₄alkylene where the heteroaryl moiety is substituted by one to three R¹¹;
R¹³ is halogen or imidazole;
each R¹⁴ is independently hydrogen, C₁-C₈alkyl, phenylsulfonyl or phenylsulfonyl substituted by one to five groups independently selected from C₁-C₄alkyl and nitro;
R¹⁵ and R¹⁶ are each independently hydrogen, C₁-C₁₂alkyl, C₁-C₁₂haloalkyl, C₃-C₈cycloalkyl, C₃-C₈halocycloalkyl, C₂-C₁₂alkenyl or C₂-C₁₂haloalkenyl, C₂-C₁₂alkynyl, C₂-C₁₂haloalkynyl cyano, C₁-C₁₂alkoxycarbonyl, C₁-C₁₂haloalkoxycarbonyl, C₁-C₁₂alkoxythiocarbonyl, C₁-C₁₂haloalkoxythiocarbonyl, or R¹⁵ and R¹⁶ together with the carbon atom to which they are attached may form a 3 to 6-membered carbocyclic ring;
R¹⁷ is hydrogen, NH₂, hydroxyl, C₁-C₁₂alkoxy, C₁-C₁₂haloalkoxy, C₁-C₁₂alkylcarbonylamino, C₁-C₁₂haloalkylcarbonylamino, C₁-C₁₂alkylamino, C₁-C₁₂haloalkylamino, C₁-C₁₂alkyl, C₁-C₁₂haloalkyl, C₃-C₈cycloalkyl, C₃-C₈halocycloalkyl, cyano, C₁-C₁₂alkenyl, C₁-C₁₂haloalkenyl, C₂-C₁₂alkynyl, C₂-C₁₂haloalkynyl, C₁-C₁₂alkylcarbonyl, C₁-C₁₂haloalkylcarbonyl, C₁-C₈alkoxycarbonyl, or C₁-C₈haloalkoxycarbonyl;
R¹⁸ is hydrogen, cyano, carbonyl, thiocarbonyl, C₁-C₁₂alkylcarbonyl, C₁-C₁₂haloalkylcarbonyl, C₁-C₁₂alkylthiocarbonyl, C₁-C₁₂haloalkylthiocarbonyl, C₁-C₁₂alkylaminocarbonyl, C₁-C₁₂alkylaminothiocarbonyl, C₂-C₂₄ (total carbon number) dialkylaminocarbonyl, C₂-C₂₄ (total carbon number) dialkylaminothiocarbonyl, C₁-C₁₂alkoxyaminocarbonyl, C₁-C₁₂alkoxyaminothiocarbonyl, C₁-C₁₂alkoxycarbonyl, C₁-C₁₂haloalkoxycarbonyl, C₁-C₁₂alkoxythiocarbonyl, C₁-C₁₂haloalkoxythiocarbonyl, C₁-C₁₂thioalkoxycarbonyl, C₁-C₁₂thioalkoxythiocarbonyl, C₁-C₁₂alkylsulfonyl, C₁-C₁₂haloalkylsulfonyl, C₃-C₁₂cycloalkylcarbonyl, C₃-C₁₂halocycloalkylcarbonyl, C₂-C₁₂alkenylcarbonyl, C₂-C₁₂haloalkenylcarbonyl, C₂-C₁₂ alkynylcarbonyl, C₂-C₁₂haloalkynylcarbonyl, C₃-C₁₂cycloalkyl-C₁-C₁₂alkylcarbonyl, C₃-C₁₂halocycloalkyl-C₁-C₁₂alkylcarbonyl, C₂-C₁₂alkylsulfenyl-C₁-C₁₂alkylcarbonyl, C₂-C₁₂haloalkylsulfenyl-C₁-C₁₂alkylcarbonyl, C₁-C₁₂alkylsulfinyl-C₁-C₁₂alkylcarbonyl, C₁-C₁₂haloalkylsulfinyl-C₁-C₁₂alkylcarbonyl, C₁-C₁₂alkylsulfonyl-C₁-C₁₂alkylcarbonyl, C₁-C₁₂haloalkylsulfonyl-C₁-C₁₂alkylcarbonyl, C₁-C₁₂alkylcarbonyl-C₁-C₁₂alkylcarbonyl, C₁-C₁₂haloalkylcarbonyl-C₁-C₁₂alkylcarbonyl, C₃-C₁₂cycloalkylaminocarbonyl, C₂-C₁₂alkenylaminocarbonyl, C₂-C₁₂alkynylaminocarbonyl. More preferably, R¹⁸ is C₁-C₄alkylcarbonyl or C₁-C₄alkylcarbonyl substituted by one to five R⁸, C₃-C₆ cycloalkylcarbonyl or C₃-C₆cycloalkylcarbonyl wherein the cycloalkyl is substituted by one to five R⁹; even more Preferably, R¹⁸ is C₁-C₄alkylcarbonyl, C₁-C₄haloalkylcarbonyl, C₃-C₆cycloalkylcarbonyl or C₃-C₆halocycloalkylcarbonyl;
R¹⁷ and R¹⁸ together with the nitrogen atom to which they are bound may form a 3- to 6-membered heterocyclic ring which may be substituted by one to five R¹¹, or may be substituted with a keto, thioketo or nitroimino group;
R¹⁹ is aryl or aryl substituted by one to five R¹¹, heterocyclyl or heterocyclyl substituted by one to five R¹¹ wherein aryl is phenyl and the heterocyclyl is preferably pyridyl;
R²⁰ is hydrogen or C₁-C₈alkyl;
each R²¹ and R²² is independently hydrogen, halogen, C₁-C₈alkyl or C₁-C₈haloalkyl;
each Z¹ is independently halogen, cyano, C₁-C₄alkyl, C₁-C₄haloalkyl, C₁-C₄alkoxy, or C₁-C₄haloalkoxy;
R²⁶ is -N(R²⁸)(R²⁹), halogen, hydroxy, C₁-C₈alkoxy, C₁-C₈haloalkoxy, C₁-C₈alkoxycarbonyl, C₁-C₈haloalkoxycarbonyl, or -CO₂H;
R²⁷ is hydrogen, halogen, hydroxy, hydrogen, C₁-C₈alkyl, C₁-C₈alkoxy, C₁-C₈haloalkoxy, more preferably hydrogen, C₁-C₈alkyl, C₁-C₈alkoxy, or C₁-C₈haloalkoxy;
R²⁶ and R²⁷ may together be oxo, optionally substituted oxime, optionally substituted imine and optionally substituted hydrazone;
R²⁸ is hydrogen, cyano, formyl, thioformyl, C₁-C₁₂alkylcarbonyl, C₁-C₁₂haloalkyl-carbonyl, C₁-C₁₂alkyl-thiocarbonyl, C₁-C₁₂haloalkyl-thiocarbonyl, mono-C₁-C₁₂ or di-C₂-C₂₄alkyl-aminocarbonyl, mono-C₁-C₁₂ or di-C₂-C₂₄alkylamino-thiocarbonyl, C₁-C₁₂alkoxy-aminocarbonyl, C₁-C₁₂alkoxyamino-thiocarbonyl, C₁-C₁₂alkoxy-carbonyl, C₁-C₁₂alkoxy-C₁-C₁₂alkyl-carbonyl, C₁-C₁₂alkoxy-thiocarbonyl, C₁-C₁₂alkylthio-carbonyl, C₁-C₁₂alkylthio-thiocarbonyl, C₁-C₁₂alkylsulfonyl, C₁-C₁₂haloalkylsulfonyl, C₃-C₈cycloalkyl-carbonyl, C₂-C₆alkenyl-carbonyl, C₂-C₆alkynyl-carbonyl, C₂-C₆alkynyl- C₁-C₁₂alkyl-carbonyl, C₃-C₈cycloalkyl- C₁-C₁₂alkyl-carbonyl, C₁-C₁₂alkylthio- C₁-C₁₂alkyl-carbonyl, C₁-C₁₂alkylsulfinyl- C₁-C₁₂alkyl-carbonyl, C₁-C₁₂alkylsulfonyl- C₁-C₁₂alkyl-carbonyl, C₁-C₁₂alkylcarbonyl-C₁-C₁₂alkyl-carbonyl, C₃-C₈cycloalkylamino-carbonyl, C₂-C₆alkenylamino-carbonyl, C₂-C₆alkynylamino-carbonyl, -CH₂-R¹⁰, -C(0)R¹⁰, or -C(S)R¹⁰, and each group from C₁-C₁₂alkylcarbonyl to C₂-C₆alkynyl-amino-carbonyl, among the definitions of R⁸ may be optionally substituted;
R²⁹ is hydrogen, amino, hydroxy, cyano, C₁-C₁₂alkyl, C₁-C₁₂haloalkyl, C₃-C₈cycloalkyl, C₂-C₆alkenyl, C₂-C₆alkynyl, C₁-C₁₂alkylimino, C₁-C₁₂alkoxy, C₁-C₁₂alkyl-carbonyl, C₁-C₁₂alkyl-carbonyiamino, C₁-C₁₂alkoxy-C₁-C₁₂alkyl, C₁-C₁₂cyanoalkyl, C₁-C₁₂alkoxycarbonyl-C₁-C₁₂alkyl, -CH₂-R³⁰, -C(0)R³⁰, or -C(S)R³⁰ and each group from C₁-C₁₂alkyl alkyl to C₁-C₁₂alkoxycarbonyl-C₁-C₁₂alkyl among the definitions of R²⁹ may be optionally substituted;
R²⁸ and R²⁹, together with the N atom to which they are bound, may form a 3- to 6-membered heterocyclic ring which may be substituted and may further comprise N, O or S;
optionally substituted phenyl, pyridyl, pyrimidinyl, or a group (H1) to (H9), or an optionally substituted C₁-C₆alkyl, amino, mono- C₁-C₆ or di(C₁-C₁₂)alkylamino group;
wherein unless otherwise stated optionaly substituents are independently selected from cyano, halogen, C₁-C₄alkyl, C₁-C₄haloalkyl, C₁-C₄alkoxy, and C₁-C₄haloalkoxy.

In one group of compounds, group A3, (applicable to all compounds of the invention bearing group A') A' is P1 or P2;
A¹, A² and A³ are C-H;
Q is cyano, halogen, nitro, NH₂, C₁-C₈alkoxy, phenylsulfonyl or phenylsulfonyl substituted by one to five groups independently selected from C₁-C₄ alkyl and nitro, -C(=O)N(R⁶)R⁷, -C(=O)OR^{7a}, -C(=O)R¹³, -C(R¹⁵)(R¹⁶)N(R¹⁷)R¹⁸, or a heterocycle selected from HI to H9;
k is 0, 1 or 2, preferably 0;
each R⁵ is independently halogen, C₁-C₈alkyl, C₁-C₈haloalkyl or C₂-C₈alkenyl;
R⁶ is hydrogen;
R⁷ is hydrogen, C₁-C₈alkyl, C₁-C₈haloalkyl, phenyl-C₁-C₆alkylene or phenyl-Ci-C₆alkylene wherein the phenyl moiety is substituted by one to five R¹⁰, pyridyl-C₁-C₆alkylene or pyridyl-C₁-C₆alkylene wherein the pyridyl moiety is substituted by one to four R¹⁰, thiazolyl-C₁-C₆alkylene or thiazolyl-C₁-C₆alkylene wherein the thiazolyl moiety is substituted by one or two R¹⁰, phenyl or phenyl substituted by one to five R¹⁰, pyridyl or pyridyl substituted by one to four R¹⁰, thiazolyl or thiazolyl substituted by one or two R¹⁰, C₃-C₆cycloalkyl or C₃-C₆cycloalkyl wherein one ring atom is replaced by O or S, C₁-C₄alkyl-O-N=CH-, C₁-C₄haloalkyl-O-N=CH-, C₁-C₄alkyl-N(R⁶)-C(=O)-CH₂-, C₁-C₄haloalkyl-N(R⁶)-C(=O)-CH₂-, or a group of formula (Y) 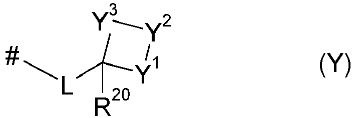 L is a single bond or C₁-C₆alkylene, preferably a bond;
Y¹, Y² and Y³ are independently of another O, CR²¹R²², C=O, C=N-OR¹², N-R¹², S, SO, SO₂, S=N-R¹² or SO=N-R¹², provided that at least one of Y¹, Y² or Y³ is not CR²¹R²², C=O or C=N-OR¹², preferably two of Y¹, Y² and Y³ are CH₂ and the other is S, SO or SO;
R^{7a} is C₁-C₁₅alkyl, C₁-C₁₅haloalkyl, C₂-C₁₅alkenyl, C₂-C₁₅haloalkenyl, pyridyl or benzyl;
each R⁸ is independently halogen, cyano, nitro, hydroxy, C₁-C₈alkoxy, C₁-C₈haloalkoxy, mercapto, C₁-C₈alkylthio, C₁-C₈haloalkylthio;
each R⁹ is independently halogen or C₁-C₈alkyl;
each R¹⁰ is independently halogen, cyano, nitro, C₁-C₈alkyl, C₁-C₈haloalkyl, C₁-C₈alkoxy, C₁-C₈haloalkoxy;
each R¹¹ is independently halogen, cyano, nitro, C₁-C₈alkyl, C₁-C₈haloalkyl, C₁-C₈alkoxy, C₁-C₈haloalkoxy or C₁-C₈alkoxycarbonyl;
each R¹² is independently hydrogen, cyano, C₁-C₈alkyl, C₁-C₈haloalkyl, C₁-C₈alkylcarbonyl, C₁-C₈haloalkylcarbonyl, C₁-C₈alkoxycarbonyl, C₁-C₈haloalkoxycarbonyl, C₁-C₈alkylsulfonyl, C₁-C₈haloalkylsulfonyl, phenyl-C₁-C₄alkylene or phenyl-C₁-C₄alkylene where the phenyl moiety is substituted by one to three R¹¹, or pyridyl-C₁-C₄alkylene or pyridyl-Ci-C₄alkylene where the pyridyl moiety is substituted by one to three R¹¹;
R¹³ is halogen or imidazole, preferably chloro, fluoro or bromo;
R¹⁵ and R¹⁶ are each independently hydrogen, halogen, cyano, C₁-C₄alkyl or C₁-C₄haloalkyl;
R¹⁷ is hydrogen, C₁-C₈alkyl, C₁-C₈alkoxy, C₁-C₈alkylcarbonyl, or C₁-C₈alkoxycarbonyl;
R¹⁸ is C₁-C₄alkylcarbonyl or C₁-C₄alkylcarbonyl substituted by one to five R⁸, C₃-C₆ cycloalkylcarbonyl or C₃-C₆cycloalkylcarbonyl wherein the cycloalkyl is substituted by one to five R⁹;
R²⁰ is hydrogen or C₁-C₈alkyl, preferably hydrogen;
each Z¹ is independently hydrogen, halogen, methyl, halomethyl, methoxy or halomethoxy;
R²⁶ is -N(R²⁸)(R²⁹), halogen, hydroxy, C₁-C₈alkoxy, C₁-C₈haloalkoxy, C₁-C₈alkoxycarbonyl, C₁-C₈haloalkoxycarbonyl, or -CO₂H;
R²⁷ is hydrogen, C₁-C₈alkyl, C₁-C₈alkoxy, or C₁-C₈haloalkoxy.
R²⁸ is C₁-C₆alkyl-carbonyl, C₁-C₆haloalkyl-carbonyl, C₃-C₆cycloalkyl-C₁-C₂alkylcarbonylor C₃-C₆cycloalkyl-carbonyl;
R²⁹ is hydrogen, C₁-C₆alkoxy or benzyl.

In one group of compounds, group A4, applicable to all compounds of the invention bearing a group R¹, R² and A', R¹ and R² are as defined in group A1 and A' is as defined in group A2.

In one group of compounds, group A5, applicable to all compounds of the invention bearing a group R¹, R² and A', R¹ and R² are as defined in group A1 and A' is as defined in group A3.

In one group of compounds, group A6, applicable to all compounds of the invention bearing a group P, P is C₁-C₆alkoxy, N-pyrrolyl, N-imidazolyl, N-1,2,4-triazolyl, N-benzotriazolyl, or C₁-C₆alkylsulfinyl.

In one group of compounds, group A7, applicable to all compounds of the invention bearing a group P, optionally P is not C₁-C₆alkoxy, N-pyrrolyl, N-imidazolyl, N-1,2,4-triazolyl, N-benzotriazolyl, or C₁-C₆alkylsulfinyl.

In one group of compounds, group A8, applicable to all compounds of the invention bearing a group R², R² is aryl or aryl substituted by one to five R⁷⁰, or heteroaryl or heteroaryl substituted by one to five R⁷⁰, preferably phenyl or phenyl substituted by one to five R⁷⁰, more preferably phenyl substituted by one to three R⁷⁰, even more preferably R² is 3-chloro-5-trifluoromethyl-phenyl-, 3,5-dichloro-phenyl-, 3,5-bis-(trifluoromethyl)-phenyl-, 3,5-dichloro-4-fluoro-phenyl-, 3,4,5-trichloro-phenyl- or 3-trifluoromethyl-phenyl-, most preferably 3,5-dichloro-phenyl; each R⁷⁰ is independently halogen, cyano, nitro, C₁-C₈alkyl, C₁-C₈haloalkyl, C₂-C₈alkenyl, C₂-C₈haloalkenyl, C₂-C₈alkynyl, C₂-C₈haloalkynyl, hydroxy, C₁-C₈alkoxy-, C₁-Cshaloalkoxy-, mercapto, C₁-C₈alkylthio-, C₁-C₈haloalkylthio-, C₁-C₈alkylsulfinyl-, C₁-Cshaloalkylsulfinyl-, C₁-C₈alkylsulfonyl-, C₁-C₈haloalkylsulfonyl-, C₁-C₈alkylcarbonyl-, C₁-C₈alkoxycarbonyl-, aryl or aryl substituted by one to five R⁷¹, or heterocyclyl or heterocyclyl substituted by one to five R⁷¹; preferably halogen, cyano, C₁-C₈alkyl, C₁-C₈haloalkyl or C₁-C₈alkoxy-, more preferably bromo, chloro, fluoro, cyano, methyl, trifluoromethyl, methoxy or trifluoromethoxy, preferably bromo, chloro, fluoro or trifluoromethyl, most preferably bromo or chloro; each R⁷¹ is independently halogen, cyano, nitro, C₁-C₈alkyl, C₁-C₈haloalkyl, C₁-C₈alkoxy-, C₁-C₈haloalkoxy- or C₁-C₈alkoxycarbonyl-, preferably bromo, chloro, fluoro, cyano, nitro, methyl, ethyl, trifluoromethyl, methoxy, difluoromethoxy or trifluoromethoxy, more preferably bromo, chloro, fluoro, nitro or methyl, most preferably chloro, fluoro or methyl.

In one group of compounds, group A9, applicable to all compounds of the invention bearing a group R², optionally R² is not aryl or aryl substituted by one to five R⁷⁰, or heteroaryl or heteroaryl substituted by one to five R⁷⁰, each R⁷⁰ is independently halogen, cyano, nitro, C₁-Csalkyl, C₁-C₈haloalkyl, C₂-C₈alkenyl, C₂-C₈haloalkenyl, C₂-C₈alkynyl, C₂-C₈haloalkynyl, hydroxy, C₁-C₈alkoxy-, C₁-C₈haloalkoxy-, mercapto, C₁-C₈alkylthio-, C₁-C₈haloalkylthio-, C₁-Csalkylsulfinyl-, C₁-C₈haloalkylsulfinyl-, C₁-C₈alkylsulfonyl-, C₁-C₈haloalkylsulfonyl-, C₁-C₈alkylcarbonyl-, C₁-C₈alkoxycarbonyl-, aryl or aryl substituted by one to five R⁷¹, or heterocyclyl or heterocyclyl substituted by one to five R⁷¹; each R⁷¹ is independently halogen, cyano, nitro, C₁-C₈alkyl, C₁-C₈haloalkyl, C₁-C₈alkoxy-, C₁-C₈haloalkoxy- or C₁-C₈alkoxycarbonyl-.

In one group of compounds, group A10, applicable to all compounds of the invention bearing a group R², R² is phenyl substituted by one to three R⁷; each R⁷ is independently halogen, cyano, C₁-C₈alkyl, C₁-C₈haloalkyl or C₁-C₈alkoxy-;
In one group of compounds, group A11, applicable to all compounds of the invention bearing a group R², optionally R² is not phenyl substituted by one to three R⁷⁰; each R⁷⁰ is independently halogen, cyano, C₁-C₈alkyl, C₁-C₈haloalkyl or C₁-C₈alkoxy-;
In one group of compounds, group A12, applicable to all compounds of the invention bearing a group R², R² is 3-chloro-5-trifluoromethyl-phenyl-, 3,5-dichloro-phenyl-, 3,5-bis-(trifluoromethyl)-phenyl-, 3,5-dichloro-4-fluoro-phenyl-, 3,4,5-trichloro-phenyl- or 3-trifluoromethyl-phenyl-.

In one group of compounds, group A13, applicable to all compounds of the invention bearing a group R², optionally R² is not 3-chloro-5-trifluoromethyl-phenyl-, 3,5-dichloro-phenyl-, 3,5-bis-(trifluoromethyl)-phenyl-, 3,5-dichloro-4-fluoro-phenyl-, 3,4,5-trichloro-phenyl- or 3-trifluoromethyl-phenyl-.

In one group of compounds, group A14, applicable to all compounds of the invention bearing a group R², R² is aryl or aryl substituted by one to five R⁷⁰, or heteroaryl or heteroaryl substituted by one to five R⁷⁰, preferably phenyl or phenyl substituted by one to five R⁷; each R⁷⁰ is independently halogen, cyano, nitro, C₁-C₈alkyl, C₁-C₈haloalkyl, C₂-C₈alkenyl, C₂-C₈haloalkenyl, C₂-C₈alkynyl, C₂-C₈haloalkynyl, hydroxy, C₁-C₈alkoxy-, C₁-C₈haloalkoxy-, mercapto, C₁-C₈alkylthio-, C₁-C₈haloalkylthio-, C₁-C₈alkylsulfinyl-, C₁-C₈haloalkylsulfinyl-, C₁-C₈alkylsulfonyl-, C₁-C₈haloalkylsulfonyl-, C₁-C₈alkylcarbonyl-, C₁-C₈alkoxycarbonyl-, aryl or aryl substituted by one to five R⁷¹, or heterocyclyl or heterocyclyl substituted by one to five R⁷¹; each R⁷¹ is independently halogen, cyano, nitro, C₁-C₈alkyl, C₁-C₈haloalkyl, C₁-C₈alkoxy-, C₁-C₈haloalkoxy- or C₁-C₈alkoxycarbonyl-.

In one group of compounds, group A15, applicable to all compounds of the invention bearing a group R², optionally R² is not aryl or aryl substituted by one to five R⁷⁰, or heteroaryl or heteroaryl substituted by one to five R⁷⁰, preferably phenyl or phenyl substituted by one to five R⁷; each R⁷⁰ is independently halogen, cyano, nitro, C₁-C₈alkyl, C₁-C₈haloalkyl, C₂-C₈alkenyl, C₂-C₈haloalkenyl, C₂-C₈alkynyl, C₂-C₈haloalkynyl, hydroxy, C₁-C₈alkoxy-, C₁-Cshaloalkoxy-, mercapto, C₁-C₈alkylthio-, C₁-C₈haloalkylthio-, C₁-C₈alkylsulfinyl-, C₁-Cshaloalkylsulfinyl-, C₁-C₈alkylsulfonyl-, C₁-C₈haloalkylsulfonyl-, C₁-C₈alkylcarbonyl-, C₁-C₈alkoxycarbonyl-, aryl or aryl substituted by one to five R⁷¹, or heterocyclyl or heterocyclyl substituted by one to five R⁷¹; each R⁷¹ is independently halogen, cyano, nitro, C₁-C₈alkyl, C₁-C₈haloalkyl, C₁-C₈alkoxy-, C₁-C₈haloalkoxy- or C₁-C₈alkoxycarbonyl-.

In one group of compounds, group A16, applicable to all compounds of the invention bearing a group A', A' may be group C 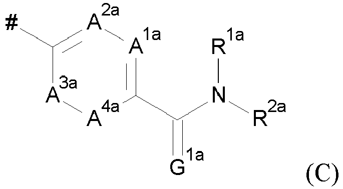 A^{1a}, A^{2a}, A^{3a} and A^{4a} are independently of each other C-H, C-R^{5a} or nitrogen; G^{1a} is oxygen or sulfur;
R^{1a} is hydrogen, C₁-C₈alkyl, C₁-C₈alkoxy-, C₁-C₈alkylcarbonyl-, C₁-C₈alkoxycarbonyl- or C₁-C₈haloalkoxycarbonyl-;
R^{2a} is a group of formula D 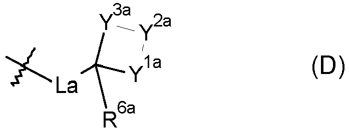 where
La is a single bond or C₁-C₆alkylene; and
Y^{1a}, Y^{2a} and Y^{3a} are independently of another CR^{8a}R^{9a}, C=O, C=N-OR^{10a}, N-R^{10a}, S, SO, SO₂, S=N-R^{10a} or SO=N-R^{10a}, provided that at least one of Y^{1a}, Y^{2a} or Y^{3a} is not CR^{8a}R^{9a}, C=O or C=N-OR^{10a}, preferably thietan-3-yl-, 1-oxo-thietan-3-yl-, 1,1-dioxo-thietan-3-yl- or 3-methyl-thietan-3-yl-, more preferably thietan-3-yl-, 1-oxo-thietan-3-yl-, or 1,1-dioxo-thietan-3-yl-;
each R^{5a} is independently halogen, cyano, nitro, C₁-C₈alkyl, C₁-C₈haloalkyl, C₂-Csalkenyl, C₂-C₈haloalkenyl, C₂-C₈alkynyl, C₂-C₈haloalkynyl, C₃-C₁₀cycloalkyl, C₁-C₈alkoxy-, C₁-C₈haloalkoxy-, C₁-C₈alkylthio-, C₁-C₈haloalkylthio-, C₁-C₈alkylsulfinyl-, C₁-C₈haloalkylsulfinyl-, C₁-C₈alkylsulfonyl- or C₁-C₈haloalkylsulfonyl-, or
   two R^{5a} on adjacent carbon atoms together form a -CH=CH-CH=CH- bridge;
R^{6a} is hydrogen, C₁-C₈haloalkyl or C₁-C₈alkyl;
each R^{8a} and R^{9a} is independently hydrogen, halogen, C₁-C₈alkyl or C₁-C₈haloalkyl;
each R^{10a} is independently hydrogen, cyano, C₁-C₈alkyl, C₁-C₈haloalkyl, C₁-C₈alkylcarbonyl-, C₁-C₈haloalkylcarbonyl-, C₁-C₈alkoxycarbonyl-, C₁-C₈haloalkoxycarbonyl-, C₁-C₈alkylsulfonyl-, C₁-C₈haloalkylsulfonyl-, aryl-C₁-C₄alkylene- or aryl-C₁-C₄alkylene- where the aryl moiety is substituted by one to three R^{12a}, or heteroaryl-C₁-C₄alkylene- or heteroaryl-Ci-C₄alkylene- where the heteroaryl moiety is substituted by one to three R^{12a};
each R^{11a} and R^{12a} is independently halogen, cyano, nitro, C₁-C₈alkyl, C₁-C₈haloalkyl, C₁-C₈alkoxy-, C₁-C₈haloalkoxy- or C₁-C₈alkoxycarbonyl-.

In one group of compounds, group A17, applicable to all compounds of the invention bearing a group A', optionally A' is not A' as defined in group A16.

Examples of chiral catalysts include chiral cinchona alkaloid derivatives, chiral thiourea derivatives, chiral urea derivatives, chiral aza-crown ether derivatives, chiral metal complexes, chiral amidine and guanidine derivatives, chiral pyrrolidine and imidazolidine derivatives, chiral scandium III complexes, chiral naphthyl phase transfer catalysts, chiral galodinium or strontium catalysts, chiral crown ether derivatives and chiral ligands for alkaline earth metals.

Chiral cinchona alkaloid derivatives are preferred and include alkaloid derivatives of the quaternary ammonium salts, tertiary amine derivatives, urea derivatives, thiourea derivatives and squaramide derivatives.

The term "chiral cinchona alkaloid derivatives" may overlap with the terms "chiral thiourea derivative" and "chiral urea derivative". Accordingly, the term "Chiral cinchona alkaloid derivatives" may in some embodiments exclude chiral thiourea derivatives and chiral urea derivatives. However, unless explicitly indicated the term "Chiral cinchona alkaloid derivatives" will include the relevant chiral thiourea derivatives and chiral urea derivatives.

In one embodiment the chiral catalysts are thiourea derivatives and chiral urea derivatives, in particular those that contain in the molecule a basic nitrogen atom in addition to the two nitrogen atoms of the urea or thiourea moiety, e.g. a primary, secondary or tertiary amine. Examples include chiral cinchona alkaloid thiourea derivatives, chiral cinchona alkaloid urea derivatives, thiourea derivatives of cyclohexanediamine and urea derivatives of cyclohexanediamine. Chiral cinchona alkaloid thiourea derivatives and thiourea derivatives of cyclohexanediamine are preferred.

For the nitromethane addition the preferred chiral catalysts are cinchona alkaloid derivatives, chiral thiourea derivatives and chiral metal complexes. These catalysts include those from groups 1, 2, 3, 4, 5, 7 and 11 below. Particularly preferred catalysts for are chiral cinchona alkaloid derivatives, particularly cinchona alkaloid derivatives of quaternary ammonium salts, cinchona alkaloid urea derivatives, cinchona alkaloid thiourea derivatives, and cinchona alkaloid squaramide derivatives. Even more preferred are cinchona alkaloid urea derivatives, cinchona alkaloid thiourea derivatives, most preferred being cinchona alkaloid thiourea derivatives.

For the cyanide addition the preferred catalysts are cinchona alkaloid derivatives, chiral ruthenium catalysts as well as gadolinium and strontium catalysts. These catalysts include those from groups 1, 2, 3, 4, 7 and 13. Most preferred catalysts are derivatives of cinchona alkaloid quaternary ammonium salts.

Examples of cinchona alkaloid quaternary ammonium salt derivatives include compounds of formula 1 (group 1) 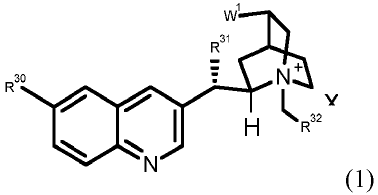 wherein
W¹ is ethyl or vinyl; R³⁰ is hydrogen or C₁-C₄alkoxy; R³¹ is hydroxyl, C₁-C₄alkoxy, C₂-C₄alkenyloxy, optionally substituted aryloxy, optionally substituted heteroaryloxy or optionally substituted benzyloxy; R³² is optionally substituted aryl or optionally substituted heteroaryl; X is an anion.

Preferably W¹ is vinyl.

Preferably R³⁰ is methoxy.

Preferably R³¹ is hydroxyl, C₁-C₄alkoxy, C₂-C₄alkenyloxy, optionally substituted heteroaryloxy or benzyloxy, more preferably hydroxyl, optionally substituted pyrimidinyloxy or benzyloxy, most preferably hydroxyl.

Preferably X is a halogen, more preferably chloride or bromide. Preferably R³² is phenyl or phenyl substituted by one to five R³³, naphthyl or naphthyl substituted by one to five R³³, anthracenyl or anthracenyl substituted by one to five R³³, or heteroaryl or heteroaryl substituted by one to four R³³; more preferably R³² is phenyl or phenyl substituted by one to five R³³, naphthyl or naphthyl substituted by one to five R³³, anthracenyl or anthracenyl substituted by one to five R³³, pyrimidinyl or pyrimidinyl substituted by one to three R³³, or pyridyl or pyridyl substituted by one to four R³³; more preferably phenyl or phenyl substituted by one to five R³³, naphthyl or naphthyl substituted by one to five R³³, anthracenyl or anthracenyl substituted by one to five R³³, or pyridyl or pyridyl substituted by one to four R³³; more preferably R³² is phenyl or phenyl substituted by one to five R³³, anthracenyl or anthracenyl substituted by one to five R³³, or pyridyl or pyridyl substituted by one to four R³³; even more preferably R³² is phenyl or phenyl substituted by one to five substituents independently selected from halogen, methyl and methoxy, anthracenyl or anthracenyl substituted by one to five substituents independently selected from halogen, methyl and methoxy, pyridyl or pyridyl substituted by one to four halogen atoms, or group B 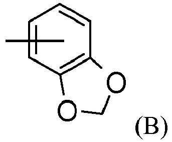 or group B substituted by one to four substituents independently selected from halogen, methyl and methoxy, even more preferably phenyl substituted by one to five substituents independently selected from halogen methyl and methoxy, anthracenyl or anthracenyl substituted by one to five substituents independently selected from halogen, methyl and methoxy or pyridyl or pyridyl substituted by one to four halogen atoms, even more preferably phenyl substituted by one to five substituents independently selected from halogen methyl and methoxy or anthracenyl. Each R³³ is independently halogen, cyano, nitro, C₁-C₈alkyl, C₁-C₈haloalkyl, C₁-C₈alkoxy, C₁-C₈haloalkoxy, C₃-C₈cycloalkyl, phenyl or phenyl substituted by one to five halogen, and wherein two R³³ substituents on adjacent carbon atoms may together form a partially saturated 5-7 membered ring containing one or two heteroatoms independently selected from O, N(R³⁴) and S; and each R³⁴ is independently hydrogen or C₁-C₄ alkyl. Preferably each R³³ is independently halogen, cyano, nitro, C₁-C₄alkyl, C₁-C₄haloalkyl, C₁-C₄alkoxy,arylor C₁-C₄haloalkoxy, and wherein any two R³³ substituents on adjacent carbon atoms may together form a partially saturated 5 membered ring containing one or two O atoms, more preferably each R³³ is independently halogen, methyl, halomethyl, methoxy, phenyl or halomethoxy, and wherein any two R³³ substituents on adjacent carbon atoms may together form a partially saturated 5 membered ring containing one or two O atoms, more preferably each R³³ is independently halogen, methyl, phenyl or methoxy, most preferably each R³³ is independently fluorine, methyl, phenyl or methoxy.

Examples include 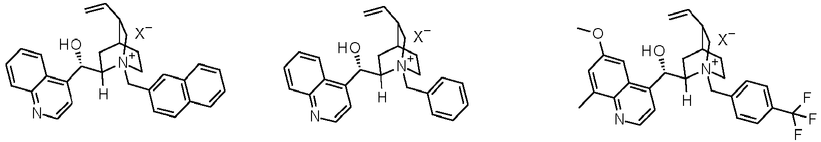 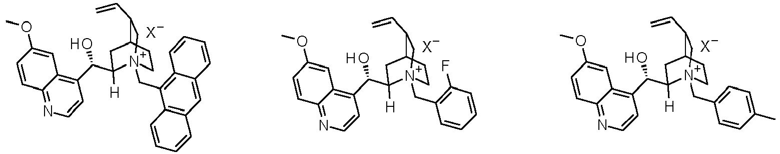 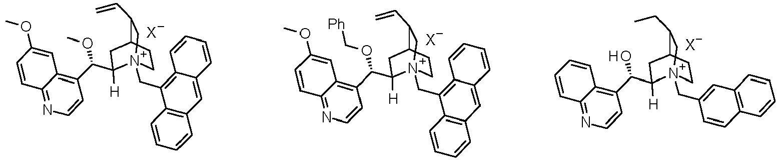 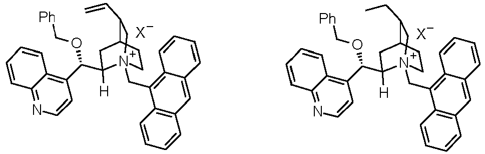 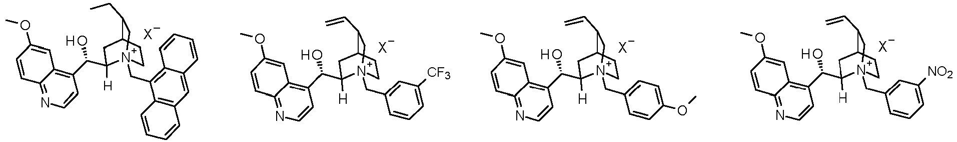 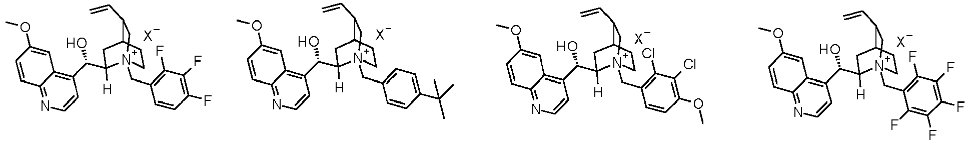 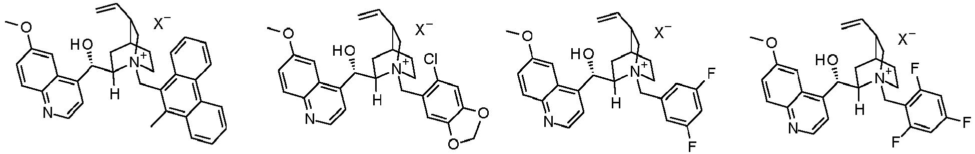 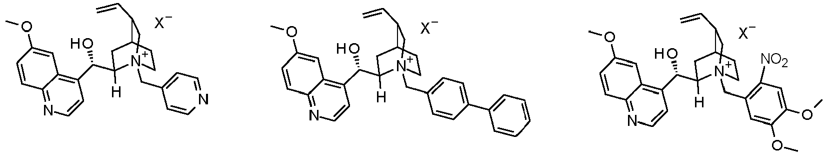 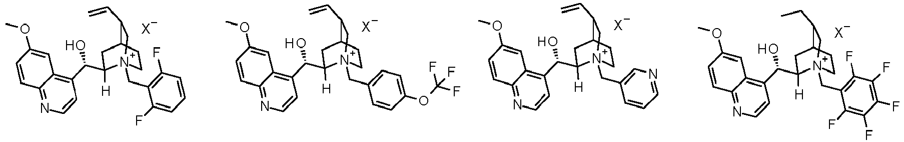 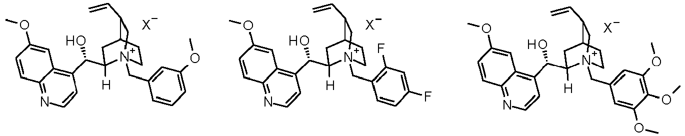 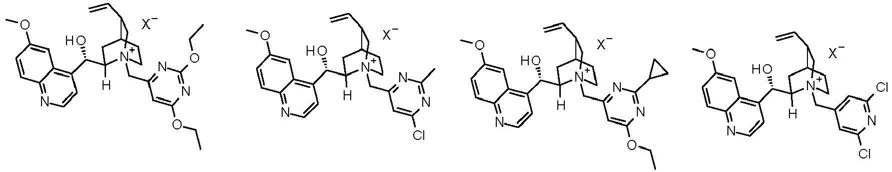 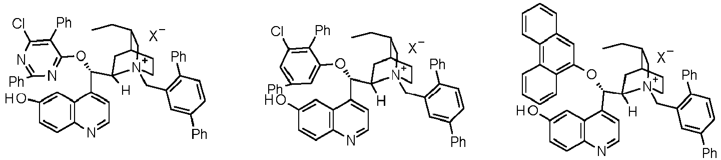 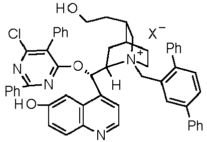 wherein X is an anion, preferably halogen, more preferably chloride or bromide.

Examples of cinchona alkaloid quaternary ammonium salt derivatives are described for example in Arai et al., Tet. Lett. 1999, 4215; S. Colonna, H. Hiemstra, H. Wynberg, J. Chem. Soc. Chem. Commun. 1978, 238; E. J. Corey, F. Y. Zhang, Org. Lett. 2000, 2, 4257; D. Y. Kim, S. C. Huh, Tetrahedron 2001, 57, 8933; M. Hua, H. Cui, L. Wang, J. Nie, J. Ma, Angew. Chem. 2010, 122, 2832; Angew. Chem. Int. Ed. 2010; and T. Ooi, K. Maruoka, Acc. Chem. Res. 2004, 37, 526; Provencher, B.A., Bartelson, K.J., Liu, Y., Foxman, B., Deng, L. Angew.Chem,.Int.Ed. 2011, 50,10565; Liu, Y., Provencher, B.A., Bartelson, K.J., Deng, L. Chem.Sci. 2011, 2, 1301

Examples of cinchona alkaloid tertiary amine derivatives include compounds of formula 2 (group 2) 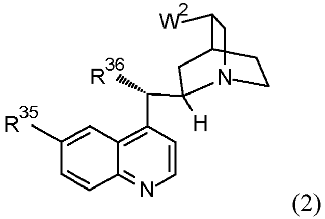 W² is ethyl or vinyl; R³⁵ is hydrogen or C₁-C₄alkoxy; R³⁶ is hydroxyl, C₁-C₄alkoxy, C₂-C₄alkenyloxy or optionally substituted benzyloxy.

Preferably W² is vinyl.

Preferably R³⁵ is methoxy.

Preferably R³⁶ is hydroxyl, C₁-C₄alkoxy, C₂-C₄alkenyloxy or benzyloxy, most preferably hydroxyl.

Examples include: 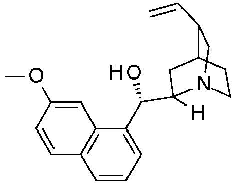 as described in A. Latvala, S. Stanchev, A. Linden, M. Hesse, Tet. Asym. 1993, 2, 173.

Examples of cinchona alkaloid urea and thiourea derivatives include compounds of formula 3 (group 3) 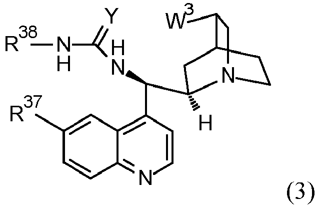 Y is S or O, W³ is ethyl or vinyl; R³⁷ is hydrogen or C₁-C₄alkoxy; R³⁸ is optionally substituted aryl or optionally substituted C₃-C₁₀cycloalkyl.

Preferably Y is S.

Preferably W³ is vinyl or ethyl.

Preferably R³⁷ is methoxy.

Preferably R³⁸ is phenyl optionally substituted by one to five R³⁹ or C₅-C₆cycloalkyl optionally substituted by R⁴⁰, more preferably phenyl optionally substituted by one to five R³⁹.

R³⁹ is halogen, cyano, C₁-C₄alkyl, C₁-C₄haloalkyl, C₁-C₄alkoxy, C₁-C₄haloalkoxy, preferably C₁-C₄ haloalkyl, more preferably C₁-C₄haloalkyl.

R⁴⁰ is NH₂, halogen, cyano, C₁-C₄alkyl, C₁-C₄haloalkyl, C₁-C₄alkoxy, C₁-C₄haloalkoxy, preferably NH₂.

Examples include 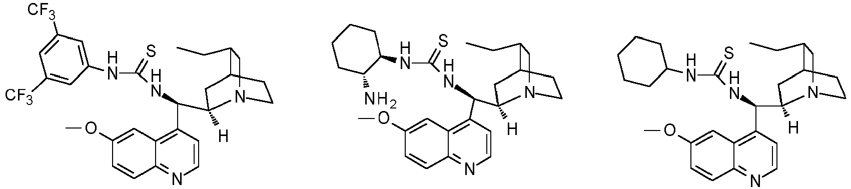 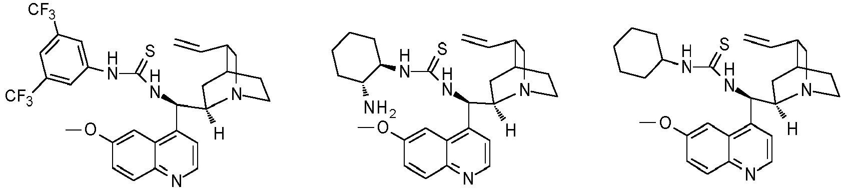 as described in B. Vakulya, S. Varga, A. Csámpai, T. Soós, Org. Lett. 2005, 7, 1967; B. Vakulya, S. Varga, T. Soós, J. Org. Chem. 2008, 73, 3475; P. Li, Y. Wang, X. Liang, J. Ye, Chem. Commun. 2008, 3302; and C. Oliva, A. Silva, F. Paz, J. Calvaleiro, Synlett, 2010, 7, 1123-1127.

Examples of squaramide catalysts include compound of formula 4 (group 4) 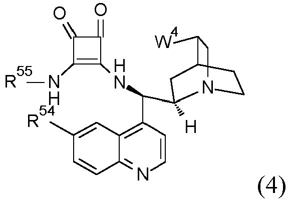 wherein W⁴ is ethyl or vinyl; R⁵⁴ is hydrogen or C₁-C₄alkoxy; R⁵⁵ is optionally substituted aryl.

Preferably W⁴ is vinyl

Preferably R⁵⁴ is methoxy.

Preferably R⁵⁵ is phenyl optionally substituted by one to five R⁵⁶ or C₅-C₆cycloalkyl optionally substituted by R⁴⁰.

R⁵⁶ is halogen, cyano, C₁-C₄alkyl, C₁-C₄haloalkyl, C₁-C₄alkoxy, C₁-C₄haloalkoxy, preferably C₁-C₄haloalkyl.

Examples include those wherein in the compound of formula X, R⁵⁴ is H or OMe and R⁵⁵ is 4-CF₃-C₆H₄ or 3,5-(CF₃)₂-C₆H₃ as described in Yang, W.; Du, D. Org. Lett., 2010, 12 (23), 5450-5453.

Examples of thiourea derivatives of cyclohexanediamine or diamines (group 5) include the following

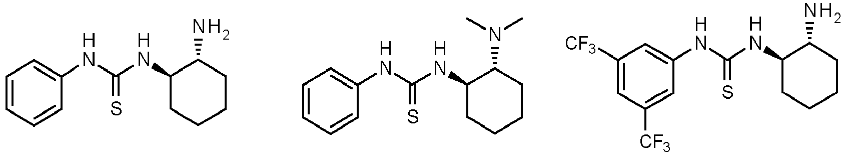

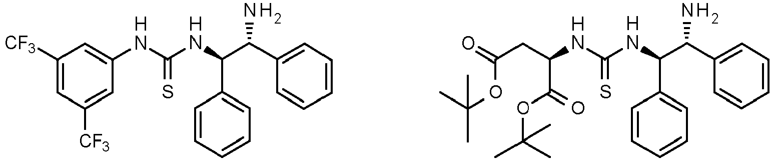

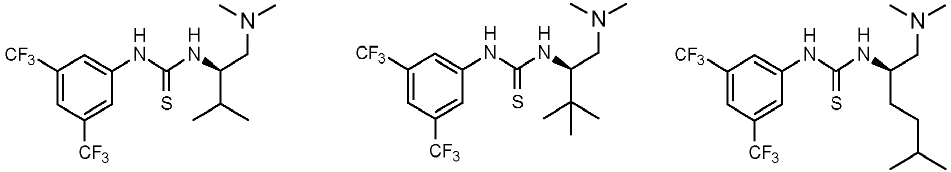

Examples of thiourea derivatives of cyclohexanediamine are described in K. Mei, M. Jin, S. Zhang, P. Li, W. Liu, X. Chen, F. Xue, W. Duan, W. Wang, Org. Lett. 2009, 11, 2864, and B. Vakulya, S. Varga, T. Soós, J. Org. Chem. 2008, 73, 3475.
Examples of thiourea derivatives of diamines are described in He, Tianxiong; Qian, Jing-Ying; Song, Hong-Liang; Wu, Xin-Yan Synlett 2009, 19, 3195-319 and Kokotos, C. G.; Kokotos, G., Advanced Synthesis & Catalysis 2009, 351(9), 1355-1362 and Manzano, R.; Andres, J.M.; Alvarez, R.; Muruzabal, M.D.; de Lera, A.R.; Pedrosa, R. Chem. Eur. J. 2011, 17, 5931.

Examples of aza-crown ethers (group 6) include compound of formula 5 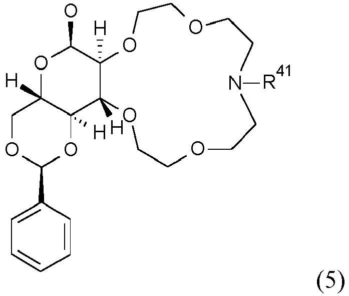 R⁴¹ is hydrogen, C₁-C₁₀alkyl, C₁-C₁₀hydroxyalkyl C₁-C₈alkoxy-C₁-C₈alkyl, C₁-C₈alkoxycarbonyl, C₁-C₈alkyl optionally substituted aryl, aryl-C₁-C₄alkyl wherein the aryl is optionally substituted, (aryl)₂P(O)C₁-C₄ alkyl wherein each aryl is optionally substituted.

Preferably R⁴¹ is hydrogen, C₁-C₁₀alkyl, C₁-C₁₀hydroxyalkyl, C₁-C₈alkoxy-C₁-C₈alkyl, C₁-C₈alkoxycarbonyl-C₁-C₈alkyl, phenyl, phenyl-C₁-C₄alkyl, (phenyl)₂P(O)C₁-C₄ alkyl.

Examples of aza crown ethers include those wherein R⁴¹ is C₆H₅, CH₂C₆H₅, CH₃-(CH₂)₃, CH₃-(CH₂)₉, CH₂CH₂OH, C₆H₁₁, CH₂CO₂CH₃, hydrogen, CH₂CH₂OCH₃, (CH₂)₄P(O)Ph₂.

Examples of aza-crown ethers are described in P. Bakó, A. Szöllősy, P. Bombicz, L. Tőke, Synlett 1997, 291 and T. Bakó, P. Bakó, A. Szöllősy, M. Czugler, G. Keglevich, L. Tőke, Tet. Asym. 2002, 203.

Examples of chiral metal complexes (group 7) include the following 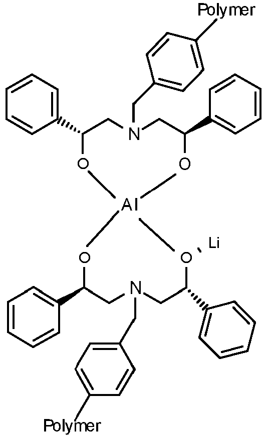 as described in G. Sundararajan, N. Prabagaran, Org. Lett. 2001, 3, 389; 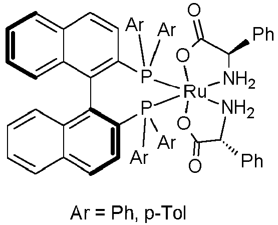 as described in Kurono, N.; Nii, N.; Sakaguchi, Y.; Uemura, M.; Ohkuma, T. Angew. Chem. Int. Ed. 2011, 50, DOI: 10.1002/anie.201100939 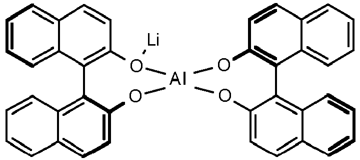 as described in. Keller, N. Veldman, A. L. Spek, B. L. Feringa, Tetrahedron: Asymmetry 1997, 8, 3403; LaK₃tris((R)-binaphthoxide)) as described in K. Funabashi, Y. Saida, M. Kanai, T. Arai, H. Sasai, M. Shibasaki, Tetrahedron Lett. 1998, 39, 7557; and 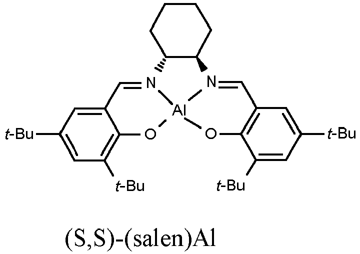 variations thereof include [(S,S)-(salen)Al]₂O, (S,S)-(salen)AlMe, (S,S)-(salen)AlCl and are described in M. S. Taylor, D. N. Zalatan, A. M. Lerchner, E. N. Jacobsen, J. Am. Chem. Soc. 2005, 127, 1313; 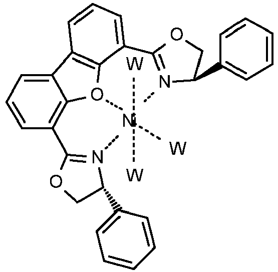 in combination with an achiral amine, e.g. 2,2,6,6-tetramethylpiperidine, as described in K. Itoh, S. Kanemasa, J. Am. Chem. Soc. 2002, 124, 13394.

Examples of chiral amidines and guanidines (group 8) include compounds of formula 6 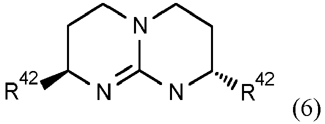 wherein each R⁴² is C(H)Ph₂, or CH₂OR⁴³, wherein R⁴³ is *t*-BuPh₂Si, H or benzyl, e.g. as described in A. P. Davis, K. J. Dempsey, Tetrahedron: Asymmetry 1995, 6, 2829; 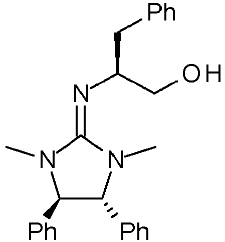 as described in Zhang, G.; Kumamoto, T.; Heima, T.; Ishikawa, T. Tetrahedron Lett. 2010, 51, 3927. 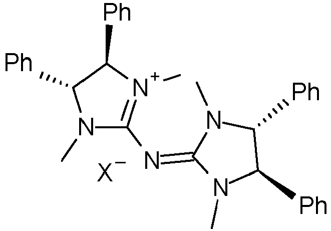 Where X is a halogen or BF₄ of PF₆, most preferably chloride as described in Ma, T.; Fu, X.; Kee, C.W.; Zong, L.; Pan, Y.; Huang, K.; Tan, C. J. Am. Chem. Soc. 2011, 133, 2828 and 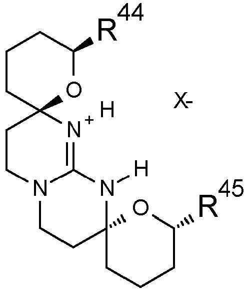 wherein R⁴⁴ and R⁴⁵ are independently C₁-C₄ alkyl, C₁-C₄ alkoxy-C₁-C₄ alkyl, TBDMS-C₁-C₄ alkyl or TBDPS-C₁-C₄ alkyl, preferably both R⁴⁴ and R⁴⁵ are either hydroxymethyl, TMDMS-methyl or TBDPS-methyl, and wherein X is an anion, preferably halogen or BF₄⁻, more preferably chloride or BF₄⁻, e.g. as described in M. T. Allingham, A. Howard-Jones, P. J. Murphy, D. A. Thomas, P. W. R. Caulkett, Tetrahedron Lett. 2003, 44, 8677.

Examples of the pyrrolidine derivatives as chiral catalysts (group 9) include proline, e.g. in combination with trans-2,5-dimethylpiperazine as described in S. Hanessian, V. Pham, Org. Lett. 2000, 2, 2975; 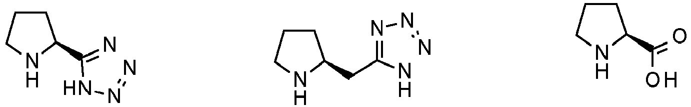 as described in C. E. T. Mitchell, S. E. Brenner and S. V. Ley, Chem. Commun., 2005, 5346 and C. E. T. Mitchell, S. E. Brenner, J. Garcia-Fortanet and S. V. Ley, Org. Biomol. Chem., 2006, 4, 2039; 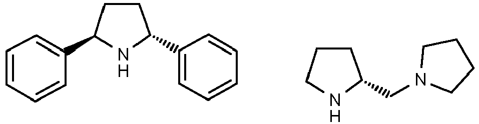 as described in N. Halland, R. G. Hazell, K. A. Jorgensen, J. Org. Chem. 2002, 67, 8331; 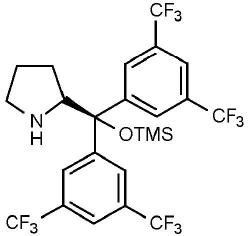 as described in C. Oliva, A. Silva, F. Paz, J. Calvaleiro, Synlett, 2010, 7, 1123-1127; and 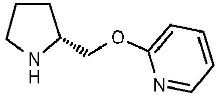 as described in Xu, D.; Shi, S.; Wang, Y. European Journal of Organic Chemistry 2009, (28), 4848-4853.

Examples of chiral imidazoline catalysts (group 10) include 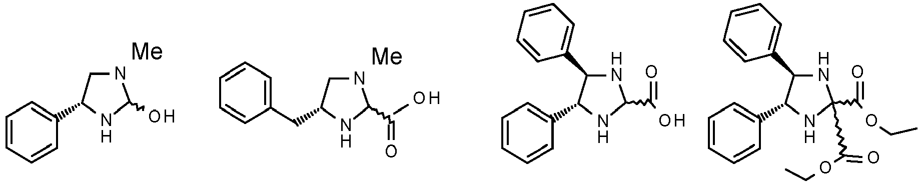 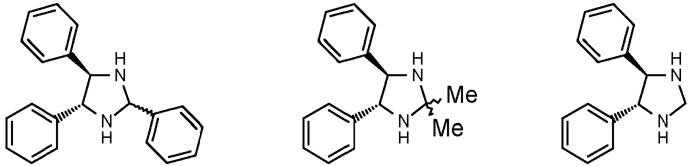 as described in N. Halland, R. G. Hazell, K. A. Jorgensen, J. Org. Chem. 2002, 67, 8331; and 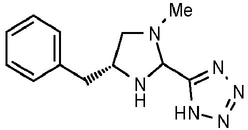 as described in A. Prieto, N. Halland, K. A. Jorgensen, Org. Lett. 2005, 7, 3897.

Examples of chiral N,N'-dioxide-scandium III complexes (group 11) include ligand-Sc(OTf)₃ complexes wherein the ligand is a compound of formula 7 or 8 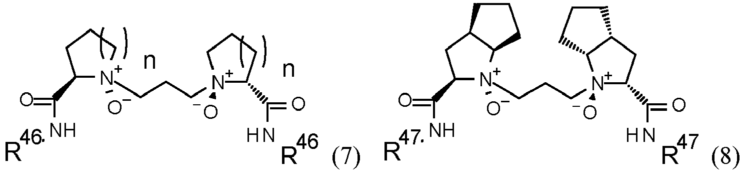 wherein R⁴⁶ and R⁴⁷ are phenyl optionally substituted by one to five halogen, C₁-C₄ alkyl, C₁-C₄ haloalkyl, C₁-C₄ alkoxy, C₁-C₄ haloalkoxy and wherein n is 1 or 2;
Examples include those wherein n is 1 and R⁴⁶ is 2,6-iPr₂C₆H₃; n is 1 and R⁴⁶ is C₆H₅; n is 1 and R⁴⁶ is 2-MeC₆H₄; n is 2 and R⁴⁶ is 2,6-iPr₂C₆H₃; R⁴⁷ is 2,6-iPr₂-C₆H₃; as described in L. Wang, Q. Zhang, X. Zhou, X. Liu, L. Lin, B. Qin, X. Feng, Chemistry-A European Journal, 2010, 16, (26), 7696-7699,
Chiral binaphthyl phase transfer catalysts (group 12) include compounds of formula 11, 12, 13and 14 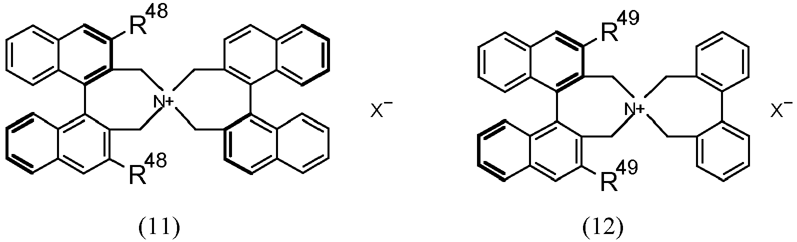 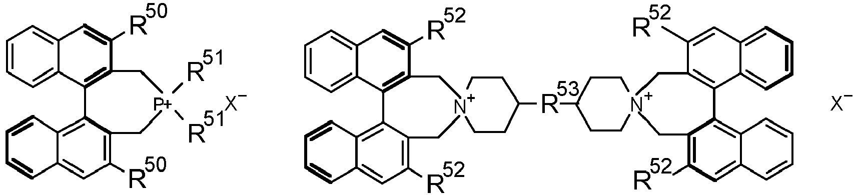 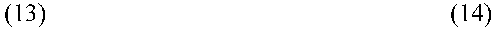 wherein R⁴⁸, R⁴⁹, R⁵⁰ and R⁵² are each independently phenyl or naphthyl optionally substituted by one to five halogen, C₁-C₄ alkyl, C₁-C₄ haloalkyl, C₁-C₄ alkoxy, C₁-C₄ haloalkoxy; each R⁵¹ is C₁-C₈ alkyl or C₁-C₈ haloalkyl, R⁵³ is a bond or C₁-C₈ alkylene and X is an anion, e.g. a halogen, preferably chlorine or bromine. Examples include those wherein each R⁴⁸ is 3,5-(CF₃)₂(C₆H₃); each R⁴⁸ is 3,4,5-F₃C₆H₂; each R⁴⁹ is 3,5-(CF₃)₂(C₆H₃); each R⁴⁹ is 3,4,5-F₃C₆H₂; each R⁵⁰ is 3,5-(CF₃)₂(C₆H₃); each R⁵⁰ is 3,4,5-F₃C₆H₂; each R⁵¹ is n-butyl; each R⁵² is H and R⁵³ is a bond; each R⁵² is H and R⁵³ is ethylene; each R⁵² is H and R⁵³ is propylene; each R⁵² is phenyl and R⁵³ is a bond; each R⁵² is phenyl and R⁵³ is ethylene; each R⁵² is phenyl and R⁵³ is propylene; each R⁵² is 3,4,5-F₃C₆H₂ and R⁵³ is a bond; each R⁵² is 3,4,5-F₃C₆H₂ and R⁵³ is ethylene; each R⁵² is 3,4,5-F₃C₆H₂ and R⁵³ is propylene; each R⁵² is ,5-(CF₃)₂C₆H₂ and R⁵³ is a bond; each R⁵² is ,5-(CF₃)₂C₆H₂ and R⁵³ is ethylene; each R⁵² is 3,5-(CF₃)₂C₆H₂ and R⁵³ is propylene; each R⁴⁸ is 2-naphthyl as described in M. Hua, H. Cui, L. Wang, J. Nie, J. Ma, Angew. Chem. 2010, 122, 2832 and T. Ooi, K. Maruoka, Acc. Chem. Res. 2004, 37, 526.

Examples of ligands for galodinium or strontium catalysis (group 13) include compounds of formula 15 and 16 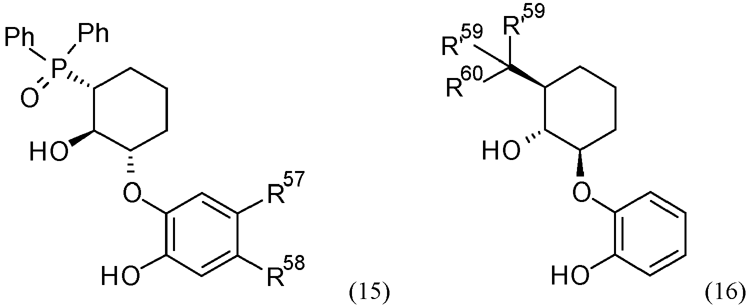 wherein R⁵⁷ is CN or F, R⁵⁸ is H or F; each R⁵⁹ is phenyl or p-tolyl; R⁶⁰ is OH, OMe or O*i*-Bu as described in Tanaka, Y.; Kanai, M.; Shibasaki, M. J. Am. Chem. Soc. 2008, 130, 6072; Tanaka, Y.; Kanai, M.; Shibasaki, M. J. Am. Chem. Soc. 2010, 132, 8862.

Examples of crown ether phase transfer catalysis (group 14) include compounds of formula XXI 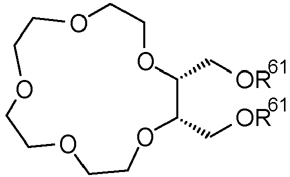 wherein each R⁶¹ is is H or benzyl as described in Dehmlow, D.E.; Sauerbier, C. Liebigs Ann. Chem. 1989, 181-185.

Examples of ligands for alkaline earth metal catalysis (group 15) include 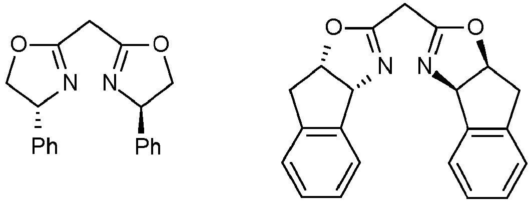 as described in Saito, S.; Tsubogo, T.; Kobayashi, S. J. Am. Chem. Soc. 2007, 129, 5364; Tsubogo, T.; Saibo, S.; Seki, K.; Yamashita, Y.; Kobayashi, S. J. Am. Chem. Soc. 2008, 130, 13321; Kobayashi, S.; Tsubogo, T.; Saito, S.; Yamashita, Y. Org. Lett. 2008, 10, 807

It will be clear to the person skilled in the art that in order to prepare the compounds of the invention with the indicated stereochemistry, the stereochemistry of the compound of formula II must be matched with the corresponding stereochemistry of the catalyst. It is understood that the stereochemistry of the catalysts depicted above is appropriate for a compound of formula IA: 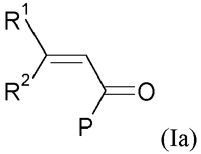 The following schemes describe the processes of the invention in more detail. In the schemes below the stereochemistry at * corresponds to the stereochemistry in the claims. The substituent definitions are as defined herein. 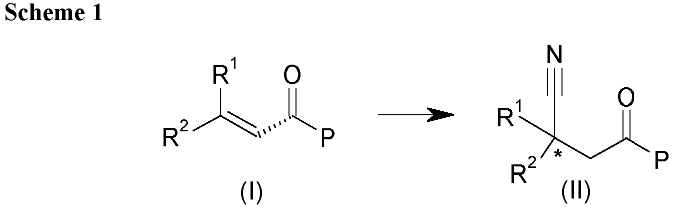
1) Enantioenriched compounds of formula (II) can be prepared by reacting a compound of formula (I) with a suitable cyanide source in the presence of a chiral catalyst. Suitable cyanide sources include, but are not limited to alkali metal cyanides, trimethylsilyl and tert-butyldimethylsilyl cyanides, hydrogen cyanide, CNCO₂Et and acetone cyanohydrin. Depending from the catalyst used, suitable solvents include dioxane, tetrahydrofuran, dichloromethane, t-butylmethyl ether, 1,2-dichloroethane, dimethoxyethane, xylenes and toluene. In certain cases additives such as cesium fluoride, cesium chloride, lithium phenolate or 2,6-dimethylphenol are often required. In most cases it is advantageous to conduct the reaction in a suitable solvent at dilution between 0.1 M to 1 M, preferably 0.3 M to 0.5 M. The reaction temperature could be from -40 °C to 100 °C, preferably between -20 °C and 50 °C. The reaction time is usually between 1 hour and 96 hours, preferably between 6 hours and 24 hours. The amount of catalyst is usually between 0.02 and 0.2 molar equivalents, preferably between 0.05 and 0.1 molar equivalents. Certain catalysts require a presence of a Lewis acid, such as galodinium trifluoromethansulfonate or strontium trifluoromethanesulfonate. If chiral phase transfer catalysts of group I are used the addition of small amounts of water (between one and four molar equivalents) is often beneficial. Conducting the reaction in a biphasic system (water/suitable organic solvent) is, however, usually detrimental to chemical reactivity. Suitable conditions for this asymmetric reaction are disclosed in the literature: (a) Sammis, G. M.; Jacobsen, E. N. J. Am. Chem. Soc. 2003, 125, 4442. (b) Sammis, G. M.; Danjo, H.; Jacobsen, E. N. J. Am. Chem. Soc. 2004, 126, 9928. (c) Mazet, C.; Jacobsen, E. N. Angew. Chem., Int. Ed. 2008, 47, 1762. (d) Madhavana, N.; Weck, M. AdV. Synth. Catal. 2008, 350, 419. (e) Mita, T.; Sasaki, K.; Kanai, M.; Shibasaki, M. J. Am. Chem. Soc. 2005, 127, 514. (f) Fujimori, I.; Mita, T.; Maki, K.; Shiro, M.; Sato, A.; Furusho, S.; Kanaia, M.; Shibasaki, M. Tetrahedron 2007, 63, 5820. (g) Tanaka, Y.; Kanai, M.; Shibasaki, M. J. Am. Chem. Soc. 2008, 130, 6072. (h) Bernardi, L.; Fini, F.; Fochi, M.; Ricci, A. Synlett 2008, 1857. (i) Jun Wang, Wei Li, Yanling Liu, Yangyang Chu, Lili Lin, Xiaohua Liu, and Xiaoming Feng Organic Letters (2010), 12, (6), 1280-1283. (j) anaka, Yuta; Kanai, Motomu; Shibasaki, Masakatsu, Journal of the American Chemical Society 2010, 132, (26), 8862-8863. (k) Brian A. Provencher, Keith J. Bartelson, Yan Liu, Bruce M. Foxman, Li Deng, Angewandte Chemie International Edition.

### Scheme 2

enantioenriched compounds of formula (V) can be directly obtained by a reductive cyclization of enantioenriched compound of formula (II) under the conditions described above. 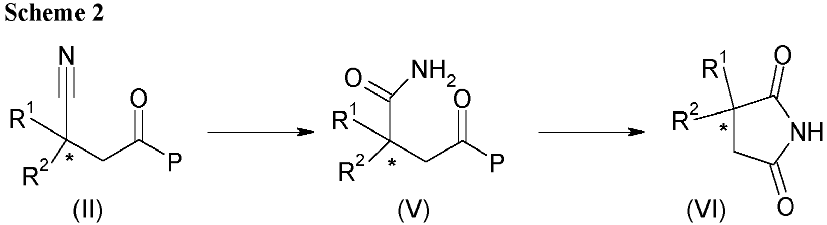
2) Enantioenriched Compounds of formula (VI) can be obtained by a cyclization of enantioenriched compound of formula (V) wherein P is e.g. C₁-C₆alkoxy, N-pyrrolyl, N-imidazolyl, N-1,2-4-triazolyl, N-benzotriazolyl, C₁-C₆alkylsulfinyl under basic conditions such as those described in Tetrahedron, 39(19), 3055-7; 1983. 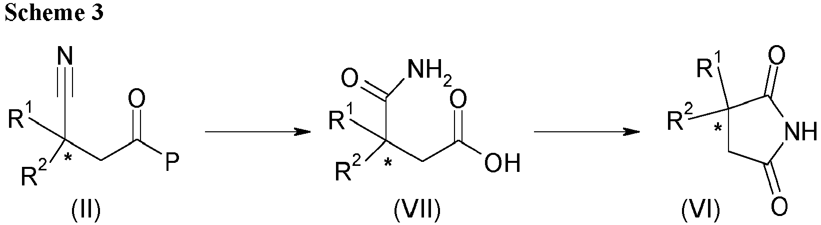
3) Enantioenriched compounds of formula (VI) can be obtained by a cyclization of enantioenriched compounds of formula (VII) by a dehydrating reaction such as those described in Chemistry-A European Journal, 9(14), 3270-3281; 2003.
4) Enantioenriched Compounds of formula (VII) can be obtained by complete hydrolysis of enantioenriched compound of formula (II) under basic aqueous conditions. 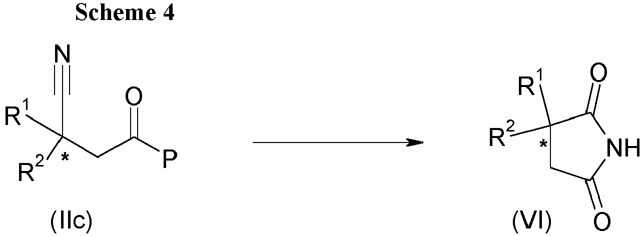
5) Enantioenriched Compounds of formula (VI) can be obtained by carrying out a Baeyer-Villiger oxidation reaction on enantioenriched compounds of formula (IIc) wherein P is e.g. an optionally substituted aryl or an optionally substituted heteroaryl. Suitable reagents for the reaction include, but are not limited to m-chloro peroxybenzoic acid, trifluoro peroxyacetic acid and peroxy sulfuric acid. Particularly preferred reagent is peroxysulfuric acid. Between 1 and 100 equivalents of the reagent is typically used (e.g. at least 1 equivalent, e.g. up to 100 equivalents).
   Alternatively, a suitable reagent is peroxide in the presence of acid, preferably a strong acid. Peroxides include, but are not limited to hydrogen peroxide, sodium peroxide, sodium perborate, sodium percarbonate, sodium persulfate, potassium persulfate. Particularly preferred is hydrogen peroxide. The concentration of hydrogen peroxide can be between 5% and 90%, preferably between 20-40% (e.g. at least 5%, at least 20%, e.g. up to 90%, up to 40%). (% refers to v/v.). Between 1 and 100 molar equivalents of the reagent is typically used (e.g. at least 1 molar equivalent, e.g. up to 100 molar equivalents).
   Strong acids are e.g. any acid with pKa lower then acetic acid. Strong acids include, but are not limited to trifluoroacetic acid, nitrobenzoic acid, methanesulfonic acid, benzenesulfonic acid, p-toluenesulfonic acid, sulfuric acid, Nafion-H. Particularly preferred is sulphuric acid. The concentration of acid, which is preferably sulphuric acid, can be between 10% and 99%, preferably between 50-97% (e.g. at least 10%, at least 50%, e.g. up to 99%, up to 97%) (% refers to v/v.) Between 1 and 100 molar equivalents of the reagent is typically used (e.g. at least 1 molar equivalent, e.g. up to 100 molar equivalents).
   The reaction can be conducted neat or in a suitable solvent. Suitable reagents for the reaction include, but are not limited to dichloromethane, dichloroethane, chloroform, carbon tetrachloride, acetic acid. The reaction temperature could be from -50 °C to 150 °C, preferably between -20 °C and 100 °C (e.g. at least -50 °C, at least -20 °C, e.g. up to 150 °C, up to 100° C). The reaction time is usually between 1 hour and 96 hours, preferably between 1 hour and 24 hours (e.g. at least 1 hour, e.g. up to 96 hours, up to 24 hours). 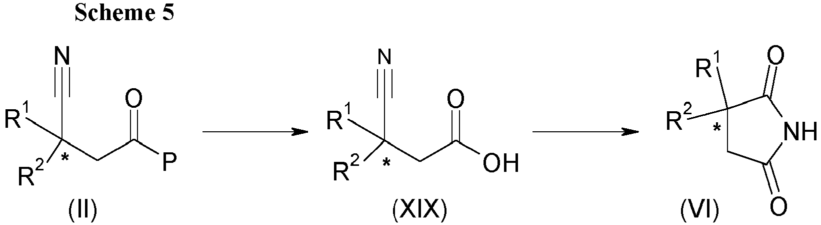
6) Enantioenriched Compounds of formula (VI) can be obtained by hydrolysis of the nitrile function in Enantioenriched compounds of formula (XIX) by acidic or basic hydrolysis followed by a dehydration reaction.
7) Enantioenriched Compounds of formula (XIX) can be obtained by hydrolysis of Enantioenriched compound of formula (II) under basic aqueous conditions.

In the above schemes a leaving group may befor example a halogen, C₁-C₈alkoxy, C₁-C₈alkylsulfonyloxy, C₁-C₈haloalkylsulfonyloxy, C₁-C₈arylsulfonyloxy, optionally substituted C₁-C₈arylsulfonyloxy (aryl is preferably phenyl), diazonium salts (e.g. X^{B} is -N₂⁺ Cl⁻, -N₂⁺ BF₄⁻, -N₂⁺ Br⁻, -N₂⁺ PF₆⁻⁾, phosphonate esters (e.g. -OP(O)(OR)₂, wherein R is methyl or ethyl), preferably bromo, iodo, chloro, trifluoromethylsulfoxy, p-toluenesulfoxy, diazonium chloride.

In the above schemes, where a reaction condition, e.g. temperature, time, concentration, is given as a range, e.g. value X to value Y, the skilled person will understand that these values serve as guidelines and that it may be possible to perform the reactions outside the given values. In addition, where such ranges are given, in each case these include separate disclsoures of "at least X", and "Y or less". For example a range of 50 °C to 150 °C includes s disclosure of "at least 50 °C" and a dislcosure of " 150 °C or less".

The following tables illustrate compounds relating to the invention. In the compounds disclosed in Tables the stereochemistry at * corresponds to that of formula II.

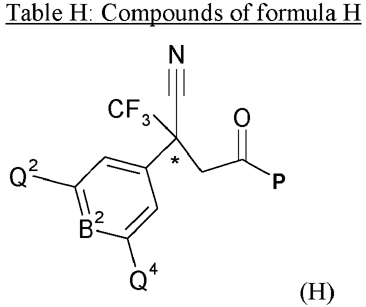

Table H discloses 630 compounds of formula H, wherein Q², B², Q⁴ and P have the values as defined in Table X.

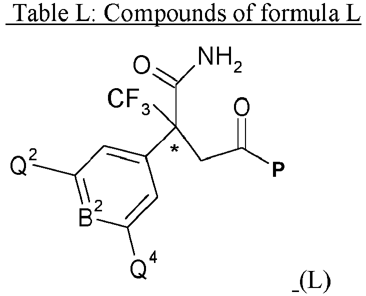

Table L discloses 630 compounds of formula L, wherein Q², B², Q⁴ and P have the values as defined in Table X.

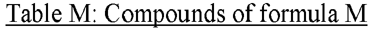

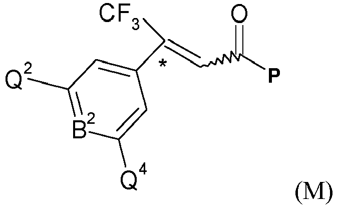

Table M discloses 630 compounds of formula M, wherein Q², B², Q⁴ and P have the values as defined in Table X.

**Table X**

| | Q2 | B2 | Q4 | P |
|---|---|---|---|---|
| X.1 | Cl | C-Cl | Cl | P1 |
| X.2 | Cl | C-H | Cl | P1 |
| X.3 | CF₃ | C-H | CF₃ | P1 |
| X.4 | Cl | C-H | CF₃ | P1 |
| X.5 | Br | C-H | CF₃ | P1 |
| X.6 | Cl | C-F | H | P1 |
| X.7 | F | C-Cl | H | P1 |
| X.8 | Cl | C-Cl | H | P1 |
| X.9 | Cl | C-F | Cl | P1 |
| X.10 | Cl | C-Br | Cl | P1 |
| X.11 | Cl | C-I | Cl | P1 |
| X.12 | F | C-F | F | P1 |
| X.13 | Cl | C-H | Br | P1 |
| X.14 | Cl | C-H | F | P1 |
| X.15 | Cl | C-Cl | CF3 | P1 |
| X.16 | CF3 | C-Cl | CF3 | P1 |
| X.17 | CF3 | C-H | H | P1 |
| X.18 | Cl | C-Cl | Cl | P2 |
| X.19 | Cl | C-H | Cl | P2 |
| X.20 | CF₃ | C-H | CF₃ | P2 |
| X.21 | Cl | C-H | CF₃ | P2 |
| X.22 | Br | C-H | CF₃ | P2 |
| X.23 | Cl | C-F | H | P2 |
| X.24 | F | C-Cl | H | P2 |
| X.25 | Cl | C-Cl | H | P2 |
| X.26 | Cl | C-F | Cl | P2 |
| X.27 | Cl | C-Br | Cl | P2 |
| X.28 | Cl | C-I | Cl | P2 |
| X.29 | F | C-F | F | P2 |
| X.30 | Cl | C-H | Br | P2 |
| X.31 | Cl | C-H | F | P2 |
| X.32 | Cl | C-Cl | CF3 | P2 |
| X.33 | CF3 | C-Cl | CF3 | P2 |
| X.34 | CF3 | C-H | H | P2 |
| X.35 | Cl | C-Cl | Cl | P3 |
| X.36 | Cl | C-H | Cl | P3 |
| X.37 | CF₃ | C-H | CF₃ | P3 |
| X.38 | Cl | C-H | CF₃ | P3 |
| X.39 | Br | C-H | CF₃ | P3 |
| X.40 | Cl | C-F | H | P3 |
| X.41 | F | C-Cl | H | P3 |
| X.42 | Cl | C-Cl | H | P3 |
| X.43 | Cl | C-F | Cl | P3 |
| X.44 | Cl | C-Br | Cl | P3 |
| X.45 | Cl | C-I | Cl | P3 |
| X.46 | F | C-F | F | P3 |
| X.47 | Cl | C-H | Br | P3 |
| X.48 | Cl | C-H | F | P3 |
| X.49 | Cl | C-Cl | CF3 | P3 |
| X.50 | CF3 | C-Cl | CF3 | P3 |
| X.51 | CF3 | C-H | H | P3 |
| X.52 | Cl | C-Cl | Cl | P4 |
| X.53 | Cl | C-H | Cl | P4 |
| X.54 | CF₃ | C-H | CF₃ | P4 |
| X.55 | Cl | C-H | CF₃ | P4 |
| X.56 | Br | C-H | CF₃ | P4 |
| X.57 | Cl | C-F | H | P4 |
| X.58 | F | C-Cl | H | P4 |
| X.59 | Cl | C-Cl | H | P4 |
| X.60 | Cl | C-F | Cl | P4 |
| X.61 | Cl | C-Br | Cl | P4 |
| X.62 | Cl | C-I | Cl | P4 |
| X.63 | F | C-F | F | P4 |
| X.64 | Cl | C-H | Br | P4 |
| X.65 | Cl | C-H | F | P4 |
| X.66 | Cl | C-Cl | CF3 | P4 |
| X.67 | CF3 | C-Cl | CF3 | P4 |
| X.68 | CF3 | C-H | H | P4 |
| X.69 | Cl | C-Cl | Cl | P5 |
| X.70 | Cl | C-H | Cl | P5 |
| X.71 | CF₃ | C-H | CF₃ | P5 |
| X.72 | Cl | C-H | CF₃ | P5 |
| X.73 | Br | C-H | CF₃ | P5 |
| X.74 | Cl | C-F | H | P5 |
| X.75 | F | C-Cl | H | P5 |
| X.76 | Cl | C-Cl | H | P5 |
| X.77 | Cl | C-F | Cl | P5 |
| X.78 | Cl | C-Br | Cl | P5 |
| X.79 | Cl | C-I | Cl | P5 |
| X.80 | F | C-F | F | P5 |
| X.81 | Cl | C-H | Br | P5 |
| X.82 | Cl | C-H | F | P5 |
| X.83 | Cl | C-Cl | CF3 | P5 |
| X.84 | CF3 | C-Cl | CF3 | P5 |
| X.85 | CF3 | C-H | H | P5 |
| X.86 | Cl | C-Cl | Cl | P6 |
| X.87 | Cl | C-H | Cl | P6 |
| X.88 | CF₃ | C-H | CF₃ | P6 |
| X.89 | Cl | C-H | CF₃ | P6 |
| X.90 | Br | C-H | CF₃ | P6 |
| X.91 | Cl | C-F | H | P6 |
| X.92 | F | C-Cl | H | P6 |
| X.93 | Cl | C-Cl | H | P6 |
| X.94 | Cl | C-F | Cl | P6 |
| X.95 | Cl | C-Br | Cl | P6 |
| X.96 | Cl | C-I | Cl | P6 |
| X.97 | F | C-F | F | P6 |
| X.98 | Cl | C-H | Br | P6 |
| X.99 | Cl | C-H | F | P6 |
| X.100 | Cl | C-Cl | CF3 | P6 |
| X.101 | CF3 | C-Cl | CF3 | P6 |
| X.102 | CF3 | C-H | H | P6 |
| X.103 | Cl | C-Cl | Cl | P7 |
| X.104 | Cl | C-H | Cl | P7 |
| X.105 | CF₃ | C-H | CF₃ | P7 |
| X.106 | Cl | C-H | CF₃ | P7 |
| X.107 | Br | C-H | CF₃ | P7 |
| X.108 | Cl | C-F | H | P7 |
| X.109 | F | C-Cl | H | P7 |
| X.110 | Cl | C-Cl | H | P7 |
| X.111 | Cl | C-F | Cl | P7 |
| X. 112 | Cl | C-Br | Cl | P7 |
| X.113 | Cl | C-I | Cl | P7 |
| X. 114 | F | C-F | F | P7 |
| X.115 | Cl | C-H | Br | P7 |
| X.116 | Cl | C-H | F | P7 |
| X. 117 | Cl | C-Cl | CF3 | P7 |
| X.118 | CF3 | C-Cl | CF3 | P7 |
| X.119 | CF3 | C-H | H | P7 |
| X.120 | Cl | C-Cl | Cl | P8 |
| X.121 | Cl | C-H | Cl | P8 |
| X.122 | CF₃ | C-H | CF₃ | P8 |
| X.123 | Cl | C-H | CF₃ | P8 |
| X.124 | Br | C-H | CF₃ | P8 |
| X.125 | Cl | C-F | H | P8 |
| X.126 | F | C-Cl | H | P8 |
| X.127 | Cl | C-Cl | H | P8 |
| X.128 | Cl | C-F | Cl | P8 |
| X.129 | Cl | C-Br | Cl | P8 |
| X.130 | Cl | C-I | Cl | P8 |
| X.131 | F | C-F | F | P8 |
| X.132 | Cl | C-H | Br | P8 |
| X.133 | Cl | C-H | F | P8 |
| X.134 | Cl | C-Cl | CF3 | P8 |
| X.135 | CF3 | C-Cl | CF3 | P8 |
| X.136 | CF3 | C-H | H | P8 |
| X.137 | Cl | C-Cl | Cl | P9 |
| X.138 | Cl | C-H | Cl | P9 |
| X.139 | CF₃ | C-H | CF₃ | P9 |
| X.140 | Cl | C-H | CF₃ | P9 |
| X.141 | Br | C-H | CF₃ | P9 |
| X.142 | Cl | C-F | H | P9 |
| X.143 | F | C-Cl | H | P9 |
| X.144 | Cl | C-Cl | H | P9 |
| X.145 | Cl | C-F | Cl | P9 |
| X.146 | Cl | C-Br | Cl | P9 |
| X.147 | Cl | C-I | Cl | P9 |
| X.148 | F | C-F | F | P9 |
| X.149 | Cl | C-H | Br | P9 |
| X.150 | Cl | C-H | F | P9 |
| X.151 | Cl | C-Cl | CF3 | P9 |
| X.152 | CF3 | C-Cl | CF3 | P9 |
| X.153 | CF3 | C-H | H | P9 |
| X.154 | Cl | C-Cl | Cl | P10 |
| X.155 | Cl | C-H | Cl | P10 |
| X.156 | CF₃ | C-H | CF₃ | P10 |
| X.157 | Cl | C-H | CF₃ | P10 |
| X.158 | Br | C-H | CF₃ | P10 |
| X.159 | Cl | C-F | H | P10 |
| X.160 | F | C-Cl | H | P10 |
| X.161 | Cl | C-Cl | H | P10 |
| X.162 | Cl | C-F | Cl | P10 |
| X.163 | Cl | C-Br | Cl | P10 |
| X.164 | Cl | C-I | Cl | P10 |
| X.165 | F | C-F | F | P10 |
| X.166 | Cl | C-H | Br | P10 |
| X.167 | Cl | C-H | F | P10 |
| X.168 | Cl | C-Cl | CF3 | P10 |
| X.169 | CF3 | C-Cl | CF3 | P10 |
| X.170 | CF3 | C-H | H | P10 |
| X.171 | Cl | C-Cl | Cl | P11 |
| X.172 | Cl | C-H | Cl | P11 |
| X.173 | CF₃ | C-H | CF₃ | P11 |
| X.174 | Cl | C-H | CF₃ | P11 |
| X.175 | Br | C-H | CF₃ | P11 |
| X.176 | Cl | C-F | H | P11 |
| X.177 | F | C-Cl | H | P11 |
| X.178 | Cl | C-Cl | H | P11 |
| X.179 | Cl | C-F | Cl | P11 |
| X.180 | Cl | C-Br | Cl | P11 |
| X.181 | Cl | C-I | Cl | P11 |
| X.182 | F | C-F | F | P11 |
| X.183 | Cl | C-H | Br | P11 |
| X.184 | Cl | C-H | F | P11 |
| X.185 | Cl | C-Cl | CF3 | P11 |
| X.186 | CF3 | C-Cl | CF3 | P11 |
| X.187 | CF3 | C-H | H | P11 |
| X.188 | Cl | C-Cl | Cl | P12 |
| X.187 | Cl | C-H | Cl | P12 |
| X.190 | CF₃ | C-H | CF₃ | P12 |
| X.191 | Cl | C-H | CF₃ | P12 |
| X.192 | Br | C-H | CF₃ | P12 |
| X.193 | Cl | C-F | H | P12 |
| X.194 | F | C-Cl | H | P12 |
| X.195 | Cl | C-Cl | H | P12 |
| X.196 | Cl | C-F | Cl | P12 |
| X.197 | Cl | C-Br | Cl | P12 |
| X.198 | Cl | C-I | Cl | P12 |
| X.199 | F | C-F | F | P12 |
| X.200 | Cl | C-H | Br | P12 |
| X.201 | Cl | C-H | F | P12 |
| X.202 | Cl | C-Cl | CF3 | P12 |
| X.203 | CF3 | C-Cl | CF3 | P12 |
| X.204 | CF3 | C-H | H | P12 |
| X.205 | Cl | C-Cl | Cl | P13 |
| X.206 | Cl | C-H | Cl | P13 |
| X.207 | CF₃ | C-H | CF₃ | P13 |
| X.208 | Cl | C-H | CF₃ | P13 |
| X.209 | Br | C-H | CF₃ | P13 |
| X.210 | Cl | C-F | H | P13 |
| X.211 | F | C-Cl | H | P13 |
| X.212 | Cl | C-Cl | H | P13 |
| X.213 | Cl | C-F | Cl | P13 |
| X.214 | Cl | C-Br | Cl | P13 |
| X.215 | Cl | C-I | Cl | P13 |
| X.216 | F | C-F | F | P13 |
| X.217 | Cl | C-H | Br | P13 |
| X.218 | Cl | C-H | F | P13 |
| X.219 | Cl | C-Cl | CF3 | P13 |
| X.220 | CF3 | C-Cl | CF3 | P13 |
| X.221 | CF3 | C-H | H | P13 |
| X.222 | Cl | C-Cl | Cl | P14 |
| X.223 | Cl | C-H | Cl | P14 |
| X.224 | CF₃ | C-H | CF₃ | P14 |
| X.225 | Cl | C-H | CF₃ | P14 |
| X.226 | Br | C-H | CF₃ | P14 |
| X.227 | Cl | C-F | H | P14 |
| X.228 | F | C-Cl | H | P14 |
| X.229 | Cl | C-Cl | H | P14 |
| X.230 | Cl | C-F | Cl | P14 |
| X.231 | Cl | C-Br | Cl | P14 |
| X.232 | Cl | C-I | Cl | P14 |
| X.233 | F | C-F | F | P14 |
| X.234 | Cl | C-H | Br | P14 |
| X.235 | Cl | C-H | F | P14 |
| X.236 | Cl | C-Cl | CF3 | P14 |
| X.237 | CF3 | C-Cl | CF3 | P14 |
| X.238 | CF3 | C-H | H | P14 |
| X.239 | Cl | C-Cl | Cl | P15 |
| X.240 | Cl | C-H | Cl | P15 |
| X.241 | CF₃ | C-H | CF₃ | P15 |
| X.242 | Cl | C-H | CF₃ | P15 |
| X.243 | Br | C-H | CF₃ | P15 |
| X.244 | Cl | C-F | H | P15 |
| X.245 | F | C-Cl | H | P15 |
| X.246 | Cl | C-Cl | H | P15 |
| X.247 | Cl | C-F | Cl | P15 |
| X.248 | Cl | C-Br | Cl | P15 |
| X.249 | Cl | C-I | Cl | P15 |
| X.250 | F | C-F | F | P15 |
| X.251 | Cl | C-H | Br | P15 |
| X.252 | Cl | C-H | F | P15 |
| X.253 | Cl | C-Cl | CF3 | P15 |
| X.254 | CF3 | C-Cl | CF3 | P15 |
| X.255 | CF3 | C-H | H | P15 |
| X.256 | Cl | C-Cl | Cl | P16 |
| X.257 | Cl | C-H | Cl | P16 |
| X.258 | CF₃ | C-H | CF₃ | P16 |
| X.259 | Cl | C-H | CF₃ | P16 |
| X.260 | Br | C-H | CF₃ | P16 |
| X.261 | Cl | C-F | H | P16 |
| X.262 | F | C-Cl | H | P16 |
| X.263 | Cl | C-Cl | H | P16 |
| X.264 | Cl | C-F | Cl | P16 |
| X.265 | Cl | C-Br | Cl | P16 |
| X.266 | Cl | C-I | Cl | P16 |
| X.267 | F | C-F | F | P16 |
| X.268 | Cl | C-H | Br | P16 |
| X.269 | Cl | C-H | F | P16 |
| X.270 | Cl | C-Cl | CF3 | P16 |
| X.271 | CF3 | C-Cl | CF3 | P16 |
| X.272 | CF3 | C-H | H | P16 |
| X.273 | Cl | C-Cl | Cl | P17 |
| X.274 | Cl | C-H | Cl | P17 |
| X.275 | CF₃ | C-H | CF₃ | P17 |
| X.276 | Cl | C-H | CF₃ | P17 |
| X.277 | Br | C-H | CF₃ | P17 |
| X.278 | Cl | C-F | H | P17 |
| X.279 | F | C-Cl | H | P17 |
| X.280 | Cl | C-Cl | H | P17 |
| X.281 | Cl | C-F | Cl | P17 |
| X.282 | Cl | C-Br | Cl | P17 |
| X.283 | Cl | C-I | Cl | P17 |
| X.284 | F | C-F | F | P17 |
| X.285 | Cl | C-H | Br | P17 |
| X.286 | Cl | C-H | F | P17 |
| X.287 | Cl | C-Cl | CF3 | P17 |
| X.288 | CF3 | C-Cl | CF3 | P17 |
| X.289 | CF3 | C-H | H | P17 |
| X.290 | Cl | C-Cl | Cl | P18 |
| X.291 | Cl | C-H | Cl | P18 |
| X.292 | CF₃ | C-H | CF₃ | P18 |
| X.293 | Cl | C-H | CF₃ | P18 |
| X.294 | Br | C-H | CF₃ | P18 |
| X.295 | Cl | C-F | H | P18 |
| X.296 | F | C-Cl | H | P18 |
| X.297 | Cl | C-Cl | H | P18 |
| X.298 | Cl | C-F | Cl | P18 |
| X.299 | Cl | C-Br | Cl | P18 |
| X.300 | Cl | C-I | Cl | P18 |
| X.301 | F | C-F | F | P18 |
| X.302 | Cl | C-H | Br | P18 |
| X.303 | Cl | C-H | F | P18 |
| X.304 | Cl | C-Cl | CF3 | P18 |
| X.305 | CF3 | C-Cl | CF3 | P18 |
| X.306 | CF3 | C-H | H | P18 |
| X.307 | Cl | C-Cl | Cl | P19 |
| X.308 | Cl | C-H | Cl | P19 |
| X.309 | CF₃ | C-H | CF₃ | P19 |
| X.310 | Cl | C-H | CF₃ | P19 |
| X.311 | Br | C-H | CF₃ | P19 |
| X.312 | Cl | C-F | H | P19 |
| X.313 | F | C-Cl | H | P19 |
| X.314 | Cl | C-Cl | H | P19 |
| X.315 | Cl | C-F | Cl | P19 |
| X.316 | Cl | C-Br | Cl | P19 |
| X.317 | Cl | C-I | Cl | P19 |
| X.318 | F | C-F | F | P19 |
| X.319 | Cl | C-H | Br | P19 |
| X.320 | Cl | C-H | F | P19 |
| X.321 | Cl | C-Cl | CF3 | P19 |
| X.322 | CF3 | C-Cl | CF3 | P19 |
| X.323 | CF3 | C-H | H | P19 |
| X.324 | Cl | C-Cl | Cl | P20 |
| X.325 | Cl | C-H | Cl | P20 |
| X.326 | CF₃ | C-H | CF₃ | P20 |
| X.327 | Cl | C-H | CF₃ | P20 |
| X.328 | Br | C-H | CF₃ | P20 |
| X.329 | Cl | C-F | H | P20 |
| X.330 | F | C-Cl | H | P20 |
| X.331 | Cl | C-Cl | H | P20 |
| X.332 | Cl | C-F | Cl | P20 |
| X.333 | Cl | C-Br | Cl | P20 |
| X.334 | Cl | C-I | Cl | P20 |
| X.335 | F | C-F | F | P20 |
| X.336 | Cl | C-H | Br | P20 |
| X.337 | Cl | C-H | F | P20 |
| X.338 | Cl | C-Cl | CF3 | P20 |
| X.339 | CF3 | C-Cl | CF3 | P20 |
| X.340 | CF3 | C-H | H | P20 |
| X.341 | Cl | C-Cl | Cl | P21 |
| X.342 | Cl | C-H | Cl | P21 |
| X.343 | CF₃ | C-H | CF₃ | P21 |
| X.344 | Cl | C-H | CF₃ | P21 |
| X.345 | Br | C-H | CF₃ | P21 |
| X.346 | Cl | C-F | H | P21 |
| X.347 | F | C-Cl | H | P21 |
| X.348 | Cl | C-Cl | H | P21 |
| X.349 | Cl | C-F | Cl | P21 |
| X.350 | Cl | C-Br | Cl | P21 |
| X.351 | Cl | C-I | Cl | P21 |
| X.352 | F | C-F | F | P21 |
| X.353 | Cl | C-H | Br | P21 |
| X.354 | Cl | C-H | F | P21 |
| X.355 | Cl | C-Cl | CF3 | P21 |
| X.356 | CF3 | C-Cl | CF3 | P21 |
| X.357 | CF3 | C-H | H | P21 |
| X.358 | Cl | C-Cl | Cl | P22 |
| X.359 | Cl | C-H | Cl | P22 |
| X.360 | CF₃ | C-H | CF₃ | P22 |
| X.361 | Cl | C-H | CF₃ | P22 |
| X.362 | Br | C-H | CF₃ | P22 |
| X.363 | Cl | C-F | H | P22 |
| X.364 | F | C-Cl | H | P22 |
| X.365 | Cl | C-Cl | H | P22 |
| X.366 | Cl | C-F | Cl | P22 |
| X.367 | Cl | C-Br | Cl | P22 |
| X.368 | Cl | C-I | Cl | P22 |
| X.369 | F | C-F | F | P22 |
| X.370 | Cl | C-H | Br | P22 |
| X.371 | Cl | C-H | F | P22 |
| X.372 | Cl | C-Cl | CF3 | P22 |
| X.373 | CF3 | C-Cl | CF3 | P22 |
| X.374 | CF3 | C-H | H | P22 |
| X.375 | Cl | C-Cl | Cl | P23 |
| X.376 | Cl | C-H | Cl | P23 |
| X.377 | CF₃ | C-H | CF₃ | P23 |
| X.378 | Cl | C-H | CF₃ | P23 |
| X.379 | Br | C-H | CF₃ | P23 |
| X.380 | Cl | C-F | H | P23 |
| X.381 | F | C-Cl | H | P23 |
| X.382 | Cl | C-Cl | H | P23 |
| X.383 | Cl | C-F | Cl | P23 |
| X.384 | Cl | C-Br | Cl | P23 |
| X.385 | Cl | C-I | Cl | P23 |
| X.386 | F | C-F | F | P23 |
| X.387 | Cl | C-H | Br | P23 |
| X.388 | Cl | C-H | F | P23 |
| X.389 | Cl | C-Cl | CF3 | P23 |
| X.390 | CF3 | C-Cl | CF3 | P23 |
| X.391 | CF3 | C-H | H | P23 |
| X.392 | Cl | C-Cl | Cl | P24 |
| X.393 | Cl | C-H | Cl | P24 |
| X.394 | CF₃ | C-H | CF₃ | P24 |
| X.395 | Cl | C-H | CF₃ | P24 |
| X.396 | Br | C-H | CF₃ | P24 |
| X.397 | Cl | C-F | H | P24 |
| X.398 | F | C-Cl | H | P24 |
| X.399 | Cl | C-Cl | H | P24 |
| X.400 | Cl | C-F | Cl | P24 |
| X.401 | Cl | C-Br | Cl | P24 |
| X.402 | Cl | C-I | Cl | P24 |
| X.403 | F | C-F | F | P24 |
| X.404 | Cl | C-H | Br | P24 |
| X.405 | Cl | C-H | F | P24 |
| X.406 | Cl | C-Cl | CF3 | P24 |
| X.407 | CF3 | C-Cl | CF3 | P24 |
| X.408 | CF3 | C-H | H | P24 |
| X.409 | Cl | C-Cl | Cl | P25 |
| X.410 | Cl | C-H | Cl | P25 |
| X.411 | CF₃ | C-H | CF₃ | P25 |
| X.412 | Cl | C-H | CF₃ | P25 |
| X.413 | Br | C-H | CF₃ | P25 |
| X.414 | Cl | C-F | H | P25 |
| X.415 | F | C-Cl | H | P25 |
| X.416 | Cl | C-Cl | H | P25 |
| X.417 | Cl | C-F | Cl | P25 |
| X.418 | Cl | C-Br | Cl | P25 |
| X.419 | Cl | C-I | Cl | P25 |
| X.420 | F | C-F | F | P25 |
| X.421 | Cl | C-H | Br | P25 |
| X.422 | Cl | C-H | F | P25 |
| X.423 | Cl | C-Cl | CF3 | P25 |
| X.424 | CF3 | C-Cl | CF3 | P25 |
| X.425 | CF3 | C-H | H | P25 |
| X.426 | Cl | C-Cl | Cl | P26 |
| X.427 | Cl | C-H | Cl | P26 |
| X.428 | CF₃ | C-H | CF₃ | P26 |
| X.429 | Cl | C-H | CF₃ | P26 |
| X.430 | Br | C-H | CF₃ | P26 |
| X.431 | Cl | C-F | H | P26 |
| X.432 | F | C-Cl | H | P26 |
| X.433 | Cl | C-Cl | H | P26 |
| X.434 | Cl | C-F | Cl | P26 |
| X.435 | Cl | C-Br | Cl | P26 |
| X.436 | Cl | C-I | Cl | P26 |
| X.437 | F | C-F | F | P26 |
| X.438 | Cl | C-H | Br | P26 |
| X.439 | Cl | C-H | F | P26 |
| X.440 | Cl | C-Cl | CF3 | P26 |
| X.441 | CF3 | C-Cl | CF3 | P26 |
| X.442 | CF3 | C-H | H | P26 |
| X.443 | Cl | C-Cl | Cl | P27 |
| X.444 | Cl | C-H | Cl | P27 |
| X.445 | CF₃ | C-H | CF₃ | P27 |
| X.446 | Cl | C-H | CF₃ | P27 |
| X.447 | Br | C-H | CF₃ | P27 |
| X.448 | Cl | C-F | H | P27 |
| X.449 | F | C-Cl | H | P27 |
| X.450 | Cl | C-Cl | H | P27 |
| X.451 | Cl | C-F | Cl | P27 |
| X.452 | Cl | C-Br | Cl | P27 |
| X.453 | Cl | C-I | Cl | P27 |
| X.454 | F | C-F | F | P27 |
| X.455 | Cl | C-H | Br | P27 |
| X.456 | Cl | C-H | F | P27 |
| X.457 | Cl | C-Cl | CF3 | P27 |
| X.458 | CF3 | C-Cl | CF3 | P27 |
| X.459 | CF3 | C-H | H | P27 |
| X.460 | Cl | C-Cl | Cl | P28 |
| X.461 | Cl | C-H | Cl | P28 |
| X.462 | CF₃ | C-H | CF₃ | P28 |
| X.463 | Cl | C-H | CF₃ | P28 |
| X.464 | Br | C-H | CF₃ | P28 |
| X.465 | Cl | C-F | H | P28 |
| X.466 | F | C-Cl | H | P28 |
| X.467 | Cl | C-Cl | H | P28 |
| X.468 | Cl | C-F | Cl | P28 |
| X.469 | Cl | C-Br | Cl | P28 |
| X.470 | Cl | C-I | Cl | P28 |
| X.471 | F | C-F | F | P28 |
| X.472 | Cl | C-H | Br | P28 |
| X.473 | Cl | C-H | F | P28 |
| X.474 | Cl | C-Cl | CF3 | P28 |
| X.475 | CF3 | C-Cl | CF3 | P28 |
| X.476 | CF3 | C-H | H | P28 |
| X.477 | Cl | C-Cl | Cl | P29 |
| X.478 | Cl | C-H | Cl | P29 |
| X.479 | CF₃ | C-H | CF₃ | P29 |
| X.480 | Cl | C-H | CF₃ | P29 |
| X.481 | Br | C-H | CF₃ | P29 |
| X.482 | Cl | C-F | H | P29 |
| X.483 | F | C-Cl | H | P29 |
| X.484 | Cl | C-Cl | H | P29 |
| X.485 | Cl | C-F | Cl | P29 |
| X.486 | Cl | C-Br | Cl | P29 |
| X.487 | Cl | C-I | Cl | P29 |
| X.488 | F | C-F | F | P29 |
| X.489 | Cl | C-H | Br | P29 |
| X.490 | Cl | C-H | F | P29 |
| X.491 | Cl | C-Cl | CF3 | P29 |
| X.492 | CF3 | C-Cl | CF3 | P29 |
| X.493 | CF3 | C-H | H | P29 |
| X.494 | Cl | C-Cl | Cl | P30 |
| X.495 | Cl | C-H | Cl | P30 |
| X.496 | CF₃ | C-H | CF₃ | P30 |
| X.497 | Cl | C-H | CF₃ | P30 |
| X.498 | Br | C-H | CF₃ | P30 |
| X.499 | Cl | C-F | H | P30 |
| X.500 | F | C-Cl | H | P30 |
| X.501 | Cl | C-Cl | H | P30 |
| X.502 | Cl | C-F | Cl | P30 |
| X.503 | Cl | C-Br | Cl | P30 |
| X.504 | Cl | C-I | Cl | P30 |
| X.505 | F | C-F | F | P30 |
| X.506 | Cl | C-H | Br | P30 |
| X.507 | Cl | C-H | F | P30 |
| X.508 | Cl | C-Cl | CF3 | P30 |
| X.509 | CF3 | C-Cl | CF3 | P30 |
| X.510 | CF3 | C-H | H | P30 |
| X.511 | Cl | N | Cl | P1 |
| X.512 | Cl | N | H | P1 |
| X.513 | CF₃ | N | CF₃ | P1 |
| X.514 | CF₃ | N | H | P1 |
| X.515 | Cl | N | Cl | P2 |
| X.516 | Cl | N | H | P2 |
| X.517 | CF3 | N | CF3 | P2 |
| X.518 | CF3 | N | H | P2 |
| X.519 | Cl | N | Cl | P3 |
| X.520 | Cl | N | H | P3 |
| X.521 | CF3 | N | CF3 | P3 |
| X.522 | CF3 | N | H | P3 |
| X.523 | Cl | N | Cl | P4 |
| X.524 | Cl | N | H | P4 |
| X.525 | CF3 | N | CF3 | P4 |
| X.526 | CF3 | N | H | P4 |
| X.527 | Cl | N | Cl | P5 |
| X.528 | Cl | N | H | P5 |
| X.529 | CF3 | N | CF3 | P5 |
| X.530 | CF3 | N | H | P5 |
| X.531 | Cl | N | Cl | P6 |
| X.532 | Cl | N | H | P6 |
| X.533 | CF3 | N | CF3 | P6 |
| X.534 | CF3 | N | H | P6 |
| X.535 | Cl | N | Cl | P7 |
| X.536 | Cl | N | H | P7 |
| X.537 | CF3 | N | CF3 | P7 |
| X.538 | CF3 | N | H | P7 |
| X.539 | Cl | N | Cl | P8 |
| X.540 | Cl | N | H | P8 |
| X.541 | CF3 | N | CF3 | P8 |
| X.542 | CF3 | N | H | P8 |
| X.543 | Cl | N | Cl | P9 |
| X.544 | Cl | N | H | P9 |
| X.545 | CF3 | N | CF3 | P9 |
| X.546 | CF3 | N | H | P9 |
| X.547 | Cl | N | Cl | P10 |
| X.548 | Cl | N | H | P10 |
| X.549 | CF3 | N | CF3 | P10 |
| X.550 | CF3 | N | H | P10 |
| X.551 | Cl | N | Cl | P11 |
| X.552 | Cl | N | H | P11 |
| X.553 | CF3 | N | CF3 | P11 |
| X.554 | CF3 | N | H | P11 |
| X.555 | Cl | N | Cl | P12 |
| X.556 | Cl | N | H | P12 |
| X.557 | CF3 | N | CF3 | P12 |
| X.558 | CF3 | N | H | P12 |
| X.559 | Cl | N | Cl | P13 |
| X.560 | Cl | N | H | P13 |
| X.561 | CF3 | N | CF3 | P13 |
| X.562 | CF3 | N | H | P13 |
| X.563 | Cl | N | Cl | P14 |
| X.564 | Cl | N | H | P14 |
| X.565 | CF3 | N | CF3 | P14 |
| X.566 | CF3 | N | H | P14 |
| X.567 | Cl | N | Cl | P15 |
| X.568 | Cl | N | H | P15 |
| X.569 | CF3 | N | CF3 | P15 |
| X.570 | CF3 | N | H | P15 |
| X.571 | Cl | N | Cl | P16 |
| X.572 | Cl | N | H | P16 |
| X.573 | CF3 | N | CF3 | P16 |
| X.574 | CF3 | N | H | P16 |
| X.575 | Cl | N | Cl | P17 |
| X.576 | Cl | N | H | P17 |
| X.577 | CF3 | N | CF3 | P17 |
| X.578 | CF3 | N | H | P17 |
| X.579 | Cl | N | Cl | P18 |
| X.580 | Cl | N | H | P18 |
| X.581 | CF3 | N | CF3 | P18 |
| X.582 | CF3 | N | H | P18 |
| X.583 | Cl | N | Cl | P19 |
| X.584 | Cl | N | H | P19 |
| X.585 | CF3 | N | CF3 | P19 |
| X.586 | CF3 | N | H | P19 |
| X.587 | Cl | N | Cl | P20 |
| X.588 | Cl | N | H | P20 |
| X.589 | CF3 | N | CF3 | P20 |
| X.590 | CF3 | N | H | P20 |
| X.591 | Cl | N | Cl | P21 |
| X.592 | Cl | N | H | P21 |
| X.593 | CF3 | N | CF3 | P21 |
| X.594 | CF3 | N | H | P21 |
| X.595 | Cl | N | Cl | P22 |
| X.596 | Cl | N | H | P22 |
| X.597 | CF3 | N | CF3 | P22 |
| X.598 | CF3 | N | H | P22 |
| X.599 | Cl | N | Cl | P23 |
| X.600 | Cl | N | H | P23 |
| X.601 | CF3 | N | CF3 | P23 |
| X.602 | CF3 | N | H | P23 |
| X.603 | Cl | N | Cl | P24 |
| X.604 | Cl | N | H | P24 |
| X.605 | CF3 | N | CF3 | P24 |
| X.606 | CF3 | N | H | P24 |
| X.607 | Cl | N | Cl | P25 |
| X.608 | Cl | N | H | P25 |
| X.609 | CF3 | N | CF3 | P25 |
| X.610 | CF3 | N | H | P25 |
| X.611 | Cl | N | Cl | P26 |
| X.612 | Cl | N | H | P26 |
| X.613 | CF3 | N | CF3 | P26 |
| X.614 | CF3 | N | H | P26 |
| X.615 | Cl | N | Cl | P27 |
| X.616 | Cl | N | H | P27 |
| X.617 | CF3 | N | CF3 | P27 |
| X.618 | CF3 | N | H | P27 |
| X.619 | Cl | N | Cl | P28 |
| X.620 | Cl | N | H | P28 |
| X.621 | CF3 | N | CF3 | P28 |
| X.622 | CF3 | N | H | P28 |
| X.623 | C1 | N | C1 | P29 |
| X.624 | C1 | N | H | P29 |
| X.625 | CF3 | N | CF3 | P29 |
| X.626 | CF3 | N | H | P29 |
| X.627 | Cl | N | Cl | P30 |
| X.628 | C1 | N | H | P30 |
| X.629 | CF3 | N | CF3 | P30 |
| X.630 | CF3 | N | H | P30 |

The values of P1 to P30 in Table X are shown in Table P.

**Table P**

| | |
|---|---|
| P1 | -OCH₃ |
| P2 | -OCH₂CH₃ |
| P3 | -OtBu |
| P4 | -NMe₂ |
| P5 | 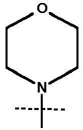 |
| P6 | 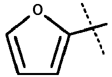 |
| P7 | 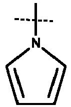 |
| P8 | 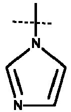 |
| P9 | 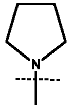 |
| P10 | OPh |
| P11 | Ph |
| P12 | 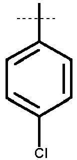 |
| P13 | 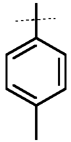 |
| P14 | 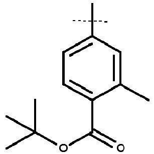 |
| P15 | tBu |
| P16 | 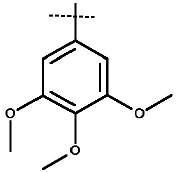 |
| P17 | 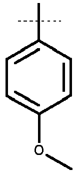 |
| P18 | 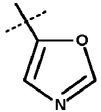 |
| P19 | 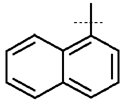 |
| P20 | 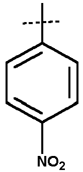 |
| P21 | 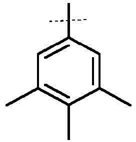 |
| P22 | 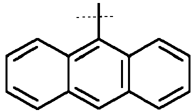 |
| P23 | 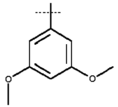 |
| P24 | 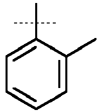 |
| P25 | -CH(CH₃)₂ |
| P26 | 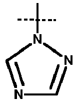 |
| P27 | 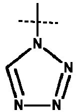 |
| P28 | 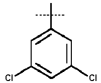 |
| P29 | OCH₂Ph |
| P30 | OH |

### Preparation Examples

The following abbreviations were used in this section: s = singlet; bs = broad singlet; d = doublet; dd = double doublet; dt = double triplet; t = triplet, tt = triple triplet, q = quartet, sept = septet; m = multiplet; Me = methyl; Et = ethyl; Pr = propyl; Bu = butyl; M.p. = melting point; RT = retention time, [M+H]⁺ = molecular mass of the molecular cation, [M-H]⁻ = molecular mass of the molecular anion.

The following LC-MS methods were used to characterize the compounds:

### Method C

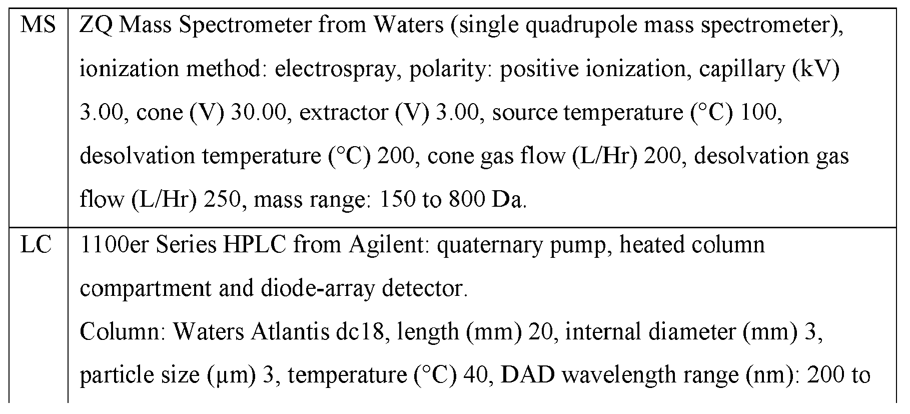

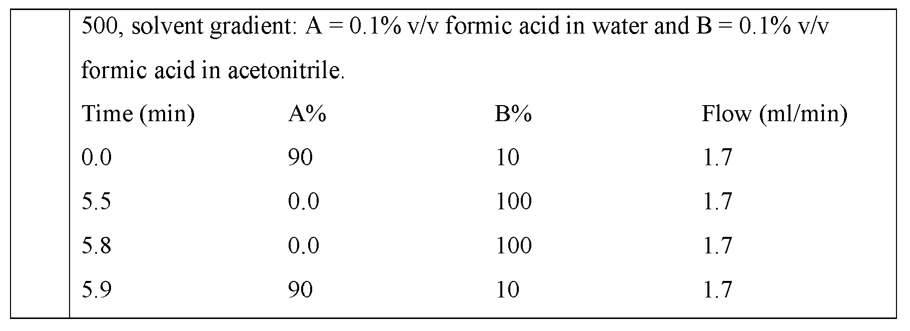

### Method D

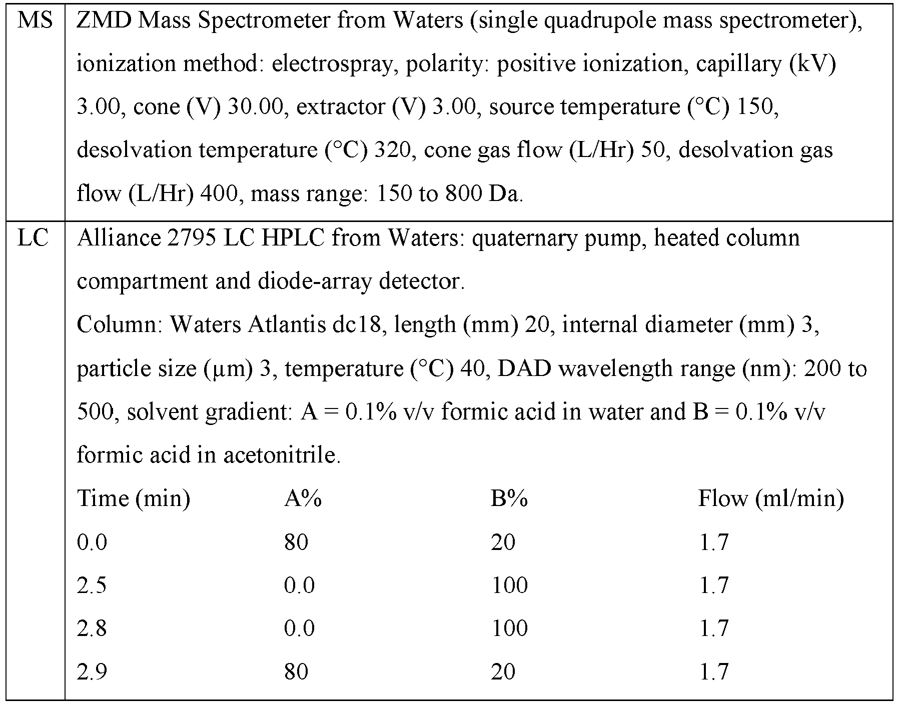

### Method F

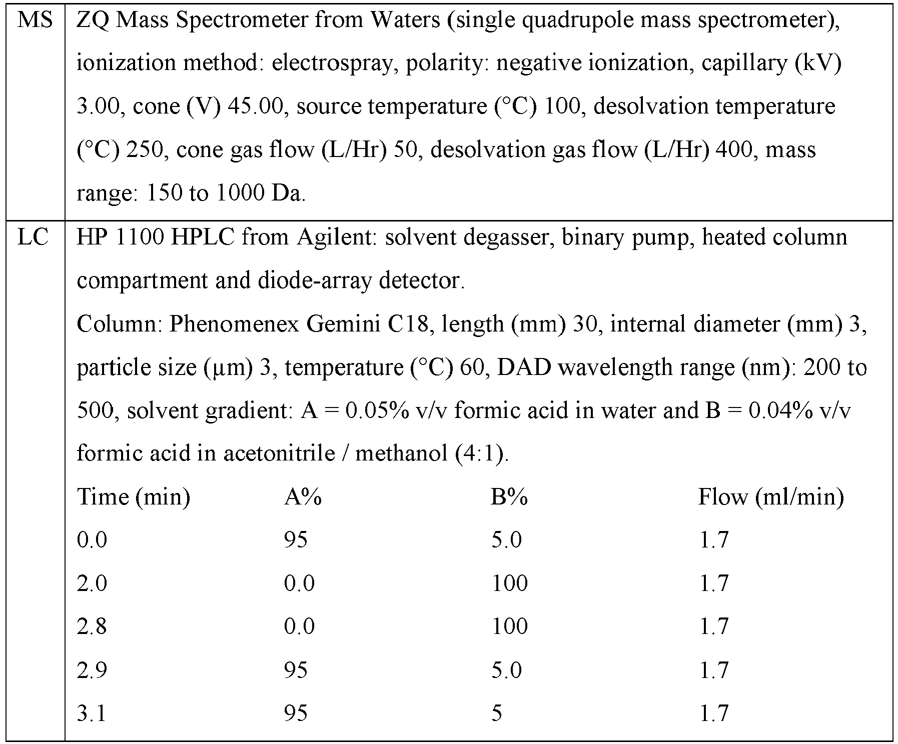

### Example 1: Preparation of enantioenritched 4-[(3R)-3-Cyano-3-(3,5-dichloro-phenyl)-4,4,4-trifluoro-butyryl]-2-methyl-benzoic acid tert-butyl ester

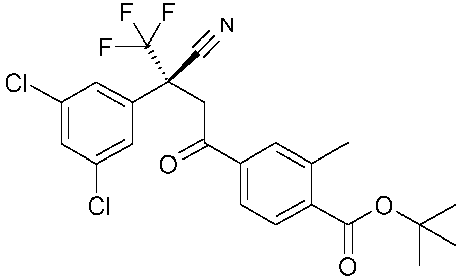

Potassium cyanide (6.465g, 99.283 mmol) and acetone cyanohydrin (23.9ml, 261.272 mmol) were added to a solution of 4-[(E)-3-(3,5-Dichloro-phenyl)-4,4,4-trifluoro-but-2-enoyl]-2-methyl-benzoic acid tert-butyl ester (40.000g, 87.091 mmol) in toluene (600.0ml). To this vigorously stirred suspension was added 9-anthrylmethyl quininium chloride (7.200g, 13.064 mmol). The reaction mixture was stirred at 60°C for 2 hours and at room temperature during 63 hours. At this time water was added and the reaction mixture was extracted with dichloromethane (3x). The crude product was purified by flash chromatography (0% to 5% ethyl acetate in cyclohexane) to afford 4-[(R)-3-Cyano-3-(3,5-dichloro-phenyl)-4,4,4-trifluoro-butyryl]-2-methyl-benzoic acid tert-butyl ester (35.80g, 67.6%) as a white amorphous solid. Chiral HPLC analysis (Chiralpack IB, Heptane:2-propanol = 98:2 1ml/min): retention time 8.11 minutes (minor enantiomer, 5%), 9.95 minutes (major enantiomer, 95%) ¹H NMR (400MHz, CDCl₃) δ 7.89 (d, 1H), 7.78-7.72 (m, 2H), 7.48 (s, 2H), 7.46-7.42 (m, 1H), 4.17 (d, 1H), 4.02 (d, 2H), 2.62 (s, 3H), 1.62 (s, 9H)

### Example 2: Preparation of 9-anthrylmethyl quinidinium chloride

A solution of 9-chloromethyl-anthracene (0.91 g, 1.3eq,0.40mmol ) and quinidine [CAS = 56-54-2] (1 g, 0.38) in toluene (10 ml) was heated at 90°C for 18 hours. The reaction mixture was filtered, washed with n-heptane. The solid was recrystallised from chloroform and n-heptane to afford the title product (1.69 g) as a yellow solid. Preparation of this compound is also reported in dissertation: contributions to the asymmetric catalysis of c-c couplings,and to the chemical induction of cardiomyogenesis from embryonic stem cells, bianca seelig, university köln 2009.

### Example 3: Preparation of enantioenritched 4-[(3S)-3-Cyano-3-(3,5-dichloro-phenyl)-4,4,4-trifluoro-butyryl]-2-methyl-benzoic acid tert-butyl ester

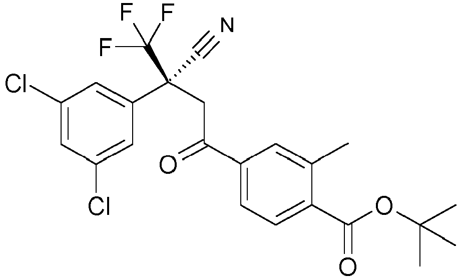

Potassium cyanide (0.021g, 0.32 mmol) and acetone cyanohydrin (0.086mg,1.01 mmol,) were added to a solution tert-butyl 4-[(E)-3-(3,5-dichlorophenyl)-4,4,4-trifluoro-but-2-enoyl]-2-methyl-benzo (0.150mg, 0.326mmol) in toluene (1.0ml). To this vigorously stirred suspension was added 9-anthrylmethyl quinidinium chloride (0.054g, 0.098 mmol). The reaction mixture was stirred at 45° C for 18 hours. At this time water was added and the reaction mixture was extracted with toluene (3x). The crude product was purified by flash chromatography (0% to 5% ethyl acetate in cyclohexane) to afford tert-butyl 4-[(3S)-3-cyano-3-(3,5-dichlorophenyl)-4,4,4-trifluoro-butanoyl]-2-methyl-benzoate (0.080g, 50%) as a white foam.

Chiral HPLC analysis (Chiralpack IB, Heptane:2-propanol = 98:2 1ml/min): retention time 7.64 minutes (major enantiomer, 78.5%), 9.53 minutes (minor enantiomer, 21.5%).¹H NMR (400MHz, CDCl₃) δ 7.89 (d, 1H), 7.78-7.72 (m, 2H), 7.48 (s, 2H), 7.46-7.42 (m, 1H), 4.17 (d, 1H), 4.02 (d, 2H), 2.62 (s, 3H), 1.62 (s, 9H)

### Example 4: Preparation of enantioenritched (3R)-3-(3,5-dichlorophenyl)-3-(trifluoromethyl)pyrrolidine-2,5-dione

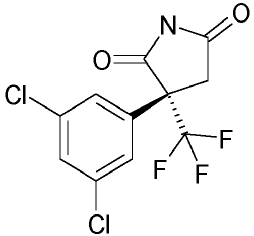

Hydrogen peroxide (aq. 30%, 2.0 mL) was added to sulfuric acid (96%, 15.0 mL) slowly at <0°C followed by *tert*-butyl 4-[(3R)-3-cyano-3-(3,5-dichlorophenyl)-4,4,4-trifluoro-butanoyl]-2-methyl-benzoate (600 mg) in dichloromethane (6.0 mL). The reaction mixture was stirred for 30 min. at 0°C. The reaction mixture was added on ice, treated with saturated aq. Na₂SO₃ and extracted with dichloromethane (3x). The combined organic phase were dried (Na₂SO₄), evaporated giving 1.03 g of yellowish foam. It was dissolved in methanol (8 mL) and treated with 8M sodium hydroxide (3 mL). The reaction mixture was stirred at 30 min at RT, acidified with conc. HCl and extracted with dichloromethane. The organic phase was washed with water, NaHCO₃ (aq., sat.), water. It was dried (Na₂SO₄) and evaporated giving the title compound as a white solid 450 mg (70%).
¹H-NMR (400 MHz, CDCl₃): δ 3.49 (d, J=18.5 Hz), 3.25(d, J=18.5 Hz), 7.47 (s, 1H), 7.57 (s, 2H), 8.53 (bs, 1H) ppm.
¹³C-NMR (101 MHz, CDCl₃): δ 39.0, 56.8 (q, J = 27 Hz), 123.7 (q, J = 284 Hz), 126.6), 130.14, 134.4, 136.0, 170.4, 171.6 ppm.
¹⁹F-NMR (377 MHz, CDCl₃): δ -71.6 ppm.
LC/MS (ES-): 310 (M-H)⁻, Rₜ = 1.72 min
GC/MS (CI): 312 (M+H)⁺, Rₜ = 6.25 min
m.p. = 138-141°C

### Example 8: Preparation of (E)-3-(3,5-dichlorophenyl)-4,4,4-trifluoro-1-(2-furyl)but-2-en-1-one

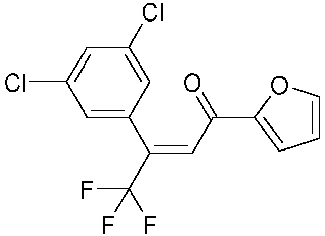

A flask was charged with starting material 1-(2-furyl)ethanone (5.00 g), 1-(3,5-dichlorophenyl)-2,2,2-trifluoro-ethanone (12.39 g), potassium carbonate (7.00 g), triethylamine (0.46 g) and 1,2-dichloroethane (50 mL) .The reaction mixture was stirred and heated to reflux for 12 hours. Then potassium carbonate (6.00 g) was added and heating was continued for another 12 hours. The reaction mixture was diluted with dichloromethane, washed with water (2x) and the organic phase was dried over Na₂SO₄ and evaporated. Purification of the crude product via column chromatography (silica, *n*-heptane/ethyl acetate gradient) gave 10.8g (71%) of the desired product.
¹H-NMR (400 MHz, CDCl₃): δ 7.66-7.64 (m, 1H); 7.42-7.39 (m, 2H); 7.26 (d, 1H, *J* = 3.7 Hz); 7.18 (d, 2H, *J =* 1.47Hz) ppm.
¹³C-NMR (101 MHz, CDCl₃): δ 176.5; 152.5; 147,7; 138.8 (q); 135.0; 133.6; 129.5; 128.4(q); 127.4; 122.1(q); 119.5; 113.1 ppm.
¹⁹F-NMR (377 MHz, CDCl₃): δ - 67.09 ppm.
GC/MS (CI): 335 (M+H)⁺, Rₜ = 5.73 min
m.p. = 72-76°C

### Example 9: Preparation of enantioenritched 2-(3,5-dichlorophenyl)-4-(2-furyl)-4-oxo-2-(trifluoromethyl)butanenitrile

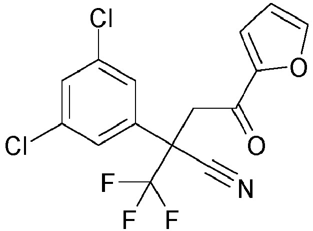

To a solution of (E)-3-(3,5-dichlorophenyl)-4,4,4-trifluoro-1-(2-furyl)but-2-en-1-one (0.200 g, 0.597 mmol) in toluene (3 mL) were added (R)-[1-(9-anthrylmethyl)-5-vinyl-quinuclidin-1-ium-2-yl]-(6-methoxy-4-quinolyl)methanol chloride (0.066 g, 0.119 mmol), acetone cyanohydrin (0.165 mL, 1.802 mmol) and potassium carbonate (0.09206 g, 0.657 mmol) sequentially. The reaction mixture was vigorously stirred at room temperature for 2h. At this time aqueous solution of NH₄Cl was added and the reaction mixture was extracted with AcOEt, dried (Na₂SO₄) and evaporated. Purification of the crude product via column chromatography (silica, *n*-heptane/ethyl acetate gradient) gave 165 mg (76%) of the desired product as white semisolid.

Chiral HPLC analysis (Chiralpack AS-R3, Acetonitrile:MeOH:Water =45:5:50, 1ml/min): retention time 56.73 minutes (minor enantiomer, 13.4%), 59.31 minutes (major enantiomer, 86.6%). (The identity of the stereochemistry was not determined. It is expected that the alternative isomer could be produced in enantiomeric excess with use an appropriate catalyst with reversed stereochemistry.)
¹H-NMR (400 MHz, CDCl₃): δ 7.68-7.67 (m, 1H); 7.51 (s, 2H); 7.46 (t, 1H);7.30-7.27(m,1H); 6.64(dd, 1H,J=1.83Hz, J=3.67Hz); 4.48(d, 1H, J=18.3Hz); 3.89(d,1H, J=18.3Hz) ppm GC/MS (CI): 362 (M+H)⁺, Rₜ = 6.60 min

### Example 10: Preparation of enantioenritched 3-cyano-3-(3,5-dichlorophenyl)-4,4,4-trifluorobutanoic acid

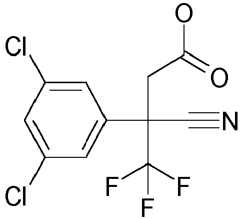

2-(3,5-dichlorophenyl)-4-(2-furyl)-4-oxo-2-(trifluoromethyl)butanenitrile (0.150 g, 0.414 mmol) was dissolved in a mixture of dichloromethane, acetonitrile and water (1:1:2). Sodium periodate (0.627 g, 2.900 mmol) was added, followed by ruthenium chloride hydrate (0.003 g, 0.035 mmol). The reaction mixture was stirred at room temperature overnight. The reaction was diluted with CH2Cl2; the organic phase was washed with H₂O and dried over Na2SO4 giving 53mg of violet solid.
¹H-NMR (400 MHz, CDCl₃): δ 7.49 (s, 1H); 7.46 (s, 2H); 3.42 (s, 2H) ppm
-LC/MS : Rₜ =1.83min; 310 (M-H)-,

### Example 11: Preparation of (E)-3-(3,5-dichlorophenyl)-4,4,4-trifluoro-1-pyrrol-1-yl-but-2-en-1-one

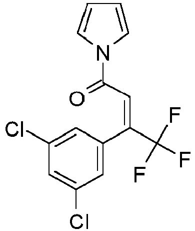

A suspension of sodium hydride (0.117 g) in 1,2-dimethoxyethane (5 ml) was cooled to 0 °C and a solution of 2-diethoxyphosphoryl-1-pyrrol-1-yl-ethanone (0.754 g) in 1,2-dimethoxyethane (2 ml) was added drop-wise and stirred for 20 min. To the reaction mixture was added drop-wise a solution of 1-(3,5-dichlorophenyl)-2,2,2-trifluoro-ethanone (0.503 g) in 1,2-dimethoxyethane (2 ml). The reaction was stirred for a further 30 min at 0 °C, then allowed to warm to RT and stirred for a further 2 h. The reaction mixture was quenched by cautious addition of saturated NH₄Cl (10 ml) solution over ice and extracted with ethyl acetate (3x15 ml). The combined organics were passed through a PTFE membrane and concentrated *in vacuo* to give a turbid orange oil, which was taken up in toluene and purified by column-chromatography on a pre-packed silica column eluting with heptanes/ ethyl acetate to give the title compound as a pale yellow oil (0.313 g)
¹H-NMR: (400 MHz, CDCl₃) *δ*_{H} ppm 7.40 (m, 2 H), 7.20 (m, 3 H), 7.18 - 7.19 (m, 1 H), 6.33 - 6.35 (m, 2 H).

### Example 12: Preparation of enantioenritched 2-(3,5-dichlorophenyl)-4-oxo-4-pyrrol-1-yl-2-(trifluoromethyl)-butanenitrile

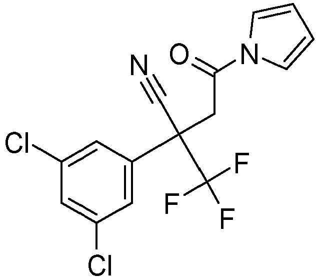

To a suspension of potassium carbonate (0.144 g) and (*R*)-(6-methoxy-4-quinolyl)-[(2*S*,4*S*,5*R*)-1-[(2,3,4,5,6-pentafluorophenyl)methyl]-5-vinyl-quinuclidin-1-ium-2-yl]methanol bromide (0.126 g) in toluene (4 ml) was added a solution of (*E*)-3-(3,5-dichlorophenyl)-4,4,4-trifluoro-1-pyrrol-1-yl-but-2-en-1-one (0.313 g) in toluene (2 ml) followed by 2-hydroxy-2-methylpropanenitrile (100 µl) in toluene (2 ml). The reaction mixture was heated to 45 °C overnight before potassium cyanide (0.077 g), a drop of water and a further aliquot of the 2-hydroxy-2-methyl-propanenitrile (100 µl) were added and the reaction mixture heated to 60 °C for a further 4 h. The reaction was poured onto saturated NH₄Cl solution and extracted with dichloromethane (3x25 ml). The combined organics were washed with brine, dried over Na₂SO₄ and concentrated *in vacuo* to give a dark amber gum, which was purified by column-chromatography on a pre-packed silica column eluting with heptanes/ ethyl acetate to give the title compound as a pale yellow oil (0.084 g). Chiral HPLC analysis (Chiralpack IA, Heptane:isopropanol = 95:5, 1 ml/min): retention time 6.09 minutes (minor enantiomer, 34%), 6.96 minutes (major enantiomer, 66%). (The identity of the stereochemistry was not determined. It is expected that the alternative isomer could be produced in enantiomeric excess with use of an appropriate catalyst with reversed stereochemistry)
¹H-NMR (400 MHz, CDCl₃) *δ*_{H} ppm 7.48 - 7.51 (m, 2 H), 7.45 - 7.48 (m, 1H), 7.21 - 7.26 (m, 2 H), 6.37 (m, 1 H), 3.90 (m, 1H).

### Example 13: Preparation of enantioenritched methyl 3-cyano-3-(3,5-dichlorophenyl)-4,4,4-trifluoro-butanoate

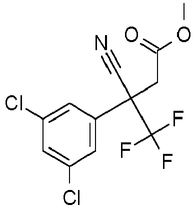

Enantioenritched 2-(3,5-Dichlorophenyl)-4-oxo-4-pyrrol-1-yl-2-(trifluoromethyl)butanenitrile (22 mg) was taken up in methanol (2 ml) and sodium methoxide (33 mg) was added. The reaction was stirred at ambient temperature for 1 h before saturated NH₄Cl solution (4 ml) was added and the mixture extracted with EtOAc (3x8 ml). The organic portions were combined, passed through a PTFE membrane and concentrated *in vacuo* to give a colourless semi-solid, which was then taken up in toluene and purified by column-chromatography on silica, eluting with cyclohexane/EtOAc to give the title compound as a colourless oil (7 mg). ¹H-NMR (400 MHz, CHLOROFORM-*d*): *δ*_{H} ppm 7.45 - 7.49 (m, 3 H), 3.68 (s, 3 H), 3.36 - 3.39 (m, 1H).

## Claims

1. A compound of formula VI 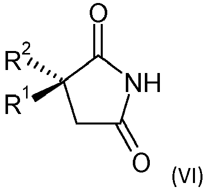 wherein
R¹ is chlorodifluoromethyl or trifluoromethyl;
R² is aryl or heteroaryl, each optionally substituted by one to five Q¹;
each Q¹ is independently selected from hydrogen, halogen, cyano, C₁-C₈alkyl, C₁-C₈haloalkyl, C₁-C₈alkoxy and C₁-C₈haloalkoxy.

2. A process for the enantio-selective preparation the compound of formula VI of claim 1 comprising:
(A) reacting a compound of formula I 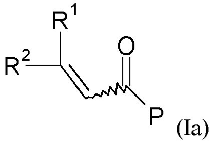 wherein P is alkyl, aryl or heteroaryl, wherein the heteroaryl is connected at P via a ring carbon atom, and R¹ and R² are defined as in claim 1, with a source of cyanide in the presence a chiral catalyst to give a compound of formula II 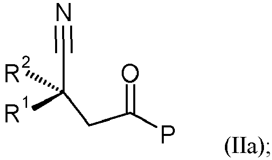 and
(B) oxidising the compound of formula II with a peroxy acid, or peroxide in the presence of an acid, preferably a strong acid; or
(C) oxidatively cleaving the compound of formula II to give a compound of formula XIX 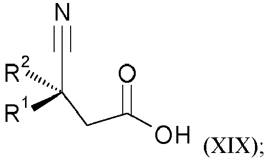 and hydrolysing and dehydrating the compound of formula XIX; or
(D) partially hydrolysing the compound of formula II to give a compound of formula V 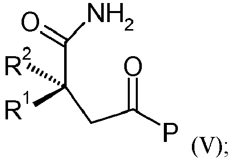 and cyclising the compound of formula V; or
(E) hydrolysing the compound of formula II to give a compound of formula VII 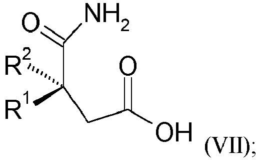 and cyclising the compound of formula VII; each of steps (B) to (E) to give the compound of formula VI.

## Patentansprüche

1. Verbindung der Formel VI 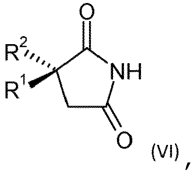 wobei
R¹ für Chlordifluormethyl oder Trifluormethyl steht;
R² für Aryl oder Heteroaryl, das jeweils gegebenenfalls durch ein bis fünf Q¹ substituiert ist, steht;
Q¹ jeweils unabhängig aus Wasserstoff, Halogen, Cyano, C₁-C₈-Alkyl, C₁-C₈-Halogenalkyl, C₁-C₈-Alkoxy und C₁-C₈-Halogenalkoxy ausgewählt ist.

2. Verfahren zur enantioselektiven Herstellung der Verbindung der Formel VI nach Anspruch 1, das Folgendes umfasst:
(A) Umsetzen einer Verbindung der Formel I 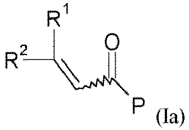 wobei P für Alkyl, Aryl oder Heteroaryl steht, wobei das Heteroaryl über ein Ringkohlenstoffatom an P gebunden ist, und R¹ und R² wie in Anspruch 1 definiert sind, mit einer Cyanidquelle in Gegenwart eines chiralen Katalysators zu einer Verbindung der Formel II 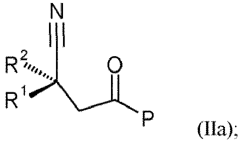 und
(B) Oxidieren der Verbindung der Formel II mit einer Peroxysäure oder Peroxid in Gegenwart einer Säure, vorzugsweise einer starken Säure; oder
(C) oxidatives Spalten der Verbindung der Formel II zu einer Verbindung der Formel XIX 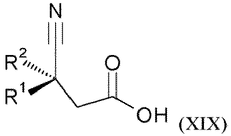 und Hydrolysieren und Dehydratisieren der Verbindung der Formel XIX; oder
(D) partielles Hydrolysieren der Verbindung der Formel II zu einer Verbindung der Formel V 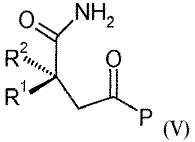 und Cyclisieren der Verbindung der Formel V; oder
(E) Hydrolysieren der Formel II zu einer Verbindung der Formel VII 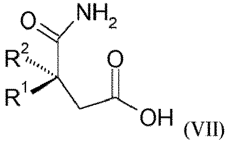 und Cyclisieren der Verbindung der Formel VII; wobei jeder der Schritte (B) bis (E) die Verbindung der Formel VI ergibt.

## Revendications

1. Composé de formule VI 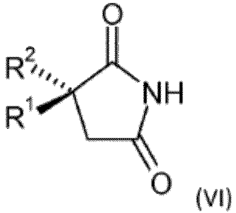 R¹ étant chlorodifluorométhyle ou trifluorométhyle ;
R² étant aryle ou hétéroaryle, chacun éventuellement substitué par un à cinq Q¹ ;
chaque Q¹ étant indépendamment choisi parmi hydrogène, halogène, cyano, C₁₋₈alkyle, C₁₋₈halogénoalkyle, C₁₋₈alcoxy et C₁₋₈halogénoalcoxy.

2. Procédé pour la préparation énantio-sélective du composé de formule VI selon la revendication 1 comprenant :
(A) la mise en réaction d'un composé de formule I 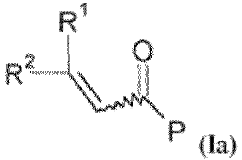 P étant alkyle, aryle ou hétéroaryle, l'hétéroaryle étant relié à P via un atome de carbone de cycle, et
R¹ et R² étant définis comme dans la revendication 1, avec une source de cyanure en la présence d'un catalyseur chiral pour donner un composé de formule II 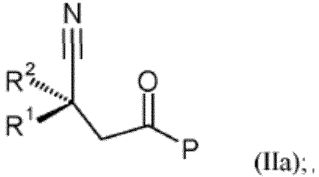 et
(B) l'oxydation du composé de formule II avec un peroxyacide, ou un peroxyde en la présence d'un acide, préférablement un acide fort ; ou
(C) le clivage de manière oxydante du composé de formule II pour donner un composé de formule XIX 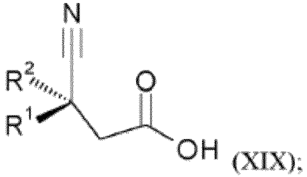 et l'hydrolyse et la déshydratation du composé de formule XIX ; ou
(D) l'hydrolyse partielle du composé de formule II pour donner un composé de formule V 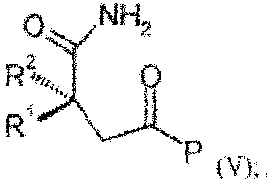 et la cyclisation du composé de formule V ; ou
(E) l'hydrolyse du composé de formule II pour donner un composé de formule VII 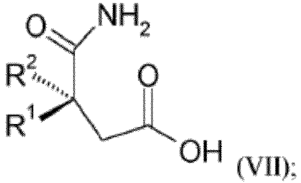 et la cyclisation du composé de formule VII ;
chacune des étapes (B) à (E) pour donner le composé de formule VI.
